(19) Europäisches Patentamt
European Patent Office
Office européen des brevets

(11) EP 2 083 017 A1

(12) # EUROPEAN PATENT APPLICATION

published in accordance with Art. 153(4) EPC

(43) Date of publication:
**29.07.2009 Bulletin 2009/31**

(21) Application number: **07807383.0**

(22) Date of filing: **14.09.2007**

(51) Int Cl.:
***C07K 16/28*** (2006.01) ***A61K 39/395*** (2006.01)
***C12N 15/02*** (2006.01) ***C12N 15/09*** (2006.01)
***C12P 21/08*** (2006.01)

(86) International application number:
**PCT/JP2007/067979**

(87) International publication number:
**WO 2008/032833 (20.03.2008 Gazette 2008/12)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **14.09.2006 JP 2006250132**
**02.11.2006 JP 2006299581**
**07.08.2007 JP 2007205860**

(71) Applicant: **Medical & Biological Laboratories Co., Ltd.**
**Nagoya-shi, Aichi 460-0002 (JP)**

(72) Inventor: **MURAKAMI, Akihiro**
**Ina-shi, Nagano 396-0002 (JP)**

(74) Representative: **Müller Fottner Steinecke**
**Rechtsanwälte Patentanwälte**
**Postfach 31 01 40**
**80102 München (DE)**

(54) **ANTIBODY HAVING ENHANCED ADCC ACTIVITY AND METHOD FOR PRODUCTION THEREOF**

(57) Disclosed is an antibody having an enhanced ADCC activity. Also disclosed is a method for producing the antibody. It was attempted to advance the technique of the amino acid mutation in an Fc region established by researchers of Genentech Inc. or the like, and a study was made on whether or not the ADCC activity can be enhanced by the mutation of an amino acid residue in an Fc region into cysteine (Cys) which may cause a drastic structural change that cannot be drawn by a computational search. As a consequence, a chemeric antibody is provided which has the mutation of an amino acid residue at at least one position selected from the group consisting of 286th, 287th, 288th, 289th, 290th, 291st, 292nd, 294th, 298th, 301st, 302nd, 303rd, 305th, 306th, 307th, 308th and 309th positions into a Cys residue in an H-chain constant region.

FIG. 4

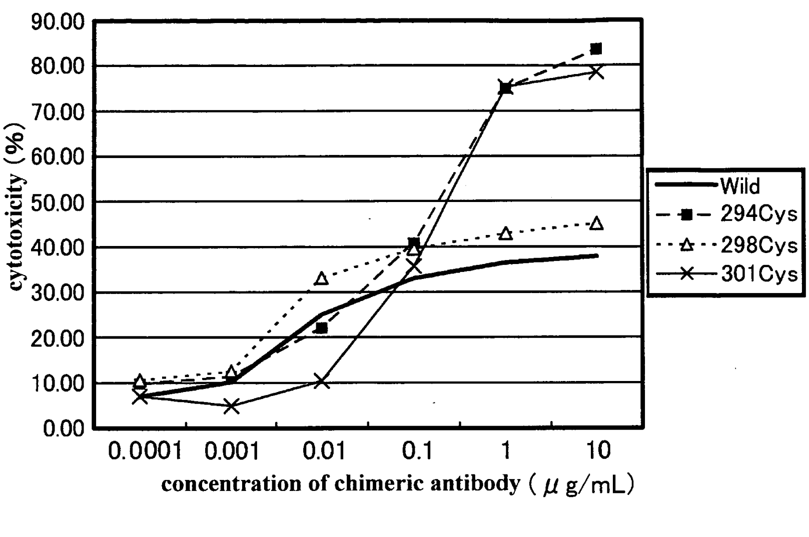


EP 2 083 017 A1

## Description

TECHNICAL FIELD

The present invention relates to a method for enhancing an ADCC activity of an antibody and to an antibody having an enhanced ADCC activity.

BACKGROUND ART

A chimeric antibody having a mouse-type variable region and a human-type constant region, and a humanized antibody having human-type variable region and constant region are promising treatment agent for cancers or chronic rheumatoid arthritis. A chemically synthesized treatment agent such as cisplatin, which has been conventionally used for treating cancers, has low identification ability between cancer cells and normal cells and has a high toxicity. Therefore, chemotherapy to a cancer is a large burden to cancer patients. On the other hand, since chimeric antibodies and humanized antibodies act on a cancer cell surface by recognizing a certain molecule, they have a low toxicity and apply less physical burden to patients. In the treatment of chronic rheumatoid arthritis, what conventional treatment mainly using a steroid drug can do is to slow the progress of symptoms of rheumatism. However, a humanized antibody to an interleukin 6 receptor can suppress causative factors of bone destruction or inflammation, exhibiting a remarkable effect of treatment with the humanized antibody.

Methods of producing these therapeutic antibodies (chimeric antibody and humanized antibody) are roughly divided into three methods. The first one is to replace protein to humanized protein with an antigen binding site of antibody left from a mouse antibody to a chimeric antibody to a humanized antibody by using gene recombination technology. The second one is a method using a phage display. In this method, since a complete human type variable region capable of recognizing an intended protein can be selected from various kinds of antibody variable regions derived from human expressed on the phage surface, a complete human type variable region in which a human constant region is further added can be produced by using the selected complete human variable region as a material and by gene recombination technology. Finally, a method of using a TC mouse that has been genetically manipulated so as to have a human antibody production gene (Nature Genetics, Vol.16, 113-114, 1997). Since this TC mouse has been genetically manipulated so that all antibodies produced in the mouse become a human type when an antigen as an intended protein is immunized, from hybridoma cells obtained by fusing lymphocyte cells and mouse myeloma cells, which are taken out from the TC mouse that has been immunized with an antigen, a complete humanized antibody capable of recognizing the immunized antigen can be produced.

According to the above-mentioned three methods, although a chimeric antibody or a humanized antibody can be produced, a manufacturing cost of therapeutic antibodies in any methods is higher as compared with treatment agents using a low molecule compound. Because the manufacturing cost is high, the price of therapeutic antibody is also extremely high. Therefore, patients with cancer or rheumatism have to pay high medical expenses.

One solution that has been thought to solve this problem includes increasing the therapeutic effect of a therapeutic antibody per unit mass with respect to cancer cells. That is to say, if the same treatment effect as that with a conventional dosage amount can be obtained even with a small amount of therapeutic antibodies, a dosage amount for a patient can be reduced, and an expense for a single dosage can also be reduced. One index showing the therapeutic effect of a therapeutic antibody includes an Antibody-Dependent-Cellular-Cytotoxicity (ADCC) activity. This ADCC activity is a mechanism in which an Fc portion of the therapeutic antibody is bonded to an Fcγ receptor on a killer cell capable of killing cancer cells and leads the variable region killer cell to the cancer cell by identification effect of a variable region of the therapeutic antibody, resulting in killing the cancer cells by the killer cell via a therapeutic antibody.

Characteristics of therapeutic antibodies required to increase the ADCC activity includes that: 1) a variable region of a therapeutic antibody is capable of strongly recognizing a protein specific to the surface of cancer cells; and 2) an Fc portion of the therapeutic antibody can strongly bind to an Fcγ receptor on the killer cell. Many studies for increasing the ADCC activity by solving the item 2) have been carried out.

Shinkawa T et al. have focused on two sugar chain structures (N-Linked oligosaccharide) linked to asparagine (Asn) that is amino acid at position 297 of an Fc region of a therapeutic antibody IgG1 and found that the ADCC activity is increased by 20 to 100 times when fucose is deleted in the sugar chain structure (J. Biol. Chem. Vol.278, 3466-3473, 2003). The ADCC activity by the structure in which fucose is deleted has been reported also by Shields R L et al (J. Biol. Chem. Vol.277, 26733-26740, 2002). Furthermore, Pablo U et al. have focused on the same sugar chain structure and controlled the binding amount of bisecting N-acetylglucosamine in the sugar chain structure, thereby finding that the ADCC activity can be increased by several times (Nature Biotechnology Vol.17, 176-180, 1999).

Other than such a method for increasing the ADCC activity of a therapeutic antibody by modifying a sugar chain structure linked to an Fc region of IgG1, researchers of U.S. Genentech Inc. have succeeded in enhancing the ADCC activity by several tens of times by triple mutation of S298A, E333A, and K334A by searching a technology for enhancing the binding with respect to an Fc receptor by mutating amino acid of an Fc structure itself of the therapeutic antibody by a computational search (J. Biol. Chem. Vol.276, 6591-6604, 2001).
The followings are prior art documents related to the present invention.

[Patent document 1] WO 2004/029207
[Patent document 2] WO 2000/042072
[Patent document 3] WO 2004/063351
[Patent document 4] WO 2004/099249
[Patent document 5] WO 2006/019447
[Patent document 6] Japanese Translation of PCT Publication No. 2006-512407
[Patent document 7] Japanese Translation of PCT Publication No. 2003-512019
[Patent document 8] WO2006/104989
[Patent document 9] WO2006/105062
[Non-patent document 1] Nature Genetics, Vol. 16, 113-114, 1997
[Non-patent document 2] J. Biol. Chem. Vol. 278, 3466-3473, 2003
[Non-patent document 3] J. Biol. Chem. Vol. 277, 26733-26740, 2002
[Non-patent document 4] Nature Biotechnology Vol. 17, 176-180, 1999
[Non-patent document 5] J. Biol. Chem. Vol. 276, 6591-6604, 2001

[DISCLOSURE OF THE INVENTION]

[Problems to be Solved by the Invention]

The present invention aims to provide an antibody having an enhanced ADCC activity and a method for production thereof.

[Means to Solve the problem]

The present inventors have further advanced the technique of the amino acid mutation in an Fc region established by researches of Genentech Inc, or the like, and have made a study on whether or not the ADCC activity can be enhanced by introducing cysteine (Cys) that is amino acid capable of causing a drastic structural change in the Fc region, which cannot be derived from the computer search. The cysteine substitution means introduction of a thiol group (-SH). Therefore, disulfide bonding (-S-S-) occurs. Thus, drastic change in the Fc structure has been expected. The present inventors have studied whether or not the ADCC activity can be enhanced by substituting amino acid at position 280, 281, 282, 283, 284, 285, 286, 287, 288, 289, 290, 291, 292, 293, 294, 298, 299, 300, 301, 302, 303, 305, 306, 307, 308, 309, 310 or 314 of the EU index numbers of human IgG1 H chain constant region shown in Sequence of proteins of Immunological Interest (NIH Publication No.91-3242, 1991) by Elvin A. Kabat et al. (hereinafter, also referred to as "kabat number" in this specification) with cysteine (hereinafter, also referred to as "Cys") in human Cγ1 (amino acid sequence of SEQ ID NO: 53).
As a result, the present inventors have found that a chimeric antibody in which amino acid at position 286, 287, 288, 289, 290, 291, 292, 294, 298, 301, 302, 303, 305, 306, 307, 308 or 309 in human Cγ1 is substituted with Cys shows an extremely high ADCC activity as compared with a wild type chimeric antibody, and reached the present invention. That is to say, the present invention provides the following [1] to [41].

[1] An antibody including an H chain constant region in which an amino acid residue at at least one position selected from the group consisting of positions 286, 287, 288, 289, 290, 291, 292, 294, 298, 301, 302, 303, 305, 306, 307, 308 and 309 is substituted with cysteine.
[2] The antibody according to [1], wherein the H chain constant region is any one constant region selected from the group consisting of human Cγ1, Cγ2, Cγ3, and Cγ4.
[3] The antibody according to [1] or [2], wherein an ADCC activity is increased as compared with an ADCC activity before substitution.
[4] The antibody according to any one of [1] to [3], wherein the antibody has an improved stability, solubility or binding affinity to an Fc ligand.
[5] The antibody according to any one of [1] to [4], wherein a sugar chain is provided.
[6] The antibody according to [5], wherein an effector function is improved.
[7] The antibody according to any one of [1] to [6], wherein the antibody binds to FcγRI, FcγRII, FcγRIII, or FcRn.
[8] The antibody according to any one of [1] to [7], which has specificity with respect to a target antigen selected from the group consisting of CD20, CD22, CD33, CD52, Her2/neu, EGFR, EpCAM, MUC1, GD3, CEA, CA125, HLA-DR, TNF alpha and VEGF.

[9] An antibody, which is a human antibody having a specificity to CD20 in which an amino acid residue at one position selected from the group consisting of positions 286, 287, 288, 289, 290, 291, 292, 294, 298, 301, 302, 303, 305, 306, 307, 308, and 309 is substituted with cysteine.

[10] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 95 as CH;
(2) an antibody including an H chain having an amino acid sequence set forth in SEQ ID NO: 93;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 97 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[11] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 101 as CH;
(2) an antibody including an H chain having an amino acid sequence set forth in SEQ ID NO: 99;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 103 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[12] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 107 as CH;
(2) an antibody including an H chain having an amino acid sequence set forth in SEQ ID NO: 105;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 109 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[13] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 54 as CH;
(2) an antibody including an H chain having an amino acid sequence set forth in SEQ ID NO: 45;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 73 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[14] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 83 as CH;
(2) an antibody including an H chain having an amino acid sequence set forth in SEQ ID NO: 81;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[15] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 89 as CH;
(2) an antibody including an H chain having an amino acid sequence set forth in SEQ ID NO: 87;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 91 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[16] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 131 as CH;
(2) an antibody including an H chain having an amino acid sequence set forth in SEQ ID NO: 129;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 133 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[17] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 137 as CH;
(2) an antibody including an H chain having an amino acid sequence set forth in SEQ ID NO: 135;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 139 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[18] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 143 as CH;
(2) an antibody including an H chain having an amino acid sequence set forth in SEQ ID NO: 141;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 145 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[19] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 149 as CH;
(2) an antibody including an H chain having an amino acid sequence set forth in SEQ ID NO: 147;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 151 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[20] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 155 as CH;
(2) an antibody including an H chain having an amino acid sequence set forth in SEQ ID NO: 153;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 157 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[21] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 161 as CH;
(2) an antibody including an H chain having an amino acid sequence set forth in SEQ ID NO: 159;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 163 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[22] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 167 as CH;
(2) an antibody including an H chain having an amino acid sequence set forth in SEQ ID NO: 165;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 169 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[23] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 173 as CH;
(2) an antibody including an H chain having an amino acid sequence set forth in SEQ ID NO: 171;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 175 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[24] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 179 as CH;
(2) an antibody including an H chain having an amino acid sequence set forth in SEQ ID NO: 177;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 181 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[25] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 185 as CH;
(2) an antibody including an H chain having an amino acid sequence set forth in SEQ ID NO: 183;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 187 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[26] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 191 as CH;
(2) an antibody including an H chain having an amino acid sequence set forth in SEQ ID NO: 189;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 193 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[27] An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 241 as CH;
(2) an antibody including an H chain having an amino acid sequence set forth in SEQ ID NO: 243;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 245 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

[28] An antibody described in any one of the following (1) to (4);

(1) an antibody including an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 83 as CH;
(2) an antibody including an H chain having an amino acid sequence set forth in SEQ ID NO: 209;
(3) an antibody including an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 203 as CDR1, an amino acid sequence set forth in SEQ ID NO: 205 as CDR2, an amino acid sequence set forth in SEQ ID NO: 207 as CDR3, and an amino acid sequence set forth in SEQ ID NO: 247 as CL; and
(ii) an L chain having an amino acid sequence set forth in SEQ ID NO: 201.

[29] A method for producing an antibody having an enhanced ADCC activity, the method including substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region.
[30] A method for producing an antibody having an enhanced ADCC activity, the method including the following steps (a) and (b);

(a)a step of expressing DNA encoding an H chain in which an amino acid residue at at least one position selected from the group consisting of positions 286, 287, 288, 289, 290, 291, 292, 294, 298, 301, 302, 303, 305, 306, 307, 308, and 309 is substituted with cysteine in an H chain constant region, and a DNA encoding an L chain; and
(b) a step of collecting an expression product in the step (a).

[31] The method according to [29] or [30], wherein the H chain constant region is any one of constant regions selected from the group consisting of human Cγ1, Cγ2, Cγ3, and Cγ4.
[32] A pharmaceutical composition including an antibody according to any of [1] to [28] and a pharmaceutically acceptable carrier.
[33] A method for treating non-human mammalian, the method including administering an antibody according to any of [1] to [28] to a subject.
[34] A nucleic acid encoding an antibody according to any of [1] to [28].
[35] A vector including a nucleic acid according to [34].
[36] A host cell having a vector according to [35].
[37] A host organism having a vector according to [35].

[38] A method for enhancing an ADCC activity, the method including substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region.
[39] The method according to [38], wherein the H chain constant region is any one constant region selected from the group consisting of human Cγ1, Cγ2, Cγ3, and Cγ4.
[40] A method for determining whether or not an ADCC activity of an antibody in a step (a) is enhanced, wherein it is determined that the ADCC activity of the antibody in a step (a) is enhanced when the ADCC activity measured in the step (a) is higher than an ADCC activity of the antibody before substitution;

(a) a step of providing a mutant of an antibody having an ADCC activity in which an amino acid residue at at least one position selected from the group consisting of positions 286, 287, 288, 289, 290, 291, 292, 294, 298, 301, 302, 303, 305, 306, 307, 308, and 309 is substituted with cysteine in an H chain constant region, and
(b) a step for measuring the ADCC activity of the step (a).

[41] A method for screening an antibody having an enhanced ADCC activity, the method including the following steps (a) to (d);

(a)providing an antibody having an ADCC activity;
(b)substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region of the antibody of the step (a);
(c) determining whether or not an ADCC activity of the antibody obtained in the step (b) is enhanced by the method according to [40]; and
(d) selecting an antibody that has been determined in the step (c) that the ADCC activity is enhanced.

[Effect of the Invention]

The present invention provides an antibody having an enhanced ADCC activity and a method for producing thereof. With respect to the ADCC activity evaluated by the lactate dehydrogenase release assay system, the antibody of the present invention has an enhanced ADCC activity as compared with that of the wild type antibody (in two points in which the wild type antibody and the mutant type antibody have the same concentration, the ratio of cytotoxicity of the mutant type antibody to that of the wild type antibody is increased). Therefore, the use of the mutant type antibody of the present invention can cause more cytotoxicity as compared with that of the wild type antibody.
Furthermore, the antibody of the present invention shows an increased ADCC activity particularly in a low concentration (in two points in which the wild type antibody and the mutant type antibody have the same cytotoxicity rate, the ratio of concentration of the mutant type antibody to that of the wild type antibody is increased). Therefore, the use of the antibody of the present invention as a therapeutic antibody can increase the therapeutic effect with respect to cancer cell per unit mass. Furthermore, since when the antibody of the present invention is used, a single dosage to a patient can be reduced, thus reducing the cost of drugs.

[BRIEF DESCRIPTION OF THE DRAWINGS]

Fig. 1 shows a DNA sequence of a genetically cloned anti-CD20 mouse L chain variable region. The beginning part of this DNA sequence matches the sequence of the MKV5 primer. Furthermore, a CDR in Fig. 1 is an abbreviation of a complementarity determining region. Bold underline shows a part of a mouse L chain constant region. A sequence excluding the constant region thereof is an anti-CD20 mouse L chain variable region.
Fig. 2 shows a DNA sequence of a genetically cloned anti-CD20 mouse H chain variable region. The beginning part of this DNA sequence matches the sequence of the MHV5 primer. Furthermore, a CDR in Fig. 1 is an abbreviation of a complementarity determining region. Bold underline shows a part of a mouse H chain constant region. A sequence excluding the constant region thereof is an anti-CD20 mouse H chain variable region.
Fig. 3 is a photograph showing electrophoretic patterns showing a purified wild type chimeric antibody or a Cys type chimeric antibody. Various purified chimeric antibodies are mixed with an SDS-PAGE Sample Buffer containing 2-mercaptoethanol and boiled for 5 minutes. The boiled sample is subjected to electrophoresis with 12.5%SDS- PAGE gel. In this photograph, a band around 50 kDa is an H chain and a band around 25 kDa is an L chain.
Fig. 4 is a graph showing the results of the ADCC activity of wild type, 294Cys type, 298Cys type and 301Cys type anti-CD20 chimeric antibodies. The ADCC activities of the 294Cys type chimeric antibody and the 301Cys type chimeric antibody are shown to be extremely high in the conditions of high concentration (0.1 to 10 μg/ml) as compared with the wild type chimeric antibody. On the other hand, the 298Cys type chimeric antibody shows a high ADCC activity in the entire measurement region including a low concentration region (at the concentration of 0.1 μg/mL or less) as compared with the wild type.

Fig. 5 is a graph showing the results of the ADCC activity of wild type, 290Cys type, 291Cys type and 292Cys type anti-CD20 chimeric antibodies. The ADCC activities of the 291Cys type chimeric antibody and the 292Cys type chimeric antibody are shown to be extremely high in the conditions of high concentration (0.01 to 10 μg/ml) as compared with the wild type chimeric antibody. On the other hand, the 290Cys type chimeric antibody shows a high ADCC activity in the substantially entire measurement region including a low concentration region (at the concentration of 0.1 μg/mL or less) as compared with the wild type.

Fig. 6 is a graph showing the results of the ADCC activity of wild type, 302Cys type and 303Cys type anti-CD20 chimeric antibodies. The ADCC activities of the 303Cys type chimeric antibody is shown to be extremely high in the conditions of high concentration (0.01 to 10 μg/ml) as compared with the wild type chimeric antibody. On the other hand, the 302Cys type chimeric antibody shows a high ADCC activity in the substantially entire measurement region including a low concentration region (at the concentration of 0.1 μg/mL or less) as compared with the wild type.

Fig. 7 is a graph showing the results of the ADCC activity of wild type, 298Cys type Anti-EGFR chimeric antibodies. The 298Cys type chimeric antibody shows a high ADCC activity in the entire measurement region including a low concentration region (at the concentration of 0.1 μg/mL or less) as compared with the wild type.

Fig. 8 is a graph showing the results of the ADCC activity of wild type, 286Cys type, 287Cys type, 288Cys type, and 289Cys type anti-CD20 chimeric antibodies. The 286Cys type chimeric antibody and 298Cys type chimeric antibody show an extremely high ADCC activity at high concentrations (0.01 to 10 μg/ml) as compared with the wild type.

Fig. 9 is a graph showing the results of the ADCC activity of wild type, 305Cys type, 306Cys type, 307Cys type, and 308Cys type anti-CD20 chimeric antibodies. As compared with that of the wild type, these chimeric antibodies show a high ADCC activity at relatively high concentrations (0.001 to 10 μg/ml).

Fig. 10 is a graph showing the results of the ADCC activity of wild type, 309Cys type anti-CD20 chimeric antibody. As compared with the wild type, these chimeric antibodies show a high ADCC activity at low concentrations (0.001 to 10 μg/ml).

[BEST MODE OF CARRYING OUT THE INVENTION]

The present invention provides an antibody in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308, 309, 310 or 314 is artificially substituted with cysteine in the EU index number (hereinafter, unless otherwise noted, the position of amino acid is described according to the EU index shown in kabat number) of human IgG1 H chain constant region shown in Sequence of proteins of Immunological Interest (NIH Publication No. 91-3242, 1991) by Elvin A. Kabat et al. The base sequence of human Cγ1 is shown in SEQ ID NO: 71 and the amino acid sequence thereof is shown in SEQ ID NO: 53.

As a human immunoglobulin, nine classes (isotypes), that is, IgG1, IgG2, IgG3, IgG4, IgA1, IgA2, IgD, IgE, and IgM are well known. In the antibody of the present invention, IgG1, IgG2, IgG3, and IgG4 of these isotypes are included. Furthermore, the antibody of the present invention also includes an antibody in which a portion other than an Fc region of the antibody is substituted with other peptide having an antigen binding ability. A portion other than the Fc region include, for example, a variable region, a CH1 region, a hinge region and the like, but not limited thereto. The isotype of the antibody is determined by a structure of a constant region. A constant region of each isotype of IgG1, IgG2, IgG3, and IgG4 is called Cγ1, Cγ2, Cγ3, and Cγ4, respectively. The antibody of the present invention also includes an antibody in which amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with Cys in Cγ1, Cγ2, Cγ3, and Cγ4. Base sequences of human Cγ2, Cγ3, and Cγ4 are shown in SEQ ID NOs: 65, 67, and 69. Furthermore, amino acid sequences of human Cγ2, Cγ3, and Cγ4 are shown in SEQ ID NOs: 66, 68 and 70.

Note here that the relation between the codon and amino acid in each sequence of Cγ1 (base sequence of SEQ ID NO: 71, amino acid sequence of SEQ ID NO: 53), Cγ2 (base sequence of SEQ ID NO: 65, amino acid sequence of SEQ ID NO: 66), Cγ3 (base sequence of SEQ ID NO: 67, amino acid sequence of SEQ ID NO: 68), and Cγ4 (base sequence of SEQ ID NO: 69, amino acid sequence of SEQ ID NO: 70) described in the attached sequence listing of the present application is shown in Tables 1 to 7. The codon and amino acid in Tables 1 to 7 correspond to the codons and amino acids of N terminal side to C terminal side in the sequence listing sequentially from the upper part to the lower part. Note here that all the sequences of the constant regions in this specification are made based on [Sequence of Proteins of Immunological Interest, 5th Ed. Public Health Service, National Institutes of Health, Bethesda, MD (1991)].

[Table 1]

| | IgG1 (Cγ1) | | IgG2(Cγ2) | | IgG3(Cγ3) | | IgG4(Cγ4) | | |
|---|---|---|---|---|---|---|---|---|---|
| EU INDEX of Kabat | DNA sequence | amino acid sequence | DNA sequence | amino acid sequence | DNA sequence | amino acid sequence | DNA sequence | amino acid sequence | |
| 118 | gcc | Ala | gcc | Ala | gct | Ala | gct | Ala | Human IgG1 constant region (CH1) |
| 119 | tcc | Ser | tcc | Ser | tcc | Ser | tcc | Ser | |
| 120 | acc | Thr | acc | Thr | acc | Thr | acc | Thr | |
| 121 | aag | Lys | aag | Lys | aag | Lys | aag | Lys | |
| 122 | ggc | Gly | ggc | Gly | ggc | Gly | ggc | Gly | |
| 123 | cca | Pro | cca | Pro | cca | Pro | cca | Pro | |
| 124 | tcg | Ser | tcg | Ser | tcg | Ser | tcc | Ser | |
| 125 | gtc | Val | gtc | Val | gtc | Val | gtc | Val | |
| 126 | ttc | Phe | ttc | Phe | ttc | Phe | ttc | Phe | |
| 127 | ccc | Pro | ccc | Pro | ccc | Pro | ccc | Pro | |
| 128 | ctg | Leu | ctg | Leu | ctg | Leu | ctg | Leu | |
| 129 | gca | Ala | gcg | Ala | gcg | Ala | gcg | Ala | |
| 130 | ccc | Pro | ccc | Pro | ccc | Pro | ccc | Pro | |
| 131 | tcc | Ser | tgc | Cys | tgc | Cys | tgc | Cys | |
| 132 | tcc | Ser | tcc | Ser | tcc | Ser | tcc | Ser | |
| 133 | aag | Lys | agg | Arg | agg | Arg | agg | Arg | |
| 134 | agc | Ser | agc | Ser | agc | Ser | agc | Ser | |
| 135 | acc | Thr | acc | Thr | acc | Thr | acc | Thr | |
| 136 | tct | Ser | tcc | Ser | tct | Ser | tcc | Ser | |
| 137 | ggg | Gly | gag | Glu | ggg | Gly | gag | Glu | |
| 138 | ggc | Gly | agc | Ser | ggc | Gly | agc | Ser | |
| 139 | aca | Thr | aca | Thr | aca | Thr | aca | Thr | |
| 140 | gcg | Ala | gcc | Ala | gcg | Ala | gcc | Ala | |
| 141 | gcc | Ala | gcc | Ala | gcc | Ala | gcc | Ala | |
| 142 | ctg | Leu | ctg | Leu | ctg | Leu | ctg | Leu | |
| 143 | ggc | Gly | ggc | Gly | ggc | Gly | ggc | Gly | |
| 144 | tgc | Cys | tgc | Cys | tgc | Cys | tgc | Cys | |
| 145 | ctg | Leu | ctg | Leu | ctg | Leu | ctg | Leu | |
| 146 | gtc | Val | gtc | Val | gtc | Val | gtc | Val | |
| 147 | aag | Lys | aag | Lys | aag | Lys | aag | Lys | |
| 148 | gac | Asp | gac | Asp | gac | Asp | gac | Asp | |
| 149 | tac | Thr | tac | Tyr | tac | Tyr | tac | Tyr | |
| 150 | ttc | Phe | ttc | Phe | ttc | Phe | ttc | Phe | |
| 151 | ccc | Pro | ccc | Pro | ccc | Pro | ccc | Pro | |
| 152 | gaa | Glu | gaa | Glu | gaa | Glu | gaa | Glu | |
| 153 | ccg | Pro | ccg | Pro | ccg | Pro | ccg | Pro | |
| 154 | gtg | Val | gtg | Val | gtg | Val | gtg | Val | |
| 155 | acg | Thr | acg | Thr | acg | Thr | acg | Thr | |
| 156 | gtg | Val | gtg | Val | gtg | Val | gtg | Val | |
| 157 | tcg | Ser | tcg | Ser | tcg | Ser | tcg | Ser | |
| 158 | tgg | Trp | tgg | Trp | tgg | Trp | tgg | Trp | |
| 159 | aac | Asn | aac | Asn | aac | Asn | aac | Asn | |
| 160 | tca | Ser | tca | Ser | tca | Ser | tca | Ser | |
| 161 | ggc | Gly | ggc | Gly | ggc | Gly | ggc | Gly | |
| 162 | gcc | Ala | gct | Ala | gcc | Ala | gcc | Ala | |
| 163 | ctg | Leu | ctg | Leu | ctg | Leu | ctg | Leu | |
| 164 | acc | Thr | acc | Thr | acc | Thr | acc | Thr | |
| 165 | agc | Ser | agc | Ser | agc | Ser | agc | Ser | |
| 166 | ggc | Gly | ggc | Gly | ggc | Gly | ggc | Gly | |
| 167 | gtg | Val | gtg | Val | gtg | Val | gtg | Val | |
| 168 | cac | His | cac | His | cac | His | cac | His | |

[Table 2]

| | | | | | | | | |
|---|---|---|---|---|---|---|---|---|
| 169 | acc | Thr | acc | Thr | acc | Thr | acc | Thr |
| 170 | ttc | Phe | ttc | Phe | ttc | Phe | ttc | Phe |
| 171 | ccg | Pro | cca | Pro | ccg | Pro | ccg | Pro |
| 172 | gct | Ala | gct | Ala | gct | Ala | gct | Ala |
| 173 | gtc | Val | gtc | Val | gtc | Val | gtc | Val |
| 174 | cta | Leu | cta | Leu | cta | Leu | cta | Leu |
| 175 | cag | Gln | cag | Gln | cag | Gln | cag | Gln |
| 176 | tcc | Ser | tcc | Ser | tcc | Ser | tcc | Ser |
| 177 | tca | Ser | tca | Ser | tca | Ser | tca | Ser |
| 178 | gga | Gly | gga | Gly | gga | Gly | gga | Gly |
| 179 | ctc | Leu | ctc | Leu | ctc | Leu | ctc | Leu |
| 180 | tac | Tyr | tac | Tyr | tac | Tyr | tac | Tyr |
| 181 | tcc | Ser | tcc | Ser | tcc | Ser | tcc | Ser |
| 182 | ctc | Leu | ctc | Leu | ctc | Leu | ctc | Leu |
| 183 | agc | Ser | agc | Ser | agc | Ser | agc | Ser |
| 184 | agc | Ser | agc | Ser | agc | Ser | agc | Ser |
| 185 | gtg | Val | gtg | Val | gtg | Val | gtg | Val |
| 186 | gtg | Val | gtg | Val | gtg | Val | gtg | Val |
| 187 | acc | Thr | acc | Thr | acc | Thr | acc | Thr |
| 188 | gtg | Val | gtg | Val | gtg | Val | gtg | Val |
| 189 | ccc | Pro | ccc | Pro | ccc | Pro | ccc | Pro |
| 190 | tcc | Ser | tcc | Ser | tcc | Ser | tcc | Ser |
| 191 | agc | Ser | agc | Ser | agc | Ser | agc | Ser |
| 192 | agc | Ser | aac | Asn | agc | Ser | agc | Ser |
| 193 | ttg | Leu | ttc | Phe | ttg | Leu | ttg | Leu |
| 194 | ggc | Gly | ggc | Gly | ggc | Gly | ggc | Gly |
| 195 | acc | Thr | acc | Thr | acc | Thr | acg | Thr |
| 196 | cag | Gln | cag | Gln | cag | Gln | aag | Lys |
| 197 | acc | Thr | acc | Thr | acc | Thr | acc | Thr |
| 198 | tac | Tyr | tac | Tyr | tac | Tyr | tac | Tyr |
| 199 | atc | Ile | acc | Thr | acc | Thr | acc | Thr |
| 200 | tgc | Cys | tgc | Cys | tgc | Cys | tgc | Cys |
| 201 | aac | Asn | aac | Asn | aac | Asn | aac | Asn |
| 202 | gtg | Val | gta | Val | gtg | Val | gta | Val |
| 203 | aat | Asn | gat | Asp | aat | Asn | gat | Asp |
| 204 | cac | His | cac | His | cac | His | cac | His |
| 205 | aag | Lys | aag | Lys | aag | Lys | aag | Lys |
| 206 | ccc | Pro | ccc | Pro | ccc | Pro | ccc | Pro |
| 207 | agc | Ser | agc | Ser | agc | Ser | agc | Ser |
| 208 | aac | Asn | aac | Asn | aac | Asn | aac | Asn |
| 209 | acc | Thr | acc | Thr | acc | Thr | acc | Thr |
| 210 | aag | Lys | aag | Lys | aag | Lys | aag | Lys |
| 211 | gtg | Val | gtg | Val | gtg | Val | gtg | Val |
| 212 | gac | Asp | gac | Asp | gac | Asp | gac | Asp |
| 213 | aag | Lys | aag | Lys | aag | Lys | aag | Lys |
| 214 | aaa | Lys | aca | Thr | aga | Arg | aga | Arg |
| | | | gtt | Val | gtt | Val | gtt | Val |
| 215 | gca | Ala | | | | | | |
| 216 | gag | Glu | gag | Glu | gag | Glu | gag | Glu |
| 217 | ccc | Pro | cgc | Arg | ctc | Leu | tcc | Ser |
| 218 | aaa | Lys | aaa | Lys | aaa | Lys | aaa | Lys |
| 219 | tct | Ser | | | acc | Thr | tat | Tyr |
| 220 | tgt | Cys | | | cca | Pro | ggt | Gly |
| 221 | gac | Asp | tgt | Cys | ctt | Leu | | |
| | | | tgt | Cys | ggt | Gly | | |

IgG1 constant region(Hing)

[Table 3]

| | | | | | | | | | |
|---|---|---|---|---|---|---|---|---|---|
| | | | | | gac | Asp | | | Human : |
| 222 | aaa | Lys | gtc | Val | aca | Thr | | | |
| 223 | act | Thr | | | act | Thr | | | |
| 224 | cac | His | gag | Glu | cac | His | ccc | Pro | |
| 225 | aca | Thr | | | aca | Thr | cca | Pro | |
| 226 | tgc | Cys | tgc | Cys | tgc | Cys | tgc | Cys | |
| 227 | cca | Pro | cca | Pro | cca | Pro | cca | Pro | |
| 228 | ccg | Pro | ccg | Pro | cgg | Arg | tca | Ser | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | ccg | Pro | | | |
| | | | | | tgc | Cys | | | |
| | | | | | cca | Pro | | | |
| | | | | | cgg | Arg | | | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | cca | Pro | | | |
| | | | | | tgc | Cys | | | |
| | | | | | cca | Pro | | | |
| | | | | | cgg | Arg | | | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | ccg | Pro | | | |
| | | | | | tgc | Cys | | | |
| | | | | | cca | Pro | | | |
| | | | | | agg | Arg | | | |
| 229 | tgc | Cys | tgc | Cys | tgc | Cys | tgc | Cys | |
| 230 | cca | Pro | cca | Pro | cca | Pro | cca | Pro | |

[Table 4]

| | | | | | | | | | |
|---|---|---|---|---|---|---|---|---|---|
| 231 | gca | Ala | gca | Ala | gca | Ala | gca | Ala | Human IgG1 constant region (CH2) |
| 232 | cct | Pro | cca | Pro | cct | Pro | cct | Pro | |
| 233 | gaa | Glu | cct | Pro | gaa | Glu | gag | Glu | |
| 234 | ctc | Leu | gtg | Val | ctc | Leu | ttc | Phe | |
| 235 | ctg | Leu | gca | Ala | ctg | Leu | ctg | Leu | |
| 236 | ggg | Gly | | | gga | Gly | ggg | Gly | |
| 237 | gga | Gly | gga | Gly | gga | Gly | gga | Gly | |
| 238 | ccg | Pro | ccg | Pro | ccg | Pro | cca | Pro | |
| 239 | tca | Ser | tca | Ser | tca | Ser | tca | Ser | |
| 240 | gtc | Val | gtc | Val | gtc | Val | gtc | Val | |
| 241 | ttc | Phe | ttc | Phe | ttc | Phe | ttc | Phe | |
| 242 | ctc | Leu | ctc | Leu | ctc | Leu | ctg | Leu | |
| 243 | ttc | Phe | ttc | Phe | ttc | Phe | ttc | Phe | |
| 244 | ccc | Pro | ccc | Pro | ccc | Pro | ccc | Pro | |
| 245 | cca | Pro | cca | Pro | cca | Pro | cca | Pro | |
| 246 | aaa | Lys | aaa | Lys | aaa | Lys | aaa | Lys | |
| 247 | ccc | Pro | ccc | Pro | ccc | Pro | ccc | Pro | |
| 248 | aag | Lys | aag | Lys | aag | Lys | aag | Lys | |
| 249 | gac | Asp | gac | Asp | gat | Asp | gac | Asp | |
| 250 | acc | Thr | acc | Thr | acc | Thr | act | Thr | |
| 251 | ctc | Leu | ctc | Leu | ctt | Leu | ctc | Leu | |
| 252 | atg | Met | atg | Met | atg | Met | atg | Met | |
| 253 | atc | Ile | atc | Ile | att | Ile | atc | Ile | |
| 254 | tcc | Ser | tcc | Ser | tcc | Ser | tcc | Ser | |
| 255 | cgg | Arg | cgg | Arg | cgg | Arg | cgg | Arg | |
| 256 | acc | Thr | acc | Thr | acc | Thr | acc | Thr | |
| 257 | cct | Pro | cct | Pro | cct | Pro | cct | Pro | |
| 258 | gag | Glu | gag | Glu | gag | Glu | gag | Glu | |
| 259 | gtc | Val | gtc | Val | gtc | Val | gtc | Val | |
| 260 | aca | Thr | acg | Thr | acg | Thr | acg | Thr | |
| 261 | tgc | Cys | tgc | Cys | tgc | Cys | tgc | Cys | |
| 262 | gtg | Val | gtg | Val | gtg | Val | gtg | Val | |
| 263 | gtg | Val | gtg | Val | gtg | Val | gtg | Val | |
| 264 | gtg | Val | gtg | Val | gtg | Val | gtg | Val | |
| 265 | gac | Asp | gac | Asp | gac | Asp | gac | Asp | |
| 266 | gtg | Val | gtg | Val | gtg | Val | gtg | Val | |
| 267 | agc | Ser | agc | Ser | agc | Ser | agc | Ser | |
| 268 | cac | His | cac | His | cac | His | cag | Gln | |
| 269 | gaa | Glu | gaa | Glu | gaa | Glu | gaa | Glu | |
| 270 | gac | Asp | gac | Asp | gac | Asp | gac | Asp | |
| 271 | cct | Pro | ccc | Pro | ccc | Pro | ccc | Pro | |
| 272 | gag | Glu | gag | Glu | gag | Glu | gag | Glu | |
| 273 | gtc | Val | gtc | Val | gtc | Val | gtc | Val | |
| 274 | aag | Lys | cag | Gln | cag | Gln | cag | Gln | |
| 275 | ttc | Phe | ttc | Phe | ttc | Phe | ttc | Phe | |
| 276 | aac | Asn | aac | Asn | aag | Lys | aac | Asn | |
| 277 | tgg | Trp | tgg | Trp | tgg | Trp | tgg | Trp | |
| 278 | tac | Tyr | tac | Tyr | tac | Tyr | tac | Tyr | |
| 279 | gtg | Val | gtg | Val | gtg | Val | gtg | Val | |
| 280 | gac | Asp | gac | Asp | gac | Asp | gat | Asp | |
| 281 | ggc | Gly | ggc | Gly | ggc | Gly | ggc | Gly | |
| 282 | gtg | Val | gtg | Val | gtg | Val | gtg | Val | |
| 283 | gag | Glu | gag | Glu | gag | Glu | gag | Glu | |
| 284 | gtg | Val | gtg | Val | gtg | Val | gtg | Val | |
| 285 | cat | His | cat | His | cat | His | cat | His | |

[Table 5]

| | | | | | | | | |
|---|---|---|---|---|---|---|---|---|
| 286 | aat | Asn | aat | Asn | aat | Asn | aat | Asn |
| 287 | gcc | Ala | gcc | Ala | gcc | Ala | gcc | Ala |
| 288 | aag | Lys | aag | Lys | aag | Lys | aag | Lys |
| 289 | aca | Thr | aca | Thr | aca | Thr | aca | Thr |
| 290 | aag | Lys | aag | Lys | aag | Lys | aag | Lys |
| 291 | ccg | Pro | cca | Pro | ccg | Pro | ccg | Pro |
| 292 | cgt | Arg | cgg | Arg | cgg | Arg | cgg | Arg |
| 293 | gag | Glu | gag | Glu | gag | Glu | gag | Glu |
| 294 | gag | Glu | gag | Glu | gag | Glu | gag | Glu |
| 295 | cag | Gln | cag | Gln | cag | Gln | cag | Gln |
| 296 | tac | Tyr | ttc | Phe | tac | Tyr | ttc | Phe |
| 297 | aac | Asn | aac | Asn | aac | Asn | aac | Asn |
| 298 | agc | Ser | agc | Ser | agc | Ser | agc | Ser |
| 299 | acg | Thr | acg | Thr | acg | Thr | acg | Thr |
| 300 | tac | Tyr | ttc | Phe | ttc | Phe | tac | Tyr |
| 301 | cgt | Arg | cgt | Arg | cgt | Arg | cgt | Arg |
| 302 | gtg | Val | gtg | Val | gtg | Val | gtg | Val |
| 303 | gtc | Val | gtc | Val | gtc | Val | gtc | Val |
| 304 | agc | Ser | agc | Ser | agc | Ser | agc | Ser |
| 305 | gtc | Val | gtc | Val | gtc | Val | gtc | Val |
| 306 | ctc | Leu | ctc | Leu | ctc | Leu | ctc | Leu |
| 307 | acc | Thr | acc | Thr | acc | Thr | acc | Thr |
| 308 | gtc | Val | gtt | Val | gtc | Val | gtc | Val |
| 309 | ctg | Leu | gtg | Val | ctg | Leu | ctg | Leu |
| 310 | cac | His | cac | His | cac | His | cac | His |
| 311 | cag | Gln | cag | Gln | cag | Gln | cag | Gln |
| 312 | gac | Asp | gac | Asp | gac | Asp | gac | Asp |
| 313 | tgg | Trp | tgg | Trp | tgg | Trp | tgg | Trp |
| 314 | ctg | Leu | ctg | Leu | ctg | Leu | ctg | Leu |
| 315 | aat | Asn | aac | Asn | aac | Asn | aac | Asn |
| 316 | ggc | Gly | ggc | Gly | ggc | Gly | ggc | Gly |
| 317 | aag | Lys | aag | Lys | aag | Lys | aag | Lys |
| 318 | gag | Glu | gag | Glu | gag | Glu | gag | Glu |
| 319 | tac | Tyr | tac | Tyr | tac | Tyr | tac | Tyr |
| 320 | aag | Lys | aag | Lys | aag | Lys | aag | Lys |
| 321 | tgc | Cys | tgc | Cys | tgc | Cys | tgc | Cys |
| 322 | aag | Lys | aag | Lys | aag | Lys | aag | Lys |
| 323 | gtc | Val | gtc | Val | gtc | Val | gtc | Val |
| 324 | tcc | Ser | tcc | Ser | tcc | Ser | tcc | Ser |
| 325 | aac | Asn | aac | Asn | aac | Asn | aac | Asn |
| 326 | aaa | Lys | aaa | Lys | aaa | Lys | aaa | Lys |
| 327 | gcc | Ala | ggc | Gly | gcc | Ala | ggc | Gly |
| 328 | ctc | Leu | ctc | Leu | ctc | Leu | ctc | Leu |
| 329 | cca | Pro | cca | Pro | cca | Pro | ccg | Pro |
| 330 | gcc | Ala | gcc | Ala | gcc | Ala | tcc | Ser |
| 331 | ccc | Pro | ccc | Pro | ccc | Pro | tcc | Ser |
| 332 | atc | Ile | atc | Ile | atc | Ile | atc | Ile |
| 333 | gag | Glu | gag | Glu | gag | Glu | gag | Glu |
| 334 | aaa | Lys | aaa | Lys | aaa | Lys | aaa | Lys |
| 335 | acc | Thr | acc | Thr | acc | Thr | acc | Thr |
| 336 | atc | Ile | atc | Ile | atc | Ile | atc | Ile |
| 337 | tcc | Ser | tcc | Ser | tcc | Ser | tcc | Ser |
| 338 | aaa | Lys | aaa | Lys | aaa | Lys | aaa | Lys |
| 339 | gcc | Ala | acc | Thr | gcc | Thr | gcc | Ala |
| 340 | aaa | Lys | aaa | Lys | aaa | Lys | aaa | Lys |

[Table 6]

| | | | | | | | | | |
|---|---|---|---|---|---|---|---|---|---|
| 341 | ggg | Gly | ggg | Gly | gga | Gly | ggg | Gly | Human IgG1 constant region (CH3) |
| 342 | cag | Gln | cag | Gln | cag | Gln | cag | Gln | |
| 343 | ccc | Pro | ccc | Pro | ccc | Pro | ccc | Pro | |
| 344 | cga | Arg | cga | Arg | cga | Arg | cga | Arg | |
| 345 | gaa | Glu | gaa | Glu | gaa | Glu | gag | Glu | |
| 346 | cca | Pro | cca | Pro | cca | Pro | cca | Pro | |
| 347 | cag | Gln | cag | Gln | cag | Gln | cag | Gln | |
| 348 | gtg | Val | gtg | Val | gtg | Val | gtg | Val | |
| 349 | tac | Tyr | tac | Tyr | tac | Tyr | tac | Tyr | |
| 350 | acc | Thr | acc | Thr | acc | Thr | acc | Thr | |
| 351 | ctg | Leu | ctg | Leu | ctg | Leu | ctg | Leu | |
| 352 | ccc | Pro | ccc | Pro | ccc | Pro | ccc | Pro | |
| 353 | cca | Pro | cca | Pro | cca | Pro | cca | Pro | |
| 354 | tcc | Ser | tcc | Ser | tcc | Ser | tcc | Ser | |
| 355 | cgg | Arg | cgg | Arg | cgg | Arg | cag | Gln | |
| 356 | gat | Asp | gag | Glu | gag | Glu | gag | Glu | |
| 357 | gag | Glu | gag | Glu | gag | Glu | gag | Glu | |
| 358 | ctg | Leu | atg | Met | atg | Met | atg | Met | |
| 359 | acc | Thr | acc | Thr | acc | Thr | acc | Thr | |
| 360 | aag | Lys | aag | Lys | aag | Lys | aag | Lys | |
| 361 | aac | Asn | aac | Asn | aac | Asn | aac | Asn | |
| 362 | cag | Gln | cag | Gln | cag | Gln | cag | Gln | |
| 363 | gtc | Val | gtc | Val | gtc | Val | gtc | Val | |
| 364 | agc | Ser | agc | Ser | agc | Ser | agc | Ser | |
| 365 | ctg | Leu | ctg | Leu | ctg | Leu | ctg | Leu | |
| 366 | acc | Thr | acc | Thr | acc | Thr | acc | Thr | |
| 367 | tgc | Cys | tgc | Cys | tgc | Cys | tgc | Cys | |
| 368 | ctg | Leu | ctg | Leu | ctg | Leu | ctg | Leu | |
| 369 | gtc | Val | gtc | Val | gtc | Val | gtc | Val | |
| 370 | aaa | Lys | aaa | Lys | aaa | Lys | aaa | Lys | |
| 371 | ggc | Gly | ggc | Gly | ggc | Gly | ggc | Gly | |
| 372 | ttc | Phe | ttc | Phe | ttc | Phe | ttc | Phe | |
| 373 | tat | Tyr | tac | Tyr | tac | Tyr | tac | Tyr | |
| 374 | ccc | Pro | ccc | Pro | ccc | Pro | ccc | Pro | |
| 375 | agc | Ser | agc | Ser | agc | Ser | agc | Ser | |
| 376 | gac | Asp | gac | Asp | gac | Asp | gac | Asp | |
| 377 | atc | Ile | atc | Ile | atc | Ile | atc | Ile | |
| 378 | gcc | Ala | gcc | Ala | gcc | Ala | gcc | Ala | |
| 379 | gtg | Val | gtg | Val | gtg | Val | gtg | Val | |
| 380 | gag | Glu | gag | Glu | gag | Glu | gag | Glu | |
| 381 | tgg | Trp | tgg | Trp | tgg | Trp | tgg | Trp | |
| 382 | gag | Glu | gag | Glu | gag | Glu | gag | Glu | |
| 383 | agc | Ser | agc | Ser | agc | Ser | agc | Ser | |
| 384 | aat | Asn | aat | Asn | agc | Ser | aat | Asn | |
| 385 | ggg | Gly | ggg | Gly | ggg | Gly | ggg | Gly | |
| 386 | cag | Gln | cag | Gln | cag | Gln | cag | Gln | |
| 387 | ccg | Pro | ccg | Pro | ccg | Pro | ccg | Pro | |
| 388 | gag | Glu | gag | Glu | gag | Glu | gag | Glu | |
| 389 | aac | Asn | aac | Asn | aac | Asn | aac | Asn | |
| 390 | aac | Asn | aac | Asn | aac | Asn | aac | Asn | |
| 391 | tac | Tyr | tac | Tyr | tac | Tyr | tac | Tyr | |
| 392 | aag | Lys | aag | Lys | aag | Lys | aag | Lys | |
| 393 | acc | Thr | acc | Thr | acc | Thr | acc | Thr | |
| 394 | acg | Thr | aca | Thr | acg | Thr | acg | Thr | |
| 395 | cct | Pro | cct | Pro | cct | Pro | cct | Pro | |

[Table 7]

| | | | | | | | | |
|---|---|---|---|---|---|---|---|---|
| 396 | ccc | Pro | ccc | Pro | ccc | Pro | ccc | Pro |
| 397 | gtg | Val | atg | Met | atg | Met | gtg | Val |
| 398 | ctg | Leu | ctg | Leu | ctg | Leu | ctg | Leu |
| 399 | gac | Asp | gac | Asp | gac | Asp | gac | Asp |
| 400 | tcc | Ser | tcc | Ser | tcc | Ser | tcc | Ser |
| 401 | gac | Asp | gac | Asp | gac | Asp | gac | Asp |
| 402 | ggc | Gly | ggc | Gly | ggc | Gly | ggc | Gly |
| 403 | tcc | Ser | tcc | Ser | tcc | Ser | tcc | Ser |
| 404 | ttc | Phe | ttc | Phe | ttc | Phe | ttc | Phe |
| 405 | ttc | Phe | ttc | Phe | ttc | Phe | ttc | Phe |
| 406 | ctc | Leu | ctc | Leu | ctc | Leu | ctc | Leu |
| 407 | tac | Tyr | tac | Tyr | tac | Tyr | tac | Tyr |
| 408 | agc | Ser | agc | Ser | agc | Ser | agc | Ser |
| 409 | aag | Lys | aag | Lys | aag | Lys | agg | Arg |
| 410 | ctc | Leu | ctc | Leu | ctc | Leu | cta | Leu |
| 411 | acc | Thr | acc | Thr | acc | Thr | acc | Thr |
| 412 | gtg | Val | gtg | Val | gtg | Val | gtg | Val |
| 413 | gac | Asp | gac | Asp | gac | Asp | gac | Asp |
| 414 | aag | Lys | aag | Lys | aag | Lys | aag | Lys |
| 415 | agc | Ser | agc | Ser | agc | Ser | agc | Ser |
| 416 | agg | Arg | agg | Arg | agg | Arg | agg | Arg |
| 417 | tgg | Trp | tgg | Trp | tgg | Trp | tgg | Trp |
| 418 | cag | Gln | cag | Gln | cag | Gln | cag | Gln |
| 419 | cag | Gln | cag | Gln | cag | Gln | gag | Glu |
| 420 | ggg | Gly | ggg | Gly | ggg | Gly | ggg | Gly |
| 421 | aac | Asn | aac | Asn | aac | Asn | aat | Asn |
| 422 | gtc | Val | gtc | Val | atc | Ile | gtc | Val |
| 423 | ttc | Phe | ttc | Phe | ttc | Phe | ttc | Phe |
| 424 | tca | Ser | tca | Ser | tca | Ser | tca | Ser |
| 425 | tgc | Cys | tgc | Cys | tgc | Cys | tgc | Cys |
| 426 | tcc | Ser | tcc | Ser | tcc | Ser | tcc | Ser |
| 427 | gtg | Val | gtg | Val | gtg | Val | gtg | Val |
| 428 | atg | Met | atg | Met | atg | Met | atg | Met |
| 429 | cat | His | cat | His | cat | His | cat | His |
| 430 | gag | Glu | gag | Glu | gag | Glu | gag | Glu |
| 431 | gct | Ala | gct | Ala | gct | Ala | gct | Ala |
| 432 | ctg | Leu | ctg | Leu | ctg | Leu | ctg | Leu |
| 433 | cac | His | cac | His | cac | His | cac | His |
| 434 | aac | Asn | aac | Asn | aac | Asn | aac | Asn |
| 435 | cac | His | cac | His | cgc | Arg | cac | His |
| 436 | tac | Tyr | tac | Tyr | ttc | Phe | tac | Tyr |
| 437 | acg | Thr | acg | Thr | acg | Thr | aca | Thr |
| 438 | cag | Gln | cag | Gln | cag | Gln | cag | Gln |
| 439 | aag | Lys | aag | Lys | aag | Lys | aag | Lys |
| 440 | agc | Ser | agc | Ser | agc | Ser | agc | Ser |
| 441 | ctc | Leu | ctc | Leu | ctc | Leu | ctc | Leu |
| 442 | tcc | Ser | tcc | Ser | tcc | Ser | tcc | Ser |
| 443 | ctg | Leu | ctg | Leu | ctg | Leu | ctg | Leu |
| 444 | tct | Ser | tct | Ser | tct | Ser | tct | Ser |
| 445 | ccg | Pro | ccg | Pro | ccg | Pro | ctg | Leu |
| 446 | ggt | Gly | ggt | Gly | ggt | Gly | ggt | Gly |
| 447 | aaa | Lys | aaa | Lys | aaa | Lys | aaa | Lys |

The cysteine of the present invention also includes the derivatives thereof. An example of the cysteine derivative of the present invention includes a compound in which a hydroxyl group, a methyl group, an ethyl group, a carboxyl group, an amino group, and the like are added to a cysteine molecule, a compound in which a part of an atom or a functional group constituting a cysteine molecule is deleted, and the like, but not limited thereto.

With respect to the ADCC activity evaluated in the lactate dehydrogenase release assay system, the ADCC activity of the antibody of the present invention is increased as compared with the wild type antibody (in two points in which the wild type antibody and the mutant type antibody have the same concentration, the ratio of cytotoxicity of the mutant type antibody to that of the wild type antibody is increased). Furthermore, the ADCC activities of a 286Cys type antibody, a 289Cys type antibody, a 305Cys type antibody, a 306Cys type antibody, a 307Cys type antibody, a 308Cys type antibody, a 290Cys type antibody, a 291Cys type antibody, a 292Cys type antibody, a 298Cys type antibody, a 302Cys type antibody, a 303Cys type antibody, and a 309Cys type antibody are increased in particularly low concentration by about two times or more, preferably, about five times or more, more preferably, about ten times or more, about 100 times or more, further preferably, about 500 timer or more, and particularly preferably about 1000 times or more (in two points in which the wild type antibody and the mutant type antibody have the same cytotoxicity rate, the concentration of the mutant type antibody to that of the wild type antibody is increased).

Furthermore, the antibody of the present invention has an improved stability, solubility, binding affinity to Fc receptor, or a CDC activity. It is preferable that the stability, solubility, binding affinity to Fc receptor, or a CDC activity of the antibody of the present invention is not changed or is increased as compared with those of the wild type. In the antibody of the present invention, at least the ADCC activity may be increased. However, the antibody of the present invention may have at least an enhanced ADCC activity, and therefore, an antibody whose stability, solubility, binding affinity to Fc receptor, or a CDC activity is reduced is also included in the antibody of the present invention. Note here that an example of the Fc receptor may include FcγRI, FcγRII, FcγRIII, FcRn, and the like, but are not limited thereto.

The antibody of the present invention includes both a polyclonal antibody and a monoclonal antibody. The preparation and purification method of the monoclonal antibody and the polyclonal antibody are known in this field and described in, for example, Harlow and Lane, Antibodies: A Laboratory Manual (New York: Cold Spring Harbor Laboratory Press, 1988).

One embodiment of the antibody of the present invention is a humanized antibody. The "humanized antibody" herein denotes an antibody that is constructed to have a structure similar to that of a human antibody and includes a human type chimeric antibody (for example, an antibody in which a part of the antibody is humanized, an antibody in which a CH2 region is humanized, an antibody in which a Fc region is humanized, and an antibody in which a constant region is humanized), a human type CDR-grafted antibody in which a part other than CDR (complementarity determining region) existing in the constant region and the variable region is humanized (P. T. Johons et al., Nature 321,522(1986)), and complete humanized antibody, and the like. In order to enhance the antigen binding activity of the human type CDR-grafted antibody, improved technologies of a method of selecting human antibody FR with high homology to mouse antibody, a method of preparing a humanized type antibody with high homology, a method of transplanting a mouse CDR into a human antibody and then substituting the amino acid of the FR region have been developed (see, for example, US patent No. 5585089, US patent No. 5693761, US patent No. 5693762, US patent No. 6180370, European patent No. 451216, European patent No. 682040, patent No. 2828340), and can be used for preparing a human type antibody of the present invention.

The human type chimeric antibody can be produced by, for example, by substituting the constant region of an antibody having the structure of the H-chain variable region and/or the structure of the L-chain variable region by the constant region of the human antibody. As the constant region of the human antibody, the well-known antibodies can be employed. Hereinafter, one example of the method of producing a human type chimeric antibody is described.

Firstly, mRNA is extracted from a hybridoma producing a mouse antibody against a specific antigen, and cDNA is synthesized according to the routine method. The synthesized cDNA is incorporated into a vector so as to construct a cDNA library. From this cDNA library, by using H-chain gene fragment and L-chain gene fragment as a probe, a vector containing the H-chain gene and the L-chain gene is selected. By carrying out sequencing of the insertion sequence of the selected vector, the sequence of the H-chain variable region and the L-chain variable region is determined. Based on the thus obtained sequence data, DNA encoding the H-chain variable region is produced by chemical synthesis, biochemical cutting / recombination, and the like. DNA encoding the obtained H-chain variable region is ligated with DNA encoding the human H-chain constant region, which is incorporated into an expression vector. Thus, the H-chain expression vector is produced. An example of the expression vector may include a SV40 virus based vector, an EB virus based vector, a BPV (papilloma virus) based vector, and the like, but the expression vector is not limited thereto. On the other hand, the L-chain expression vector is produced by the same method. These H-chain expression vector and L-chain expression vector co-transform a host cell. As the host cell, a CHO (Chinese hamster ovary) cell (A. Wright & S. L. Morrison, J. Immunol. 160, 3393-3402 (1998)), SP2/0 cell (mouse myeloma) (K. Motmans et al., Eur. J. Cancer Prev. 5, 512-519 (1996), R. P. Junghans et al., Cancer Res. 50, 1495-1502 (1990)), and the like, can be suitably used. Furthermore, for transformation, a lipofectin method (R. W. Malone et al., Proc. Natl. Acad. Sci. USA 86,6077 (1989), P. L. Felgner et al., Proc. Natl. Acad. Sci. USA 84,7413 (1987), an electroporation method, calcium phosphate method (F. L. Graham & A. J. van der Eb, Virology 52,456-467 (1973)), a DEAE-Dextran method and the like can be suitably used.

After the transformant is cultured, human type chimeric antibodies are separated from the transformant cell or the culture solution. For separation and purification of antibodies, any appropriate combination of centrifugation, ammonium sulfate fractionation, salting out, ultra-filtration, affinity chromatography, ion exchange chromatography, gel filtration chromatography, protein A column chromatography, protein G column chromatography, protein L column chromatography, and the like, can be used.

On the other hand, a human-type CDR graft antibody can be prepared, for instance, as the following manner. First of all, by the method described in the above-described preparation method of the chimeric antibody, an amino acid sequence of an H chain variable region and an L variable region of an antibody against a specific antigen and a base sequence encoding thereof. In addition, an amino acid sequence and a base sequence of each CDR region are determined.

Next, FR (framework region) that is present surrounding the CDR region is selected. As a method for selecting FR, approximately three methods can be employed. First method is a method of using a human antibody frame such as NEWM, REI, and the like, whose three dimensional structure has been clarified (Riechmann L. et al., Nature 332, 323-3Z7 (1988); Tempst, PR. et al., Protein Engineering 7, 1501-1507 (1994); Ellis JH. etal., J. Immunol 155, 925-937 (1995)). The second method is a method of selecting the human antibody variable region having the highest homology to the objective mouse antibody variable region from database, and using the FR thereof (Queen C. et al., Proc Natl Acad SciUSA 86, 10029-10033 (1989); Rozak MJ. et al., J Biol Chem 271, 22611-22618 (1996); Shearman CW. et al., J.Immunol 147, 4366-4373 (1991)). The third method is a method of selecting amino acid used most commonly in the FR of the human antibody (Sato K. et al., Mol Immunol 31, 371-381 (1994); Kobinger F. et al., Protein Engineering 6, 971-980 (1993); Kettleborough CA. et al., Protein Engineering 4, 773-783 (1991)). In the present invention, any one of these methods can be used.

Note here that an amino acid sequence in which the amino acid sequence of the human FR is modified can be also used as the amino acid sequence of FR as long as a finally produced human type CDR-grafted antibody has a specific binding property against a subject antigen. In particular, when a part of the amino acid of the selected human FR is changed into the amino acid of FR of the antibody that is an origin of the CDR, the probability that the property of the antibody can be maintained is high. The number of amino acids to be modified is not more than 30% with respect to an entire FR. Further preferably, the number is not more than 20% with respect to an entire FR. Yet further preferably, the number is not more than 10% with respect to an entire FR.

Next, by combining the FR selected by any of the methods mentioned above and the above-mentioned CDR, DNA encoding the H-chain variable region and the L-chain variable region is designed. Based on this design, DNA encoding the H-chain variable region and DNA encoding the L-chain variable region are produced by chemical synthesis, biochemical cutting / recombination, and the like, respectively. DNA encoding the H-chain variable region is incorporated into an expression vector together with DNA encoding the human immunoglobulin H-chain constant region. Thus, the H-chain expression vector is constructed. Similarly, DNA encoding the L-chain variable region is incorporated into an expression vector together with DNA encoding the human immunoglobulin L-chain constant region. Thus, the L-chain expression vector is constructed. An example of the expression vector may include a SV40 virus based vector, an EB virus based vector, a BPV (papilloma virus) based vector, and the like. However, the expression vector is not limited thereto.

The H-chain expression vector and L-chain expression vector, which have been produced by the above-mentioned method, co-transform a host cell. As the host cell, a CHO (Chinese hamster ovary) cell (A. Wright & S. L. Morrison, J. Immunol. 160, 3393-3402 (1998)), an SP2/0 cell (mouse myeloma) (K. Motmans et al., Eur. J. Cancer Prev. 5, 512-519 (1996), R. P. Junghans et al., Cancer Res. 50, 1495-1502 (1990)), and the like, can be preferably used. Furthermore, for transformation, a lipofectin method (R. W. Malone et al., Proc. Natl. Acad. Sci. USA 86,6077 (1989), P. L. Felgner et al., Proc. Natl. Acad. Sci. USA 84,7413 (1987), an electroporation method, a calcium phosphate method (F. L. Graham & A. J. van der Eb, Virology 52,456-467 (1973)), a DEAE-Dextran method and the like can be preferably used.

After the transformant is cultured, human type CDR-grafted antibodies are separated from the transformant cell or the culture solution. For separation and purification of antibodies, any appropriate combination of centrifugation, ammonium sulfate fractionation, salting out, ultra-filtration, affinity chromatography, ion exchange chromatography, gel filtration chromatography, protein A column chromatography, protein G column chromatography, protein L column chromatography, and the like, can be used.

Furthermore, a method for obtaining a human antibody is well known. For example, human lymphocyte is sensitized with a desirable antigen or a cell expressing a desirable antigen in vitro, and the sensitized lymphocyte is fused to a human myeloma cell, for example, U266 so as to obtain a desired human antibody having a binding activity to an antigen can be obtained (see, Japanese Patent Examined No. H1-59878). Furthermore, a transgenic animal having all repertoires of human antibody genes is immunized with a desirable antigen, and thereby a desired human antibody can be obtained (see, International Publication Nos. WO 93/12227, WO 92/03918, WO 94/02602, WO 94/25585, WO 96/34096, and WO 96/33735).

In further embodiment, an antibody or an antibody fragment can be separated from an antibody phage library by using a technology described in McCafferty et al. (Nature, 348: 552-554 (1990)). Clackson et al. (Nature, 352: 624-628 (1991)) and Marks et al. (J. Mol. Biol., 222: 581-597 (1991)) describe separation between a mouse antibody and a human antibody using a phage library. The following publication describes the generation of a human body with high affinity (nM range) by chain shuffling (Marks et al, Bio/Technology, 10: 779-783 [1992]), as well as combinatorial infection and in vivo recombination as a strategy for constructing an extremely large phage library (Waterhouse et al, Nuc. Acids. Res., 21: 2265-2266 [1993]). Therefore, these technologies are executable methods for separating a monoclonal antibody with relative to a conventional monoclonal antibody hybridoma method.

In this point, a bacteriophage (phage) display is one of the well-known technologies capable of searching a large oligopeptide library and identifying these library members that can be specifically bonded to a polypeptide target. The phage display is a technology in which various polypeptides are presented on a coat protein on the surface of the bacteriophage particle as a fused protein (Scott, J. K. and Smith G. P. (1990) Science 249: 386). The usefulness of the phage display is that it is possible to rapidly and effectively classify the sequences in which the large library of selectively randomized protein mutants (or random clone cDNA) are bonded to a target molecule with high affinity. Display of peptide (Cwirla, S.E. et al. (1990) Proc. Natl. Acad. Sci. USA, 87: 6378) or protein (Lowman, H. B. et al. (1991) Biochemistry, 30: 10832; Clackson, T. et al. (1991) Nature, 352: 624; Marks, J. D. et al. (1991), J. Mol. Biol., 222:581; Kang, A. S. et al. (1991) Proc. Natl. Acad. Sci. USA, 88: 8363) libraries in phage are used for screening a large number of polypeptides or oligopeptides which have specific bonding property (Smith, G. P. (1991) Current Opin. Biotechnol., 2: 668). The classification of phage libraries of random mutants requires a method for constructing and proliferating a large number of mutants, a method of purifying affinity using a target receptor, and a means for evaluating a result of the enhancement of binding (see, U.S. Patent No. 5223409, No. 5403484, No. 5571689, and No. 5663143).

Although most of phage display methods use fibrous phage, λ phage display system (WO95/34683;U.S. Patent No. 5627024), T4 phage display system (Ren J. et al., Gene 215: 439 (1998); Zhu et al., Cancer Researc, 58 (15): 3209-3214 (1998); Jiang et al., Infection & Immunity, 65 (11): 4770-4777 (1997); Ren et al., Gene, 195 (2): 303-311 (1997); Ren, Protein Sci. 5: 1833 (1996); Efimov et al., VirusGenes 10: 173 (1995) and T7 phage display system (Smith and Scott Methods in Enzymology, 217, 228-257 (1993); U.S. Patent No. 5766905) are also known.

Currently, with respect to basic phage display method, much improvement and variations have been developed. With such improvement, a binding property to a selected target molecule, and the like, a screening method from the peptide library or the protein library based on the properties and ability have been improved. Recombinant reaction means for phage display methods is described in WO 98/14277. The phage display library is used for analyzing and controlling the interaction between two molecules (WO 98/20169; WO 98/20159) and restrictive helix peptide property (WO 98/20036). WO 97/35196 describes a method for isolating an affinity ligand by bringing a first solution in which ligand can bind to the target molecule and a second solution in which an affinity ligand does not bind to a target molecule, and the phage display library into contact with each other. WO 97/46251 describes a method for separating high affinity binding phase by bio-panning a random phage display library with affinity purification antibody, isolating a binding phage, then, micro-panning thereof on the well of the micro plate. The use as an affinity tag of Staphlylococcus aureus protein A has been reported (Li et al., (1998) Mol Biotech., 9: 187). WO97/47314 describes the use of substrate subtraction library for recognizing an enzyme specificity by using a combinatorial library that may be a phage display library. A method for selecting an enzyme suitable for the use in a washing agent used in the phage display is described in WO 97/09446. A method for selecting protein specifically bound is described in U. S. Patent No. 5498538, No. 5432018, and WO 98/15833. A production method of a peptide library is described in U. S. Patent No. 5723286, No. 5432018, No. 5580717, No. 5427908, No. 5498530, No. 5770434, No. 5734018, No. 5698426, No. 5763192, and No. 5723323.

A technology for obtaining a human antibody by panning by using a human antibody library. For example, a variable region of the human antibody can be expressed as a single chain antibody (scFv) on the surface of the phage by a phage display method so as to select a phage bound to an antigen. By analyzing the gene of the selected phage, a DNA sequence of the variable region encoding a human antibody bonded to an antigen can be determined. When the DNA sequence of scFv bonded to an antigen is clarified, an expression vector having the sequence can be produced. The vector is introduced and expressed in a suitable host, and thereby a human antibody can be obtained. These methods are already known and can be executed with the reference to WO 92/01047, WO 92/20791, WO 93/06213, WO 93/11236, WO 93/19172, WO 95/01438, and WO 95/15388.

Alternatively, by using a phage display technology (McCafferty et al., Nature 348:552-553 [1990]), a human antibody and an antibody fragment can be produced in vitro from an immunoglobulin variable (V) domain gene repertoire of a non-immunized donor. According to this technique, an antibody V domain gene is cloned by a fibrous bacteriophage, for example, an M13 phage or an fd coat protein gene in each frame unit so as to be presented as a functional antibody fragment on the surface of the phage particle. Since fibrous particles contain single strand DNA copy of a phage genome, also based on the functional property of an antibody, as a result, a gene encoding an antibody showing these properties can be selected. Therefore, this phage mimics some properties of B cells. The phage display can be carried out in various forms (see, for example, Johnson, Kevin S. and Chiswell, David J., Current Opinion in Structural Biology 3: 564-571 (1993)). Some supply sources of V-gene segments can be used as a phage display. According to Clackson et al., Nature, 352:624-628 (1991), from small random combinatorial library of the V genes derived from immunized mouse spleen, various anti-oxazolone antibodies are isolated. A repertoire of V genes of a non-immunized human donor can be constructed. Antibodies to various kinds of many antigens (including autoantigens) can be isolated according to the technology Marks et al., J. Mol. Biol. 222:581-597 (1991), or Griffith, EMBO J. 12:725-734 (1993). Furthermore, see U.S. Patent No.5565332 and No. 5573905.

The antibody of the present invention also includes a fused protein obtained by fusing the antibody of the present invention and other peptide or protein. In a method for producing the fused protein, polynucleotide encoding the antibody of the present invention and polynucleotide encoding other peptide or polypeptide may be linked to each other so that the frames match to each other and the linked polynucleotide is introduced into an expression vector and allowed to express in the host. A technique known to a person skilled in the art can be used. As the other peptide or polypeptide to be fused with the antibody of the present invention, for example, well-known peptides including, for example, FLAG (Hopp, T. P. et al., BioTechnology (1988) 6, 1204-1210 ), 6 × His consisting of six His (histidine) residues, 10 × His, influenza agglutinin (HA), human c-myc fragment, VSV-GP fragment, p18HIV fragment, T7-tag, HSV-tag, E-tag, SV40T antigen fragments, lck tag, α-tubulin fragment, B-tag, Protein C fragment and the like can be used. Furthermore, as the other polypeptides to be used for fusing the antibody of the present invention, for example, GST (glutathione-S-transferase), HA (influenza agglutinin), β-galactosidase, MBP (maltose binding protein ), and the like, can be used.

Furthermore, in the antibody of the present invention, it is possible to provide and modify a sugar chain, or substitute it with a fucose deleted sugar chain. Provision of a sugar chain to the antibody can be carried out by binding an -SH group in cysteine into which mutation has been introduced and a bromoacetyl group introduced into a sugar chain to each other and introducing the sugar chain into an antibody. By providing a sugar chain, peptide or polypeptide, an effector function of antibody can be improved. The effector function may include an ADCC activity and complement-independent cytotoxicity (CDC) activity but not limited thereto.

In addition, the antibody of the present invention include an antibody in which a part of amino acids have been subjected to chemical modification such as acetylation, formation into PEG, phosphorylation, and amidation. Such a chemical modification can be carried out by a method known to a person skilled in the art.

The antibody of the present invention can be used in combination with a technology for improving other effector functions. The technology for improving other effector functions may include a technology for producing by using culture cells such as CHO cells in which a fucose transferase is knocked out or knocked down (WO 2003/085119), or a technology for substituting an amino acid residue in a heavy chain of an antibody with an amino acid other than an original amino acid (WO 2004/029207 and WO 2000/042072) but not limited thereto.

The antibody of the present invention is **characterized in that** it binds to FcγRI, FcγRII, or FcγRIII on the surface of the effector cell. The effector function is also mediated by the interaction between an Fc receptor (FcR) and an Fc region of an antibody. An Fc receptor is known as a differentiated cell surface receptor existing on the hematopoietic cell. Among Fc receptors, an Fc receptor to an IgG antibody is called FcγR, an Fc receptor to an IgE is called FcεR, an Fc receptor to an IgA is called FcαR. FcR is defined as an immunoglobulin isotype. The Fcγ receptor is confirmed to have three subclasses: FcγRI (CD64), FcγRII (CD32) and FcγRIII (CD16). Since each FcγR sub-class is encoded by two or three genes and selective RNA splicing provides a plurality of transcripts, a wide diversity in the FcγR isoforms are present. Three genes encoding FcγRI subclasses (FcγRIA, FcγRIB, and FcγRIC) form a cluster in a region 1q21.1 of the first chromosome long arm, a gene encoding FcγRII isoforms (FcγRIIA, FcγRIIB, and FcγRIIC) and two genes encoding FcγRill (FcγRIIIA and FcγRIIIB) form a cluster in a region 1q22. FcR is described in Ravetch and Kinet, Annu. Rev. Immunol 9:457-92 (1991); Capel et al., Immunomethods 4: 25-34 (1994); and de Haas et al., J Lab. Clin. Med. 126: 330-41 (1995).

FcγRI binds to a monomer IgG with high affinity. On the other hand, FcγRII and FcγRIII, which are low-affinity receptors, interact with composite type or massive type IgG. As a classical method for detecting these low-affinity receptors, "rosette formation" using includes erythrocyte (EA) covered with antibody sensitized with IgG is known (Bredius et al. Immunology 83: 624-630 (1994). Furthermore, with respect to a rosette assay, see, Tax et al. J. Immunol. 133 (3): 1185-1189 (1984); Nagarajan et al. J. Biol. Chem. 270 (43): 25762-25770 (1995); and Warmerdam et al. J. Immunol. 147(4): 1338-1343 (1991)).

In the present invention, the effector cells are not particularly limited but may include, for example, PBMC.

Furthermore, the present invention provides nucleic acid encoding the antibody of the present invention, a vector into which the nucleic acid is inserted, and a transformed cell into which the vector is introduced. An example of the vector may include an M13 vector, pUC vector, pBR322, pBluescript, pCR-Script, and the like. Furthermore, for the purpose of sub-cloning or cutting of cDNA, an example of the vector may include pGEM-T, pDIRECT, pT7, and the like, in addition to the above-mentioned vector.

As an expression vector, when it is expressed in, for example, Escherichia coli, a vector has the above-mentioned features that it is amplified in Escherichia coli. Besides, when a host is Escherichia coli such as JM109, DH5α, HB101, and XL1-Blue, it is essential to have a promoter for efficiently expressing in Escherichia coli, for example, a lacZ promoter (Ward et al., Nature (1989) 341, 544-546; FASEB J. (1992) 6, 2422-2427), an araB promoter (Better et al., Science (1988) 240, 1041-1043), a T7 promoter, or the like. An example of such a vector includes pGEX-5X-1 (Pharmacia), "QIAexpress system" (QIAGEN), pEGFP, or pET (in this case, it is preferable that a host is BL21 expressing T7 RNA polymerase), and the like, in addition to the above-mentioned vector.

Furthermore, the vector may include a signal sequence for secreting polypeptide. As the signal sequence for secreting protein, when it is expressed in periplasm of Escherichia coli, a pelB signal sequence may be used (Lei, S. P. et al J. Bacteriol. (1987) 169, 4379). The vector can be introduced into a host cell by, for example, a calcium chloride method, an electroporation method.

Other than Escherichia coli, an expression vector includes, for example, expression vectors derived from mammalian (for example, pcDNA3 (Invitrogen), pEGF-BOS (Nucleic Acids. Res.1990, 18(17), p5322), pEF, pCDM8), expression vectors derived from insect (for example, "Bac-to-BAC baculovairus expression system" (GIBCO BRL), pBacPAK8), example, expression vectors derived from plant (for example, pMH1 and pMH2), expression vectors derived from animal virus (for example, pHSV, pMV, and pAdexLcw), expression vectors derived from retrovirus (for example, pZIPneo), expression vectors derived from yeast (for example, "Pichia Expression Kit" (Invitrogen), pNV11, SP-Q01), and expression vectors derived from Bacillus subtilis (for example, pPL608 and pKTH50).

When a vector is intended to be expressed in animal cells such as CHO, COS, NIH3T3 cells, it is essential that a promoter necessary for expression in a cell, for example, a vector has an SV40 promoter (Mulligan et al., Nature (1979) 277, 108), an MMTV-LTR promoter, an EF1$\alpha$ promoter (Mizushima et al., Nucleic Acids Res. (1990) 18, 5322), a CMV promoter, and the like. It is preferable to have a gene for selecting the transformation into cells (for example, a drug resistance gene capable of being determined by drugs (neomycin, G418, puromycin, and the like)). An example of a vector having such a property may include pMAM, pDR2, pBK-RSV, pBK-CMV, pOPRSV, pOP13, ahd the like.

Furthermore, for the purpose of stably expressing a gene and amplifying the number of gene copies in the cell, a method of introducing a vector having a DHFR gene complimentary to the CHO cell (for example, pCHOI) into a CHO cell in which a nucleic acid synthesizing passage is deleted, and amplifying it with methotrexate (MTX) can be carried out. Furthermore, for the purpose of transient expression of a gene, a method for transforming a vector having a replication origin of SV40 (pcD and the like) by using a COS cell having a gene for expression SV40 T antigen on the chromosome can be carried out. As the replication origin, origins of polyoma virus, adenovirus, bovine papilloma virus (BPV), and the like, may be used. Furthermore, in order to amplify the number of gene copies in a host cell system, an expression vector can include an aminoglycoside transferase (APH) gene, a thymidine kinase (TK) gene, an Escherichia coli xanthine guanine phosphoribosyl transferase (Ecogpt) gene, a dihydrofolate reductase (dhfr) gene, and the like, as a selection marker.

A host cell into which a vector is introduced is not particularly limited. For example, various kinds of animal cells such as Escherichia coli can be used. The host cell can be used for manufacture or expression as a production system of the antibody of the present invention. The production system for production or expression of polypeptide includes in vitro and in vivo production systems. The in vitro production system includes a production system using a eukaryotic cell and a production system using a prokaryotic cell.

When a eukaryotic cell is used, for example, an animal cell, a plant cell, and a fungal cell can be used as a host. An example of the animal cell includes a mammalian cell, for example, CHO (J. Exp. Med. (1995) 108, 945), COS, 3T3, myeloma, BHK (baby hamster kidney), HeLa, Vero, amphibian cell such as Xenopus laevis oocyte (Valle, et al., Nature (1981) 291, 358-340), or insect cell such as Sf9, Sf21, and Tn5. In the present invention, CHO-DG44, CHO-DXB11, COS7 cell, and BHK cell are suitably used. In animal cells, for the purpose of massively expression, in particular, CHO cells are preferable. A host cell can be introduced into a vector by the potassium phosphate method, DEAE dextran method, a method using cationic ribosome DOTAP (Boehringer Mannheim), an electroporation method, lipofection method, and the like.

As a plant cell, for example, cells derived from Nicotiana tabacum is known as a protein production system and this may be subjected to callus culture. As the fungal cell, yeast such as Saccharomyces, for example, Saccharomyces cerevisiae and Saccharomyces pombe, filamentous fungi such as Aspergillus, for example, Aspergillus niger are well known.

When a prokaryotic cell is used, a production system using a bacterial cell is used. An example of the bacterial cell includes Escherichia coli (E. coli), for example, JM109, DH5$\alpha$, HB101, and the like. Besides, Bacillus subtilis is known. Cells transformed by DNA of the present invention is cultured in vitro and purified by a routine method carried out by a person skilled in the art, and thereby the antibody of the present invention can be obtained.

Furthermore, the present invention provides a host organism having a vector containing nucleic acid encoding the antibody of the present invention. The host organism of the present invention is useful for production of a recombinant antibody. An example of the host organism in the present invention includes a goat, and the like. For example, a transgenic goat of the present invention can be carried out as follows. That is to say, a fusion gene inserted into a gene encoding protein in frame (e.g. goat $\beta$ casein) in which an antibody gene is intrinsically produced is constructed. When a DNA fragment containing a fusion gene into which an antibody gene is inserted is infused into an embryo of a goat, the infused embryo is introduced into a female goat. From milk produced by a transgenic goat created by a goat receiving an embryo or its progeny, the antibody of the present invention can be obtained. In order to increase the amount of milk including the antibody of the present invention produced from the transgenic goat, hormone can be appropriately used (Ebert, K.M. et al., Bio/Technology (1994) 12, 699-702).

It is preferable that the antibody of the present invention is an antibody that recognizes a molecule existing on the surface of a target cell to which cytotoxicity is intended to be given. The target cell to which cytotoxicity is intended to be given is preferably derived from human but not limited thereto. Furthermore, a target cell to which cytotoxicity is intended to be given is not particularly limited and an example of the cell may include cancer cells, Raji, KG-1a, TL-1, HUT78, Jurkat, BALL-1, HEPG2, MKN-7, KB or Hela but not limited thereto. An example of the molecules (antigen) existing on the surface of the target cell to which cytotoxicity is intended to be given may include a target antigen selected from the group consisting of CD20, CD22, CD33, CD52, Her2/neu, EGFR, EpCAM, MUC1, GD3, CEA, CA125, HLA-DR, TNF alpha and VEGF, but the example is not limited thereto.
A 290Cys type anti-CD20 antibody may specifically include the following antibodies but are not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 95 as CH;
(2) an antibody including an H chain having an amino acid sequence set forth in SEQ ID NO: 93;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 97 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 291 Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 101 as CH;
(2) an antibody including an H chain having an amino acid sequence set forth in SEQ ID NO: 99;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 103 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 292Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 107 as CH;
(2) an antibody including an H chain having an amino acid sequence set forth in SEQ ID NO: 105;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 109 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 293Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 113 as CH;
(2) an antibody including an H chain having an amino acid sequence set forth in SEQ ID NO: 111;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 115 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 294Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 54 as CH;
(2) an antibody including an H chain having an amino acid sequence set forth in SEQ ID NO: 45;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 73 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 298Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 83 as CH;
(2) an antibody including an H chain having an amino acid sequence set forth in SEQ ID NO: 81;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 299Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 119 as CH;
(2) an antibody including an H chain having an amino acid sequence set forth in SEQ ID NO: 117;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 121 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 300Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 125 as CH;
(2) an antibody including an H chain having an amino acid sequence set forth in SEQ ID NO: 123;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 127 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 301Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 89 as CH;
(2) an antibody including an H chain having an amino acid sequence set forth in SEQ ID NO: 87;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 91 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 302Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 131 as CH;
(2) an antibody including an H chain having an amino acid sequence set forth in SEQ ID NO: 129;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 133 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 303Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 137 as CH;
(2) an antibody including an H chain having an amino acid sequence set forth in SEQ ID NO: 135;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 139 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 304Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 143 as CH;
(2) an antibody including an H chain having an amino acid sequence set forth in SEQ ID NO: 141;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 145 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 286Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 149 as CH;
(2) an antibody including an H chain having an amino acid sequence set forth in SEQ ID NO: 147;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 151 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 287Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 155 as CH;
(2) an antibody including an H chain having an amino acid sequence set forth in SEQ ID NO: 153;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 157 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 288Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 161 as CH;
(2) an antibody including an H chain having an amino acid sequence set forth in SEQ ID NO: 159;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 163 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 289Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 167 as CH;
(2) an antibody including an H chain having an amino acid sequence set forth in SEQ ID NO: 165;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 169 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 305Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 173 as CH;
(2) an antibody including an H chain having an amino acid sequence set forth in SEQ ID NO: 171;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 175 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 306Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 179 as CH;
(2) an antibody including an H chain having an amino acid sequence set forth in SEQ ID NO: 177;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 181 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 307Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 185 as CH;
(2) an antibody including an H chain having an amino acid sequence set forth in SEQ ID NO: 183;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 187 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 308Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 191 as CH;
(2) an antibody including an H chain having an amino acid sequence set forth in SEQ ID NO: 189;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 193 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 309Cys type anti-CD20 antibody may specifically include the following antibodies but not limited thereto.

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 243 as CH;
(2) an antibody including an H chain having an amino acid sequence set forth in SEQ ID NO: 241;
(3) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 245 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

A 298Cys type anti-EGFR antibody may specifically include the following antibodies but not limited thereto.

(1) an antibody including an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 83 as CH;
(2) an antibody including an H chain having an amino acid sequence set forth in SEQ ID NO: 209;
(3) an antibody including an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 203 as CDR1, an amino acid sequence set forth in SEQ ID NO: 205 as CDR2, an amino acid sequence set forth in SEQ ID NO: 207 as CDR3, and an amino acid sequence set forth in SEQ ID NO: 247 as CL; and
(ii) an L chain having an amino acid sequence set forth in SEQ ID NO: 201.

As mentioned above, the antibody of the present invention also includes a recombinant antibody such as a humanized antibody. The humanized antibody includes a chimeric (in particular, a human-type chimeric antibody), and a CDR graft antibody (in particular, a human-type CDR graft antibody). The specific description of these antibodies are described above.
Furthermore, the present invention provides a 290Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 94,
(2) an antibody including an H chain encoded by a base sequence set forth in SEQ ID NO: 92,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 96,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides a 291 Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 100,
(2) an antibody including an H chain encoded by a base sequence set forth in SEQ ID NO: 98,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 102,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides a 292Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 106,
(2) an antibody including an H chain encoded by a base sequence set forth in SEQ ID NO: 104,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 108,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, DNA encoding a 293Cys type anti-CD20 antibody includes the following DNAs. Furthermore, the present invention provides 293Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 112,
(2) an antibody including an H chain encoded by a base sequence set forth in SEQ ID NO: 110,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 114,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 294Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 52,
(2) an antibody including an H chain encoded by a base sequence set forth in SEQ ID NO: 44,

(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 72,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 298Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 82,
(2) an antibody including an H chain encoded by a base sequence set forth in SEQ ID NO: 80,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 84,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 299Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 118,
(2) an antibody including an H chain encoded by a base sequence set forth in SEQ ID NO: 116,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 120,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 300Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 124,
(2) an antibody including an H chain encoded by a base sequence set forth in SEQ ID NO: 122,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 126,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 301 Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 88,
(2) an antibody including an H chain encoded by a base sequence set forth in SEQ ID NO: 86,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 90,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 302Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 130,
(2) an antibody including an H chain encoded by a base sequence set forth in SEQ ID NO: 128,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 132,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 303Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 136,
(2) an antibody including an H chain encoded by a base sequence set forth in SEQ ID NO: 134,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 138,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 304Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 142,
(2) an antibody including an H chain encoded by a base sequence set forth in SEQ ID NO: 140,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 144,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 286Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 148,
(2) an antibody including an H chain encoded by a base sequence set forth in SEQ ID NO: 146,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 150,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 287Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 154,
(2) an antibody including an H chain encoded by a base sequence set forth in SEQ ID NO: 152,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 156,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 288Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 160,
(2) an antibody including an H chain encoded by a base sequence set forth in SEQ ID NO: 158,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 162,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 289Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 166,
(2) an antibody including an H chain encoded by a base sequence set forth in SEQ ID NO: 164,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 168,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 305Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 172,
(2) an antibody including an H chain encoded by a base sequence set forth in SEQ ID NO: 170,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 174,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 306Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 178,
(2) an antibody including an H chain encoded by a base sequence set forth in SEQ ID NO: 176,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 180,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 307Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 184,
(2) an antibody including an H chain encoded by a base sequence set forth in SEQ ID NO: 182,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 186,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 308Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 190,
(2) an antibody including an H chain encoded by a base sequence set forth in SEQ ID NO: 188,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 192,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 309Cys type anti-CD20 antibody described in any of the following (1) to (4).

(1) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and CH encoded by a base sequence set forth in SEQ ID NO: 242,
(2) an antibody including an H chain encoded by a base sequence set forth in SEQ ID NO: 240,
(3) an antibody including an H chain having CDR1 encoded by a base sequence set forth in SEQ ID NO: 46, CDR2 encoded by a base sequence set forth in SEQ ID NO: 48, CDR3 encoded by a base sequence set forth in SEQ ID NO: 50, and an Fc region encoded by a base sequence set forth in SEQ ID NO: 244,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 57, CDR2 encoded by a base sequence set forth in SEQ ID NO: 59, CDR3 encoded by a base sequence set forth in SEQ ID NO: 61, and CL encoded by a base sequence set forth in SEQ ID NO: 63, and
(ii) an L chain encoded by the base sequence set forth in SEQ ID NO: 55.

Furthermore, the present invention provides 298Cys type anti-EGFR antibody described in any of the following (1) to (4).

(1) an antibody including an H chain having CDR1 encoded by a base sequence set forth in SEQ ID NO: 194, CDR2 encoded by a base sequence set forth in SEQ ID NO: 196, CDR3 encoded by a base sequence set forth in SEQ ID NO: 198, and CH encoded by a base sequence set forth in SEQ ID NO: 82,
(2) an antibody including an H chain encoded by a base sequence set forth in SEQ ID NO: 208,
(3) an antibody including an H chain having CDR1 encoded by a base sequence set forth in SEQ ID NO: 194, CDR2 encoded by a base sequence set forth in SEQ ID NO: 196, CDR3 encoded by a base sequence set forth in SEQ ID NO: 198, and an Fc region encoded by a base sequence set forth in SEQ ID NO: 84,
(4) an antibody having an H chain described in the above-mentioned (1), (2) or (3), and a pair of L chains described in the following (i) or (ii);

(i) an L chain having CDR1 encoded by a base sequence set forth in SEQ ID NO: 202, CDR2 encoded by a base sequence set forth in SEQ ID NO: 204, CDR3 encoded by a base sequence set forth in SEQ ID NO: 206, and CL encoded by a base sequence set forth in SEQ ID NO: 246, and
(ii) an L chain encoded by the base sequence set forth in SEQ ID NO: 200.

Furthermore, the present invention provides Cγ1, Cγ2, Cγ3, and Cγ4 in which amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine. Such Cγ1, Cγ2, Cγ3, and Cγ4 is useful in manufacturing an antibody having an enhanced ADCC activity.
A person skilled in the art can make an antibody that recognizes any antigen having an enhanced ADCC activity by, for example, combining the Cγ1, Cγ2, Cγ3, and Cγ4 in which amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with Cys and arbitrary antibody variable region. Furthermore, person skilled in the art can make an antibody that recognizes any antigen having an enhanced ADCC activity by, for example, combining the Fc region of the Cγ1, Cγ2, Cγ3, and Cγ4 in which amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with Cys and arbitrary antibody Fab region. As mentioned above, it is preferable that the antibody of the present invention is an antibody that recognizes a molecule existing on the surface of a target cell to which cytotoxicity is intended to be given. The target cell to which cytotoxicity is intended to be given is preferably derived from human but not limited thereto. An example of the molecules (antigen) existing on the surface of the target cell to which cytotoxicity is intended to be given may include a target antigen selected from the group consisting of CD20, CD22, CD33, CD52, Her2/neu, EGFR, EpCAM, MUC1, GD3, CEA, CA125, HLA-DR, TNF alpha and VEGF, but the example is not limited thereto.
The present invention provides a method of manufacturing an antibody having an enhanced ADCC activity and the method includes substituting an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 with cysteine in the H chain constant region.
According to one embodiment of the manufacturing method, in the H chain constant region of an antibody having an enhanced ADCC activity, which is well known to a person skilled in the art, amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine. A method for obtaining an antibody having an ADCC activity known to a person skilled in the art is not particularly limited. For example, persons carrying out a manufacturing method of the present invention can manufacture an antibody by themselves or can purchase from others. A method for substituting an amino acid residue with cysteine includes, for example, site-specific mutation method mutation induction method (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ(1984) The gapped duplex DNA approach to oligonucleotide-directed mutation construction. Nucleic Acids Res. 12, 9441-9456, Kramer W, and Fritz HJ(1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci U S A. 82, 488-492). By using the method, a desirable amino acid of an antibody can be substituted with cysteine.
As another embodiment of the manufacturing method, firstly, an antibody bonded to a desired antibody is obtained by a method well known to a person skilled in the art. If an obtained antibody is a non-human antibody, the antibody can be humanized. Next, whether or not the obtained antibody has an ADCC activity or not is determined by the method well known to a person skilled in the art. The ADCC activity of the antibody can be measured by, for example, a lactate dehydrogenase release assay system described in Examples but not limited thereto. It can also be measured by, for example, Cr51-release assay. Next, in an H chain constant region of an antibody that is determined to have an ADCC activity, an amino acid residue at least one position selected from the group consisting of 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine.
More specifically, a method of manufacturing an antibody having an enhanced ADCC activity is provided. The method includes the following (a) and (b):

(a) a step of expressing a DNA encoding an H chain in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine in an H chain constant region, and a DNA encoding an L chain; and

(b) a step of collecting an expression product in the step (a).

The manufacturing method of the present invention, firstly, expresses a DNA encoding an H chain of an antibody having an ADCC activity, a DNA encoding an H chain in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine, and a DNA encoding an L chain of an antibody having an ADCC activity. In the H chain constant region, the DNA encoding an H chain in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine can be obtained by appropriately obtaining an H chain constant region portion of DNA encoding a wild type H chain, and appropriately introducing substitution so that codon encoding a specific amino acid in the H chain constant region encodes cysteine. Note here that the codon encoding cysteine is TGT or TGC.

Furthermore, in advance, by designing a DNA encoding protein in which amino acid in a constant region of the wild type H chain is substituted with cysteine, the DNA is chemically synthesized. Thereby, a DNA encoding an H chain in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine in an H chain constant region can be obtained.

Furthermore, DNA encoding an H chain in which an amino acid residue at at least one position selected from the group consisting of positions 286, 287, 288, 289, 290, 291, 292, 293, 294, 298, 299, 300, 301, 302, 303, 305, 306, 307, 308 and 309, 310 and 314 is substituted with cysteine in an H chain constant region of an Fc region can be manufactured by separating DNA into parts. An example of the combination of the parts of DNA includes a combination of a DNA encoding a variable region and a DNA encoding a constant region, or a combination of a DNA encoding an Fab region and a DNA encoding an Fc region. But the combination is not limited thereto. A DNA encoding an L chain can be also manufactured by separating DNA into parts.

For example, an example of a method for expressing a DNA encoding an H chain constant region in which amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine, a DNA encoding an H chain variable region, a DNA encoding an L chain constant region, and a DNA encoding an H chain constant region includes the following methods. That is to say, a DNA encoding an H chain variable region together with a DNA encoding an H chain constant region are incorporated into an expression vector so as to construct an H chain expression vector. Similarly, a DNA encoding an L chain variable region together with a DNA encoding an L chain constant region are incorporated into an expression vector so as to construct an L chain expression vector. An example of the expression vector can include an SV40 virus based vector, an EB virus based vector, a BPV (papilloma virus) based vector, and the like, but not limited thereto.

A method for introducing the above-mentioned four DNAs into vectors includes a method of introducing four DNAs into two vectors, a method of introducing four DNAs into three vectors (a method of introducing one DNA into one vector, one DNA into another vector, and rest of vectors into two DNAs), a method of introducing four DNAs into four vectors (a method of introducing four DNAs into each vector, separately). Herein, when four DNAs are introduced into two vectors and into three vectors, the way of grouping four DNAs is not particularly limited.

A host cell is co-transformed by an antibody expression vector produced by the above-mentioned method. As a host cell, a CHO (Chinese hamster ovary) cell hamster, and the like can be suitably used. Furthermore, for transformation, the lipofectin method (R. W. Malone et al., Proc. Natl. Acad. Sci. USA 86, 6077 (1989); P. L. Felgner et al., Proc. Natl. Acad. Sci. USA 84, 7413 (1987)), an electroporation method, a calcium phosphate method (F. L. Graham & A. J. van der Eb, Virology 52, 456-467 (1973)), a DEAE-Dextran method, and the like are suitably used.

In the method of manufacturing the antibody of the present invention, next, expression products obtained in step (a) are collected. The collection of the expression products can be carried out by, for example, culturing a transformed product and then separating the transformed product from cells or a culture solution. The antibody can be isolated and purified by appropriately combining methods, for example, centrifugation, ammonium sulfate fractionation, salting out, ultrafiltration, protein A, protein G and protein L columns, affinity chromatography, ion-exchange chromatography, gel-filtration chromatography, and the like.

By the above-mentioned method, it is possible to enhance the ADCC activity of an antibody. That is to say, the present invention provides a method of enhancing an ADCC activity of an antibody, which includes a step of substituting an amino acid residue in amino acid at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 in an H chain constant region with cysteine.

The present invention provides a pharmaceutical composition including an antibody of the present invention and a pharmaceutically acceptable carrier. Furthermore, the present invention provides a method for treating non-human mammalian, the method including administering the antibody of the present invention. A mammalian includes human, non-human mammalian (for example, mouse, rat, monkey, and the like). A pharmaceutical composition of the present invention is useful for treating B cell type malignant lymphoma and the like, when the antibody is an antibody recognizing CD20. Furthermore, it is useful for treating lung cancer and the like when the antibody is an antibody recognizing EGFR. Furthermore, it is useful for treating breast cancer, and the like, when the antibody is an antibody recognizing Her2.

The pharmaceutical composition of the present invention can be formulated by introducing a pharmaceutically acceptable carrier in addition to the antibody by a well-known method. For example, it can be used in a parenteral form as a sterile solution of water or other pharmaceutical acceptable liquid, or an injectable drug of a suspension drug. For example, it can be thought that the pharmaceutical composition is formulated by appropriately combining a pharmacologically acceptable carrier or medium, specifically, sterile water or a physiological salt solution, a vegetable oil, an emulsifying agent, a suspension, a surface-active agent, a stabilizer, a flavoring agent, excipient, vehicle, preservative, a binding agent, and the like, and admixing them in a form of a unit amount required to carry out generally recognized formulation. An effective amount of these drugs can be set so that an appropriately indicated range of dosage can be obtained.

As a sterile composition for injection, vehicle such as distilled water for injection can be formulated by using a vehicle by a usual formulation method.

As an aqueous solution for injection, for example, a physiological salt solution, an isotonic solution including glucose and other adjuvant, for example, sorbitol, D-mannitol, sodium chloride. It may be used together with an appropriate solubilizer, for example, alcohol, specifically ethanol, polyalcohol, for example, propylene glycol, polyethylene glycol, nonionic surfactant, for example, polysorbate 80 (TM), and HCO-50.

As an oily solution, sesame oil and soybean oil can be used. It may be used in combination with benzyl benzoate and benzyl alcohol as a solubilizer, and may be used together with a buffer agent, for example, a phosphate buffer solution, a sodium acetate buffer solution, a soothing agent, for example, hydrochloric acid procaine, stabilizer, for example, benzyl alcohol, phenol, and antioxidant. The prepared solution is usually filled in an appropriate ample.

Administration is carried out preferably via parenteral administration. A specific example of the parenteral administration includes injection, transnasal administration, transpulmonary administration, and transderrmal administration. An example of an injectable administration includes intravenous injection, intramuscular injection, intraperitoneal administration, subcutaneous injection, and the like. Administration can be carried out via systemic administration or local systemic.

Furthermore, an appropriate administration method can be selected based on ages and symptoms of agents. The dosage of pharmaceutical composition containing an antibody or polynucleotide encoding the antibody can be selected in a range from 0.0001 to 1000 mg per kg of body weight. Alternatively, for example, a dosage amount can be selected in the range from 0.001 to 100000 mg/body each patient. However, the dosage amount is not necessarily limited to these numeric values. A dose is different dependent upon weight, age, and symptom, etc., of a patient, and a person skilled in the art can select the suitable dose.

Furthermore, the present invention provides a method of determining whether or not an ADCC activity of a subject antibody is enhanced. Specifically, the present invention provides a method of determining that an ADCC activity of an antibody in the following step (a) is enhanced when an ADCC activity measured by the following steps (b) is higher than the ADCC activity before substitution.

(a) a step of providing a mutant of an antibody having an ADCC activity in which an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 is substituted with cysteine in an H chain constant region, and
(b) a step for measuring the ADCC activity of the step (a).

Furthermore, the present invention provides a method for screening an antibody having an enhanced ADCC activity. Specifically, the present invention provides a screening method of an antibody having an enhanced ADCC activity, and the method includes the following steps (a) to (d);

(a) providing an antibody having an ADCC activity;
(b) substituting an amino acid residue at at least one position selected from the group consisting of positions 280, 281, 282, 283, 284, 285, 286, 287, 288, 289, 290, 291, 292, 293, 294, 298, 299, 300, 301, 302, 303, 305, 306, 307, 308 and 309, 310 and 314 with cysteine in an H chain constant region of the antibody of the step (a);
(c) determining whether or not an ADCC activity of the antibody obtained in the step (b) is enhanced by the above-mentioned method; and
(d) selecting an antibody that has been determined in the step (c) that the ADCC activity is enhanced.

In these determination methods and screening methods, an antibody having an enhanced ADCC activity is not particularly limited as long as the antibody has an ADCC activity. A method of obtaining an antibody having an ADCC activity is mentioned above.

Note here that a base sequence and an amino acid sequence of an antibody disclosed in the specification is described according to the following sequence number.

< 290Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 92: base sequence of full length H chain (290Cys type)
SEQ ID NO: 93: amino acid sequence of H chain full length (290Cys type)
SEQ ID NO: 94: base sequence of H chain constant region (290Cys type)
SEQ ID NO: 95: amino acid sequence of H chain constant region (290Cys type)
SEQ ID NO: 96: base sequence of H chain Fc region (290Cys type)
SEQ ID NO: 97: amino acid sequence of H chain Fc region (290Cys type)

< 291Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 98: base sequence of full length H chain (290Cys type)
SEQ ID NO: 99: amino acid sequence of full length H chain (291Cys type)
SEQ ID NO: 100: base sequence of H chain constant region (291Cys type)
SEQ ID NO: 101: amino acid sequence of H chain constant region (291Cys type)
SEQ ID NO: 102: base sequence of H chain Fc region (291Cys type)
SEQ ID NO: 103: Amino acid sequence of H chain Fc region (291Cys type)

< 292Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 104: base sequence of full length H chain (292Cys type)
SEQ ID NO: 105: amino acid sequence of full length H chain (292Cys type)
SEQ ID NO: 106: base sequence of H chain constant region (292Cys type)
SEQ ID NO: 107: amino acid sequence of H chain constant region (292Cys type)
SEQ ID NO: 108: base sequence of H chain Fc region (292Cys type)
SEQ ID NO: 109: amino acid sequence of H chain Fc region (292Cys type)

<293Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 110: base sequence of full length H chain (293Cys type)
SEQ ID NO: 111: amino acid sequence of full length H chain (293Cys type)
SEQ ID NO: 112: base sequence of H chain constant region (293Cys type)
SEQ ID NO: 113: amino acid sequence of H chain constant region (293Cys type)
SEQ ID NO: 114: base sequence of H chain Fc region (293Cys type)
SEQ ID NO: 115: amino acid sequence of H chain Fc region (293Cys type)

<294Cys type anti-CD20 antibody >

SEQ ID NO: 44: base sequence of full length H chain (294Cys type)
SEQ ID NO: 45: amino acid sequence of full length H chain (294Cys type)
SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2

SEQ ID NO: 50: base sequence of H chain CDR3
SEQ ID NO: 51: amino acid sequence of H chain CDR3
SEQ ID NO: 52: base sequence of H chain constant region (294Cys type)
SEQ ID NO: 54: amino acid sequence of H chain constant region (294Cys type)
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 72: base sequence of H chain Fc region (294Cys type)
SEQ ID NO: 73: amino acid sequence of H chain Fc region (294Cys type)

<298Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 80: base sequence of full length H chain (298Cys type)
SEQ ID NO: 81: amino acid sequence of full length H chain (298Cys type)
SEQ ID NO: 82: base sequence of H chain constant region (298Cys type)
SEQ ID NO: 83: amino acid sequence of H chain constant region (298Cys type)
SEQ ID NO: 84: base sequence of H chain Fc region (298Cys type)
SEQ ID NO: 85: amino acid sequence of H chain Fc region (298Cys type)

<299Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3

SEQ ID NO: 63: base sequence L chain constant region
SEQ ID NO: 64: amino acid sequence of L chain constant region
SEQ ID NO: 116: base sequence of full length H chain (299Cys type)
SEQ ID NO: 117: amino acid sequence of full length H chain (299Cys type)
SEQ ID NO: 118: base sequence of H chain constant region (299Cys type)
SEQ ID NO: 119: amino acid sequence of H chain constant region (299Cys type)
SEQ ID NO: 120: base sequence of H chain Fc region (299Cys type)
SEQ ID NO: 121: amino acid sequence of H chain Fc region (299Cys type)

<300Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 122: base sequence of full length H chain (300Cys type)
SEQ ID NO: 123: amino acid sequence of full length H chain (300Cys type)
SEQ ID NO: 124: base sequence of H chain constant region (300Cys type)
SEQ ID NO: 125: amino acid sequence of H chain constant region (300Cys type)
SEQ ID NO: 126: base sequence of H chain Fc region (300Cys type)
SEQ ID NO: 127: amino acid sequence of H chain Fc region (300Cys type)

<301Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 86: base sequence of full length H chain (301 Cys type)
SEQ ID NO: 87: amino acid sequence of full length H chain (301Cys type)
SEQ ID NO: 88: base sequence of H chain constant region (301Cys type)
SEQ ID NO: 89: amino acid sequence of H chain constant region (301 Cys type)
SEQ ID NO: 90: base sequence of H chain Fc region (301 Cys type)
SEQ ID NO: 91: amino acid sequence of H chain Fc region (301Cys type)

<302Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 128: base sequence of full length H chain (302Cys type)
SEQ ID NO: 129: amino acid sequence of full length H chain (302Cys type)
SEQ ID NO: 130: base sequence of H chain constant region (302Cys type)
SEQ ID NO: 131: amino acid sequence of H chain constant region (302Cys type)
SEQ ID NO: 132: base sequence of H chain Fc region (302Cys type)
SEQ ID NO: 133: amino acid sequence of H chain Fc region (302Cys type)

<303Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 134: base sequence of full length H chain (303Cys type)
SEQ ID NO: 135: amino acid sequence of full length H chain (303Cys type)
SEQ ID NO: 136: base sequence of H chain constant region (303Cys type)
SEQ ID NO: 137: amino acid sequence of H chain constant region (303Cys type)
SEQ ID NO: 138: base sequence of H chain Fc region (303Cys type)
SEQ ID NO: 139: amino acid sequence of H chain Fc region (303Cys type)

< 304Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3

SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 140: base sequence of full length H chain (304Cys type)
SEQ ID NO: 141: amino acid sequence of full length H chain (304Cys type)
SEQ ID NO: 142: base sequence of H chain constant region (304Cys type)
SEQ ID NO: 143: amino acid sequence of H chain constant region (304Cys type)
SEQ ID NO: 144: base sequence of H chain Fc region (304Cys type)
SEQ ID NO: 145: amino acid sequence of H chain Fc region (304Cys type)

<286Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 146: base sequence of full length H chain (286Cys type)
SEQ ID NO: 147: amino acid sequence of full length H chain (286Cys type)
SEQ ID NO: 148: base sequence of H chain constant region (286Cys type)
SEQ ID NO: 149: amino acid sequence of H chain constant region (286Cys type)
SEQ ID NO: 150: base sequence of H chain Fc region (286Cys type)
SEQ ID NO: 151: amino acid sequence of H chain Fc region (286Cys type)

<287Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3

SEQ ID NO: 63: base sequence L chain constant region
SEQ ID NO: 64: amino acid sequence of L chain constant region
SEQ ID NO: 152: base sequence of full length H chain (287Cys type)
SEQ ID NO: 153: amino acid sequence of full length H chain (287Cys type)
SEQ ID NO: 154: base sequence of H chain constant region (287Cys type)
SEQ ID NO: 155: amino acid sequence of H chain constant region (287Cys type)
SEQ ID NO: 156: base sequence of H chain Fc region (287Cys type)
SEQ ID NO: 157: amino acid sequence of H chain Fc region (287Cys type)

<288Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 158: base sequence of full length H chain (288Cys type)
SEQ ID NO: 159: amino acid sequence of full length H chain (288Cys type)
SEQ ID NO: 160: base sequence of H chain constant region (288Cys type)
SEQ ID NO: 161: amino acid sequence of H chain constant region (288Cys type)
SEQ ID NO: 162: base sequence of H chain Fc region (288Cys type)
SEQ ID NO: 163: amino acid sequence of H chain Fc region (288Cys type)

<289Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 164: base sequence of full length H chain (289Cys type)
SEQ ID NO: 165: amino acid sequence of full length H chain (289Cys type)
SEQ ID NO: 166: base sequence of H chain constant region (289Cys type)
SEQ ID NO: 167: amino acid sequence of H chain constant region (289Cys type)
SEQ ID NO: 168: base sequence of H chain Fc region (289Cys type)
SEQ ID NO: 169: amino acid sequence of H chain Fc region (289Cys type)

<305Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 170: base sequence of full length H chain (305Cys type)
SEQ ID NO: 171: amino acid sequence of full length H chain (305Cys type)
SEQ ID NO: 172: base sequence of H chain constant region (305Cys type)
SEQ ID NO: 173: amino acid sequence of H chain constant region (305Cys type)
SEQ ID NO: 174: base sequence of H chain Fc region (305Cys type)
SEQ ID NO: 175: amino acid sequence of H chain Fc region (305Cys type)

<306Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 176: base sequence of full length H chain (306Cys type)
SEQ ID NO: 177: amino acid sequence of full length H chain (306Cys type)
SEQ ID NO: 178: base sequence of H chain constant region (306Cys type)
SEQ ID NO: 179: amino acid sequence of H chain constant region (306Cys type)
SEQ ID NO: 180: base sequence of H chain Fc region (306Cys type)
SEQ ID NO: 181: amino acid sequence of H chain Fc region (306Cys type)

<307Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3

SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 182: base sequence of full length H chain (307Cys type)
SEQ ID NO: 183: amino acid sequence of full length H chain (307Cys type)
SEQ ID NO: 184: base sequence of H chain constant region (307Cys type)
SEQ ID NO: 185: amino acid sequence of H chain constant region (307Cys type)
SEQ ID NO: 186: base sequence of H chain Fc region (307Cys type)
SEQ ID NO: 187: amino acid sequence of H chain Fc region (307Cys type)

<308Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3
SEQ ID NO: 63: base sequence L chain constant region
SEQ ID NO: 64: amino acid sequence of L chain constant region
SEQ ID NO: 188: base sequence of full length H chain (308Cys type)
SEQ ID NO: 189: amino acid sequence of full length H chain (308Cys type)
SEQ ID NO: 190: base sequence of H chain constant region (308Cys type)
SEQ ID NO: 191: amino acid sequence of H chain constant region (308Cys type)
SEQ ID NO: 192: base sequence of H chain Fc region (308Cys type)
SEQ ID NO: 193: amino acid sequence of H chain Fc region (308Cys type)

<309Cys type anti-CD20 antibody >

SEQ ID NO: 46: base sequence of H chain CDR1
SEQ ID NO: 47: amino acid sequence of H chain CDR1
SEQ ID NO: 48: base sequence of H chain CDR2
SEQ ID NO: 49: amino acid sequence of H chain CDR2
SEQ ID NO: 50: base sequence of H chain CDR3
SEQ ID NO: 51: amino acid sequence of H chain CDR3
SEQ ID NO: 55: base sequence of full length L chain
SEQ ID NO: 56: amino acid sequence of full length L chain
SEQ ID NO: 57: base sequence of L chain CDR1
SEQ ID NO: 58: amino acid sequence of L chain CDR1
SEQ ID NO: 59: base sequence of L chain CDR2
SEQ ID NO: 60: amino acid sequence of L chain CDR2
SEQ ID NO: 61: base sequence of L chain CDR3
SEQ ID NO: 62: amino acid sequence of L chain CDR3

SEQ ID NO: 63: base sequence L chain constant region
SEQ ID NO: 64: amino acid sequence of L chain constant region
SEQ ID NO: 240: base sequence of full length H chain (309Cys type)
SEQ ID NO: 241: amino acid sequence of full length H chain (309Cys type)
SEQ ID NO: 242: base sequence of H chain constant region (309Cys type)
SEQ ID NO: 243: amino acid sequence of H chain constant region (309Cys type)
SEQ ID NO: 244: base sequence of H chain Fc region (309Cys type)
SEQ ID NO: 245: amino acid sequence of H chain Fc region (309Cys type)

< 298Cys type anti-EGFR antibody >

SEQ ID NO: 194: base sequence of H chain CDR1
SEQ ID NO: 195: amino acid sequence of H chain CDR1
SEQ ID NO: 196: base sequence of H chain CDR2
SEQ ID NO: 197: amino acid sequence of H chain CDR2
SEQ ID NO: 198: base sequence of H chain CDR3
SEQ ID NO: 199: amino acid sequence of H chain CDR3
SEQ ID NO: 200: base sequence of full length L chain
SEQ ID NO: 201: amino acid sequence of full length L chain
SEQ ID NO: 202: base sequence of L chain CDR1
SEQ ID NO: 203: amino acid sequence of L chain CDR1
SEQ ID NO: 204: base sequence of L chain CDR2
SEQ ID NO: 205: amino acid sequence of L chain CDR2
SEQ ID NO: 206: base sequence of L chain CDR3
SEQ ID NO: 207: amino acid sequence of L chain CDR3
SEQ ID NO: 246: base sequence of L chain constant region
SEQ ID NO: 247: amino acid sequence of L chain constant region
SEQ ID NO: 208: base sequence of full length H chain (298Cys type)
SEQ ID NO: 209: amino acid sequence of full length H chain (298Cys type)
SEQ ID NO: 82: base sequence of H chain constant region (298Cys type)
SEQ ID NO: 83: amino acid sequence of H chain constant region (298Cys type)
SEQ ID NO: 84: base sequence of H chain Fc region (298Cys type)
SEQ ID NO: 85: amino acid sequence of H chain Fc region (298Cys type)

[Examples]

Hereinafter, the present invention is described with reference to Examples, but the range of the present invention is not limited to these Examples.

[Examples 1]

293, 294, 298, 299, 300, and 301 Cys types of anti-CD20 chimeric antibodies were produced, and the ADCC activity of each of these Cys types of anti-CD20 chimeric antibodies was evaluated. As a result, as compared with the ADCC activity of the wild type anti-CD20 chimeric antibody, 294, 298, and 301 Cys types of anti-CD20 chimeric antibodies showed extremely high ADCC activity.
Hereinafter, the production method of the wild type of the anti-CD20 chimeric antibody and three kinds of mutant types (294Cys, 298Cys, and 301Cys) and the ADCC activity measurement thereof are the reactivity with respect to CD20 molecule are described.

1) Production of anti-CD20 Chimeric antibody

A chimeric antibody is obtained as a purified chimeric antibody through the following steps A) to F).

A) cloning a gene necessary for production of a chimeric antibody,
B) introducing a mutation of the cloned gene,
C) constructing a chimeric antibody expression vector combining the cloned gene and the mutation-introduced gene,
D) carrying a gene transfer of a chimeric antibody expression vector into a CHO cell and screening a CHO cell highly expressing a chimeric antibody,

E) culturing a CHO cell highly expressing a chimeric antibody, and
F) carrying out column purification from a culture supernatant of a cell highly expressing a chimeric antibody.

Hereinafter, steps A) to F) are described sequentially in this order.

A) Cloning a Gene Necessary for Production of Chimeric Antibody

In order to produce one kind of anti-CD20 chimeric antibody, four kinds of genes are needed. These four kinds of genes include an anti-CD20 mouse L chain variable region gene, an anti-CD20 mouse H chain variable region gene, a human IgG1 L chain constant region gene, and a human IgG1 H chain constant region gene. Hereinafter, an example of cloning of these genes is described.

(Anti-CD20 mouse L chain variable region gene)

From hybridoma cells producing anti-CD20 mouse monoclonal antibody possessed by the present applicants, mRNA was obtained by using QuickPrep micro mRNA purification kit (Amersham Biosciences, code 27-9255-01). The mRNA was made into cDNA by using First-Strand cDNA Synthesis kit (Amersham Biosciences, code 27-9261-01). A gene is amplified by PCR method using this cDNA as a template. The PCR reaction was carried out in the following 11 patterns of combination of primers.

Conditions of PCR reaction:

| | |
|---|---|
| cDNA from mouse hybridoma | 4 μL |
| 2.5 mM dNTPs | 4 μL |
| One of 11 kinds of primers, MKV1 - MKV 11 primers (20 μM) | 2.5 μL |
| MKC primer (20μM) | 2.5 μL |
| DMSO | 2.5 μL |
| × 10 pfu polymerase Buffer | 5 μL |
| pfu polymerase | 1 μL |
| sterile water | 28.5 μL |
| total | 50 μL |

94°C, 2 min
94°C, 1 min; 55°C, 2 min; 72°C, 2 min (30 cycles)
72°C, 4 min
4°C, unlimited time

DNA sequence of primers follows:

MKV1 primer: ATGAAGTTGCCTGTTAGGCTGTTGGTGCTG (SEQ ID NO: 1)
MKV2 primer: ATGGAGWCAGACACACTCCTGYTATGGGTG (SEQ ID NO: 2)
MKV3 primer: ATGAGTGTGCTCACTCAGGTCCTGGSGTTG (SEQ ID NO: 3)
MKV4 primer: ATGAGGRCCCCTGCTCAGWTTYTTGGMWTCTTG (SEQ ID NO: 4)
MKV5 primer: ATGGATTTWCAGGTGCAGATTWTCAGCTTC (SEQ ID NO: 5)
MKV6 primer: ATGAGGTKCYYTGYTSAGYTYCTGRGG (SEQ ID NO: 6)
MKV7 primer: ATGGGCWTCAAGATGGAGTCACAKWYYCWGG (SEQ ID NO: 7)
MKV8 primer: ATGTGGGGAYCTKTTTYCMMTTTTTCAATTG (SEQ ID NO: 8)
MKV9 primer: ATGGTRTCCWCASCTCAGTTCCTTG (SEQ ID NO: 9)
MKV10 primer: ATGTATATATGTTTGTTGTCTATTTCT (SEQ ID NO: 10)
MKV11 primer: ATGGAAGCCCCAGCTCAGCTTCTCTTCC (SEQ ID NO: 11)
MKC primer: ACTGGATGGTGGGAAGATGG (SEQ ID NO: 12)
(M = A or C, R = A or G, W = A or T, S = C or G, Y = C or T, K = G or T)

In the PCR reaction, an anti-CD20 mouse L chain variable region gene was amplified by the combination of MKV5 primer and MKC primer, and this gene was temporarily inserted into a pCR2.1 vector (Invitogen) and stored. Furthermore, a vector into which this gene had been introduced was referred to as pCR2.1-MLV. A DNA sequence of genetically cloned anti-CD20 mouse L chain variable region is attached to Fig. 1.
Furthermore, the base sequences of CDR1, CDR2, and CDR3 of the anti-CD20 mouse L chain variable region are shown in SEQ ID NOs: 57, 59, and 61. Furthermore, the amino acid sequences of CDR11, CDR2, and CDR3 of the anti-CD20 mouse L chain variable region are shown in SEQ ID NOs: 58, 60, and 62.

(Anti-CD20 mouse H chain variable region gene)

Similar to the cloning of an anti-CD20 mouse L chain variable region gene, the following 12 patterns of PCR amplification were carried out by using cDNA prepared from hybridoma cells producing an anti-CD20 mouse monoclonal antibody as a template.

| | |
|---|---|
| cDNA from mouse hybridoma | 4 μL |
| 2.5 mM dNTPs | 4 μL |
| One of 12 kinds of primers, MHV1 - MHV12 primers (20 μM) | 2.5 μL |
| MHCG2b primer (20μM) | 2.5 μL |
| DMSO | 2.5 μL |
| × 10 pfu polymerase Buffer | 5 μL |
| pfu polymerase | 1 μL |
| sterile water | 28.5 μL |
| total | 50 μL |

94°C, 2 min
94°C, 1 min; 55°C, 2 min; 72°C, 2 min (30 cycles)
72°C, 4 min
4°C, unlimited time

DNA sequence of primers follows:

MHV1 primer: ATGAAATGCAGCTGGGGCATSTTCTTC (SEQ ID NO: 13)
MHV2 primer: ATGGGATGGAGCTRTATCATSYTCTT (SEQ ID NO: 14)
MHV3 primer: ATGAAGWTGTGGTTAAACTGGGTTTTT (SEQ ID NO: 15)
MHV4 primer: ATGRACTTTGGGYTCAGCTTGRTTT (SEQ ID NO: 16)
MHV5 primer: ATGGACTCCAGGCTCAATTTAGTTTTCCTT (SEQ ID NO: 17)
MHV6 primer: ATGGCTGTCYTRGSGCTRCTCTTCTGC (SEQ ID NO: 18)
MHV7 primer: ATGGRATGGAGCKGGRTCTTTMTCTT (SEQ ID NO: 19)
MHV8 primer: ATGAGAGTGCTGATTCTTTTGTG (SEQ ID NO: 20)
MHV9 primer: ATGGMTTGGGTGTGGAMCTTGCTATTCCTG (SEQ ID NO: 21)
MHV10 primer: ATGGGCAGACTTACATTCTCATTCCTG (SEQ ID NO: 22)
MHV11 primer: ATGGATTTTGGGCTGATTTTTTTTATTG (SEQ ID NO: 23)
MHV12 primer: ATGATGGTGTTAAGTCTTCTGTACCTG (SEQ ID NO: 24)
MHCG2b primer: CAGTGGATAGACTGATGGGGG (SEQ ID NO: 25)
(M = A or C, R = A or G, W = A or T, S = C or G, Y = C or T, K = G or T)

In the PCR reaction, an anti-CD20 mouse H chain variable region gene was amplified by the combination of MHV7 primer and MHCG2b primer, and this gene was temporarily inserted into pCR2.1 vector (Invitogen) and stored. Furthermore, a vector into which this gene had been introduced was referred to as pCR2.1-MHV. A DNA sequence of the genetically cloned anti-CD20 mouse H chain variable region is attached to Fig. 1.
Furthermore, the base sequences of CDR1, CDR2, and CDR3 of the anti-CD20 mouse H chain variable region are shown in SEQ ID NOs: 46, 48, and 50. Furthermore, the amino acid sequences of CDR1, CDR2, and CDR3 of the anti-CD20 mouse H chain variable region are shown in SEQ ID NOs: 47, 49, and 51.

(Human IgG1 L chain Constant Region Gene )

Lymphocytes were collected from human blood by using Lymphoprep (Axis Shield). From the lymphocyte, mRNA was obtained by using QuickPrep micro mRNA purification kit (Amersham Biosciences, code 27-9255-01). The mRNA was made into cDNA by using First-Strand cDNA Synthesis kit (Amersham Biosciences, code 27-9261-01). The following PCR reaction was carried out by using the cDNA from human lymphocyte as a template, and a human IgG1 L chain constant region gene was obtained. This gene was also temporarily inserted into pCR2.1 vector (Invitogen) and stored. Furthermore, a vector into which this gene had been introduced was referred to as pCR2.1-LC.
The condition of PCR reactions follows.

| | |
|---|---|
| human lymphocyte cDNA | 4 μL |
| 2.5 mM dNTPs | 4 μL |
| hIgG1 LCF primer (20 μM) | 2.5 μL |
| hIgG1 LCR primer (20μM) | 2.5 μL |
| DMSO | 2.5 μL |
| × 10 pfu polymerase Buffer | 5 μL |
| pfu polymerase | 1 μL |
| sterile water | 28.5 μL |
| total | 50 μL |

94°C, 2 min
94°C, 1 min; 55°C, 2 min; 72°C, 2 min (30 cycles)
72°C, 4 min
4°C, unlimited time

DNA sequence of primers follows:

hIgG1 LCF primer: ACTGTGGCTGCACCATCTGTCTTC (SEQ ID NO: 26)
hIgG1 LCR primer: TTAACACTCTCCCCTGTTGAAGCTCTT (SEQ ID NO: 27)

(Human IgG1 H Chain Constant Region Gene)

Similar to the cloning of a human IgG1 L chain constant region gene, the following PCR amplification was carried out by using cDNA from human lymphocyte as a template so as to obtain a human IgG1 H chain constant region gene. This gene was also temporarily inserted into pCR2.1 vector (Invitogen) and stored. This vector was referred to as per2.1- HC (wild type).
The condition of PCR reactions follows.

| | |
|---|---|
| human lymphocyte cDNA | 4 μL |
| 2.5 mM dNTPs | 4 μL |
| hIgG1 HCF primer (20 μM) | 2.5 μL |
| hIgG1 HCR primer (20μM) | 2.5 μL |
| DMSO | 2.5 μL |
| × 10 pfu polymerase Buffer | 5 μL |
| pfu polymerase | 1 μL |
| sterile water | 28.5 μL |
| total | 50 μL |

94°C, 2 min
94°C, 1 min; 55°C, 2 min; 72°C, 2 min (30 cycles)
72°C, 4 min
4°C, unlimited time

DNA sequence of primers follows:

hIgG1 HCF primer: GCCTCCACCAAGGGCCCATCGGTC (SEQ ID NO: 28)
hIgG1 HCR primer: TTATTTACCCGGAGACAGGGAGAGGCT (SEQ ID NO: 29)

B) Introducing Mutation of Cloned Gene

In order to obtain 294Cys chimeric antibody, 298Cys chimeric antibody, and 301Cys chimeric antibody, the mutation needs to be introduced into a certain position of the cloned human IgG1 H chain constant region. According to the number of human IgG1 H chain constant region in Sequence of proteins of Immunological Interest (NIH Publication No. 91-3242, 1991) shown by Elvin A.Kabat, in order to change Glu at a position 294 into Cys, Ser at a position 298 into Cys, and Arg at a position 301 into Cys, mutation was introduced by PCR method by using a pCR2.1 vector into which a cloned hunan IgG1 H chain constant region had been inserted : pCR2.1-HC (wild type) as a template. The detail of this mutation introduction was described as follows.

(1) Mutation Introduction into 294 (Glu → Cys)

| | |
|---|---|
| pCR2.1-HC (wild type ) (25 ng/μL) | 2 μL |
| 2.5 mM dNTPs | 4 μL |
| 294 Cys1 primer (20 μM) | 1.25 μL |
| 294 Cys2 primer (20 μM) | 1.25 μL |
| × 10 pfu polymerase Buffer | 5 μL |
| pfu polymerase | 1 μL |
| sterile water | 35.5 μL |
| total | 50 μL |

95°C, 30 sec
95°C, 30 sec; 55°C, 1 min; 68°C, 13 min (12 cycles)
68°C, 4 min
4°C, unlimited time

DNA sequences of primers follows:

294 Cys1 primer: AAGCCGCGTGAGTGCCAGTACAACAGC (SEQ ID NO: 74)
294 Cys2 primer: GCTGTTGTACTGGCACTCACGCGGCTT (SEQ ID NO: 75)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 294 (Glu → Cys) had been introduced. This mutant-introduced vector was referred to as a pCR2.1-HC (294Cys).

(2) Mutation Introduction into 298 (Ser → Cys)

This mutation introduction was carried out similar to the above-mentioned mutation introduction of 294 (Glu → Cys). The both mutation introductions are different from each other only in that 298Cys1 primer and 298Cys2 primer are used instead of used 294Cys1 primer and 294Cys2 primer. Hereinafter, the sequences of the 298Cys1 primer and 298Cys2 primer are shown. pCR2.1 vector including human IgG1 H chain constant region into which mutation 298 (Ser → Cys) obtained by these operations had been introduced was referred to as pCR2.1-HC (298Cys).

298 Cys1 primer: GAGCAGTACAACTGCACGTACCGTGTG (SEQ ID NO: 76)
298 Cys2 primer: CACACGGTACGTGCAGTTGTACTGCTC (SEQ ID NO: 77)

(3) Mutation Introduction into 301 (Arg → Cys)

This mutation introduction was carried out in the same way as the above-mentioned mutation introduction of 294 (Glu → Cys). The both mutation introductions are different from each other only in that 301Cys1 primer and 301 Cys2 primer are used instead of used 294Cys1 primer and 294Cys2 primer. Hereinafter, the sequences of the 301Cys1 primer and 301Cys2 primer are shown. pCR2.1 vector including human IgG1 H chain constant region into which mutation 301 (Arg → Cys) obtained by these operations had been introduced was referred to as pCR2.1-HC (301 Cys).

301Cys1 primer: TACAACAGCACGTACTGTGTGGTCAGCGTCCTC (SEQ ID NO: 78)
301 Cys2 primer: GAGGACGCTGACCACACAGTACGTGCTGTTGTA (SEQ ID NO: 79)

C) Construction of Chimeric Antibody Expression Vector Combining Cloned Gene and Mutation-Introduced Gene

One kind of anti-CD20 chimeric antibody can be expressed by transfection of two kinds of expression vectors of L chain and H chain of the anti-CD20 chimeric antibody into a CHO cell. Herein, an expression vector of the L chain of the anti-CD20 chimeric antibody was obtained by binding an anti-CD20 mouse L chain variable region gene and a human IgG1 L chain constant region gene and introducing thereof into an expression vector, and the H chain of the anti-CD20 chimeric antibody was obtained by binding an anti-CD20 mouse H chain variable region gene and a human IgG1 H chain constant region gene and introducing thereof into an expression vector. When wild type, 294Cys type, 298Cys type or 301Cys type anti-CD20 chimeric antibodies were produced, an expression vector of the L chain of the anti-CD20 chimeric antibody are used in common for expressing these four kinds of chimeric antibodies, but an expression vector of an H chain of an anti-CD20 chimeric antibody needs an H chain expression vector specific to each of the wild type, 294Cys type, 298Cys type or 301 Cys type chimeric antibodies. Herein, firstly, a construction of an expression vector of an anti-CD20 chimeric antibody L chain used in common in expression of each chimeric antibody is described in detail. Then, a construction of an expression vector of an anti-CD20 chimeric antibody H chain that is necessary to expression of the wild type, 294Cys type, 298Cys type or 301Cys type chimeric antibodies is described.

Construction of Expression Vector of L Chain of Anti-CD20 Antibody

As the expression vector, a BCMGneo vector is used. The anti-CD20 mouse L chain variable region gene + human IgG1 L chain constant region gene are inserted into the XhoI and NotI sites of this vector. A fragment of the anti-CD20 mouse L chain variable region gene was obtained by carrying out the below-mentioned PCR reaction by using a pCR2.1-MLV as a template. Furthermore, a fragment of the human IgG1 L chain constant region gene was obtained by carrying out the below-mentioned PCR reaction by using a pCR2.1-LC as a template.

(Amplification Reaction of Anti-CD20 Mouse L Chain Variable Region Gene)

| | |
|---|---|
| pCR2.1-MLV (20 ng/μL) | 2 μL |
| 2.5 mM dNTPs | 4 μL |
| L1 primer (20 μM) | 2.5 μL |
| L2 primer (20 μM) | 2.5 μL |
| DMSO | 2.5 μL |
| ×10 pfu polymerase Buffer | 5 μL |
| pfu polymerase | 1 μL |
| sterile water | 30.5 μL |
| total | 50 μL |

94°C, 2 min
94°C, 1 min; 55°C, 2 min; 72°C, 2 min (20 cycles)
72°C, 4 min
4°C, unlimited time
L1 primer: ACCGCTCGAGATGGATTTTCAGGTGCAGATTATCAGC (SEQ ID NO: 36)
L2 primer: TTTCAGCTCCAGCTTGGTCCCAGCACC (5'-phosphorylated) (SEQ ID NO: 37)

(Amplification Reaction of Human Igg1 L Chain Constant Region Gene)

| | |
|---|---|
| pCR2.1-LC (20 ng/μ**L**) | 2 μL |

(continued)

| | |
|---|---|
| 2.5 mM dNTPs | 4 μL |
| L3 primer (20 μM) | 2.5 μL |
| L4 primer(20 μM) | 2.5 μL |
| DMSO | 2.5 μL |
| ×10 pfu polymerase Buffer | 5 μL |
| pfu polymerase | 1 μL |
| sterile water | 30.5 μL |
| total | 50 μL |

94°C, 2 min
94°C, 1 min; 55°C, 2 min; 72°C, 2 min (20 cycles)
72°C, 4 min
4°C, unlimited time
L3 primer: ACTGTGGCTGCACCATCTGTCTTCATC (5'- phosphorylated) (SEQ ID NO: 38)
L4 primer: ATAGTTTAGCGGCCGCTTAACACTCTCCCCTGTTGAAGCTCTTTGT (SEQ ID NO: 39)

The fragment of the anti-CD20 mouse L chain variable region gene obtained in the above-mentioned PCR reaction was cut with a restriction enzyme XhoI (TAKARA BIO) and then purified. Furthermore, the fragment of the human IgGI L chain constant region gene obtained by the PCR reaction was cut with a restriction enzyme NotI (TAKARA BIO) and then purified. A BCMG-neo vector was ligated to a fragment cut with XhoI and NotI and then purified, by mixing the previously-mentioned purified anti-CD20 mouse L chain variable region gene fragment and purified human IgG1 L chain constant region gene fragment. Whether or not the intended fragment had been inserted into the vector obtained by this ligation by confirming the sequence. The expression vector of this L chain of the anti-CD20 antibody was referred to as p chimeric LC.

Construction of Anti-CD20 Chimeric Antibody H Chain Expression Vector

As the expression vector, a BCMGneo vector is used. The anti-CD20 mouse L chain variable region gene + human IgG1 H chain constant region gene are inserted into the XhoI and NotI sites of this vector. A fragment of the anti-CD20 mouse H chain variable region gene was obtained by carrying out the below-mentioned PCR reaction by using a pCR2.1-MLV as a template. Furthermore, a fragment of the human IgG1 H chain constant region gene was obtained by carrying out the below-mentioned PCR reaction by using a pCR2.1-LC as a template.
An H chain expression vector specific to each of the wild type, 294Cys type, 298Cys type or 301Cys type chimeric antibody was subjected to the same operation although a plasmid used as a template for PCR amplification of a human IgG1 H chain constant region gene, thus a various human IgG1 H chain fragment was obtained. Hereinafter, detail including a PCR reaction is described.

(Amplification Reaction of Anti-CD20 Mouse H Chain Variable Region Gene)

| | |
|---|---|
| pCR2.1-MHV (20 ng/μL) | 2 μL |
| 2.5 mM dNTPs | 4 μL |
| H1 primer (20 μM) | 2.5 μL |
| H2 primer(20 μM) | 2.5 μL |
| DMSO | 2.5 μL |
| ×10 pfu polymerase Buffer | 5 μL |
| pfu polymerase | 1 μL |
| sterile water | 30.5 μL |
| total | 50 μL |

94°C, 2 min
94°C, 1 min; 55°C, 2 min; 72°C, 2 min (20 cycles)

72°C, 4 min
4°C, unlimited time
H1 primer: ACCGCTCGAGATGGGATGGAGCTGGGTCTTTCTCTTC (SEQ ID NO: 40)
H2 primer: TGAGGAGACGGTGACCGTGGTCCC (5'- phosphorylated) (SEQ ID NO: 41)

(Amplification Reaction of Human IgG1 H Chain Constant Region Gene)

| | |
|---|---|
| # various kinds of template plasmids (20 ng/μL) | 2 μL |
| 2.5 mM dNTPs | 4 μL |
| H3 primer(20μM) | 2.5 μL |
| H4 primer(20μM) | 2.5 μL |
| DMSO | 2.5 μL |
| ×10 pfu polymerase Buffer | 5 μL |
| pfu polymerase | 1 μL |
| sterile water | 30.5 μL |
| total | 50 μL |

94°C, 2 min
94°C, 1 min; 55°C, 2 min; 72°C, 2 min (20 cycles)
72°C, 4 min
4°C, unlimited time
H3 primer: GCCTCCACCAAGGGCCCATCGGTC(5'- phosphorylated) (SEQ ID NO: 42)
H4 primer: ATAGTTTAGCGGCCGCTTATTTACCCGGAGACAGGGAGAGGCTCTT (SEQ ID NO: 43)

#: in order to prepare a gene fragment for the wild type anti-CD20 chimeric antibody, pCR2.1-HC (wild type) was used as a template. In order to prepare a gene fragment for 294Cys type anti-CD20 chimeric antibody, pCR2.1-HC (294Cys) was used as a template. In order to prepare a gene fragment for 298Cys type anti-CD20 chimeric antibody, pCR2.1-HC (298Cys) was used as a template. In order to prepare a gene fragment for 301Cys type anti-CD20 chimeric antibody, pCR2.1-HC (301Cys) was used as a template.

The fragment of the anti-CD20 mouse H chain variable region gene obtained in the above-mentioned PCR reaction was cut with a restriction enzyme XhoI (TAKARA) and then purified. Furthermore, the fragment of the human IgG1 H chain constant region gene obtained by various PCR reactions using different templates were cut with a restriction enzyme NotI (TAKARA BIO) and then purified. A BCMG-neo vector was ligated to a fragment cut with XhoI and NotI and then purified, by mixing the previously-mentioned purified anti-CD20 mouse H chain variable region gene fragment and purified human IgG1 H chain constant region gene fragment. Whether or not the intended fragment had been inserted into the vector obtained by this ligation by confirming the sequence. The expression vector of this L chain of the anti-CD20 antibody was referred to as p chimeric HC (wild type), p chimeric HC (294Cys), p chimeric HC (298Cys) or p chimeric HC (301Cys), respectively.

D) Gene Transfer of Chimeric Antibody Expression Vector into CHO Cell and Screening of CHO Cell Highly Expressing Chimeric Antibody

Gene introduction of various kinds of constructed expression vectors into a CHO cell was carried out in the following combinations by using TransIT-CHO Transfection kit (Mirus, MIR2170).
wild type chimeric antibody : p chimeric LC + p chimeric HC (wild type )

294Cys chimeric antibody : p chimeric LC +p chimeric HC (294Cys)
298Cys chimeric antibody : p chimeric LC +p chimeric HC (298Cys)
301Cys chimeric antibody : p chimeric LC +p chimeric HC (301Cys)

A gene-transferred CHO cell was proliferated by culturing it in 10% Fetal Bovine Serum (EQITEC-BIO) and Dulbecco's Modified Eagle's Medium (Sigma, D5796) supplemented with 1 mg/mL Geneticin (GIBCO, 10131-035) under the conditions at 37°C in 5% carbon dioxide. When CHO cells highly expressing a chimeric antibody were selected from CHO cells that had been proliferated and formed colonies, firstly, a part of each colony was transferred to a 96 well plate and cultured for another 10 days. A part of the culture supernatant thereof was taken out and subjected to ELISA measurement, thus determining a high expression CHO cell clone.

(ELISA measurement)

1) A culture supernatant of transfected CHO cells was added into a 96 well plate coated with anti-human IgG (γ-chain) (MBL, 103AG) at 100 μL/well and stood still at room temperature for one hour.
2) A culture supernatant solution was discarded from the 96 well plate and the plate was sufficiently washed with PBS containing 0.05% Tween20 (PBS-0.05% Tween20).
3) A solution obtained by 2000-folded diluting anti-human IgG (γ-chain) Peroxidase conjugated (MBL, 208) with PBS-0.05% Tween20 was added at 100 μL/well and stood still at room temperature for one hour.
4) The solution was discarded from the 96 well plate and the plate was sufficiently washed with PBS-0.05% Tween20.
5) TMB Peroxidase Substrate (Moss, TMBE-1000s) was added to 100 μL/well to cause a color reaction.
6) After color reaction for 10 - 15 minutes, 100 μL/well of 1N sulfuric acid was added so as to stop the color reaction.
7) 450 nm absorbance was measured.

Colonies showing high coloring value in this ELISA measurement is a clone highly expressing a chimeric antibody.

E) Culturing CHO Cell Highly Expressing Chimeric Antibody

The CHO cell highly expressing a chimeric antibody selected by ELISA measurement proliferated by culturing it in 10% Fetal Bovine Serum and Dulbecco's Modified Eagle's Medium supplemented with 1 mg/mL Geneticin under the conditions at 37°C in 5% carbon dioxide. The sufficiently proliferated CHO cells highly expressing a chimeric antibody were cultured in the medium that had been replaced with a serum-free medium CHO-S-(GIBCO, 12052-098) supplemented with 0.1 mg/mL of Geneticin and continuously cultured in about 1000 mL.

F) Column Purification of Chimeric Antibody High Expression Cell from Culture Supernatant

A culture supernatant of chimeric antibody high-expression CHO cells cultured in a serum-free medium CHO-S-SFMII was collected. Purification of a wild type chimeric antibody was carried out by protein A column purification generally used for purifying an antibody since an Fc region structure was not changed. However, the 294Cys type chimeric antibody, 298Cys type chimeric antibody, and 301Cys type chimeric antibody are hardly adsorbed to a general protein A column since the Fc region structure thereof is mutated and the structure of the Fc region is changed. For this reason, purification of these Cys type chimeric antibodies was carried out by using a Protein L column. In the anti-CD20 chimeric antibody produced at this time, the variable region is κ chain. This Protein L is a protein capable recognizing the variable region κ chain and being bonded thereto. Hereinafter, the detail of the Protein A column purification of a wild type chimeric antibody and the Protein L column purification of the Cys type chimeric antibody is described.

Protein A Column Purification of Wild Type Chimeric Antibody

1) Culture supernatant obtained by culturing CHO cells highly expressing a wild type chimeric antibody in 1000 mL of CHO-S-SFMII was concentrated from 1000 mL to 100 mL by using Amicon Stirred Ultrafiltration Cell (Millipore, Model 8400) to which Amicon Ultrafiltration Membranes (Millipore, Code YM10) was attached.
2) Eight mL of rProteinA Sepharose Fast Flow (Amersham Bioscience, 17-1279-03) that was a Protein A-Sepharose was added to a culture supernatant concentrated solution, followed by stirring at 4°C for two days so as to allow a wild type chimeric antibody to Protein A.
3) ProteinA-Sepharose adsorbing a wild type chimeric antibody was filled in a column having a diameter of 1.5 cm and length of 8 cm.
4) The column filled with ProteinA-Sepharose was washed with 100 mL of PBS and then a wild type chimeric antibody was eluted from column by using an Elution Buffer (0.17M Glycine-HCl, pH 2.3).
5) The eluted wild type chimeric antibody solution was made to be neutral in pH by immediately adding an appropriate amount of 1M Tris-HCl (pH 8.5).
6) A eluted solution of the wild type chimeric antibody was placed in a Cell Sep T2 (Membrane filtration products Inc, 8030-23) that was a dialyzer tube and dialyzed into 5L of PBS at 4° C. A wild type chimeric antibody collected after dialysis was made to be a purified product.

Protein L Column Purification of Cys Type Chimeric Antibody

1) Culture supernatant obtained by culturing CHO cells highly expressing a Cys type chimeric antibody in 1000 mL of CHO-S-SFMII was concentrated from 1000 mL to 100 mL by using Amicon Stirred Ultrafiltration Cell (Millipore, Model 8400) to which Amicon Ultrafiltration Membranes (Millipore, Code YM10) was attached.
2) 100 mL of ProteinL Binding Buffer (0.1M Phosphate, 0.15 M NaCl, pH 7.2) was added to 100 mL of culture supernatant concentrated solution and mixed thereof.
3) Two mL of ImmunoPure Immobilized Protein L Plus (PIERCE, 20250) that was Protein L-Sepharose was filled in a column having a diameter of 1.5 cm and length of 8 cm.
4) The solution prepared in 2) was added from the upper end of the column and allowed to slowly flow from the lower end of the column. With this operation, a Cys type chimeric antibody was allowed to be adsorbed to Protein L-Sepharose. Thereafter, the column was washed with 50 mL of Protein L Binding Buffer so as to remove excessive protein adsorbed to the column.
5) A Cys type chimeric antibody was diluted from the column by using Elution Buffer (0.17M Glycine-HCl, pH2.3). The eluted solution of the Cys type chimeric antibody solution was made to be neutral in pH by adding immediately adding an appropriate amount of 1M Tris-HCl (pH 8.5).
6) A eluted solution of the Cys type chimeric antibody was placed in a Cell Sep T2 (Membrane filtration products Inc, 8030-23) that was a dialyzer tube and dialyzed into 5L of PBS at 4° C. A Cys type chimeric antibody collected after dialysis was made to be a purified product.

As mentioned above, the purified wild type chimeric antibody or the Cys type chimeric antibody were subjected to electrophoresis with 12.5% SDS-PAGE, followed by silver staining. This electrophoretic pattern was attached to Fig. 3.

< Evaluation of ADCC Activities of Various Chimeric Antibodies >

Antibody-dependent Cellular Cytotoxicity (ADCC) activities of various chimeric antibodies were carried out by a lactate dehydrogenase release assay. In this assay, a Daudi cell having a CD20 molecule on the cell membrane surface was used as a target cell and a human peripheral blood mononuclear cell (PBMC) was used as an effector cell. The human PBMC was prepared from human blood by using Lymphoprep (Axis Shield).
The Daudi cells ($1\times10^4$ cells/50 $\mu$L) were placed in each well of a 96-well U-bottomed plate and $2\times10^5$ cells of human PBMC as the effector cells were added so that the E/T ratio became 20/1. Various anti-CD20 chimeric antibodies were added to this cell solution so as to be the serial dilution line and kept warm at 37°C for 20 hours. After keeping warm, the 96-well U-bottomed plate was subjected to centrifugation and the lactate dehydrogenase activity in the supernatant was measured by using CytoTox 96 Non-Radioactive Cytotoxicity Assay Kit (Promega, G1780). The antibody-dependent and specific cytotoxicity (%) was calculated from the following equation.

$$\%\ \text{Cytotoxicity} = 100 \times (E - SE - ST) / (M - ST)$$

Herein, E denotes an experimental release, which is a lactate dehydrogenase activity released from target cells when an antibody and effector cells are kept warm together with target cells. $S_E$ denotes a lactate dehydrogenase activity spontaneously released from an effector cells. $S_T$ is a lactate dehydrogenase activity also spontaneously released from a target cell. M denotes a lactate dehydrogenase activity released from a target cell at maximum. This is a lactate dehydrogenase activity released from target cells by the addition of lysis solution (9% Triton X-100).
According to the above-mentioned evaluation method, the result of the ADCC activities of the wild type, 294Cys type, 298Cys type, and 301Cys type of anti-CD20 chimeric antibodies are attached to Fig. 4. From Fig. 4, 294Cys type chimeric antibody and 301Cys type chimeric antibody showed an extremely high ADCC activity under the condition in which the antibody concentration was high (0.1 to 10 $\mu$g/mL) as compared with the ADCC activity of the wild type chimeric antibody. The 298Cys type chimeric antibody showed higher ADCC activity in the entire measurement region including low concentration (concentration of 0.1 $\mu$g/mL or less) as compared with that of the wild-type antibody.
Furthermore, the cytotoxicity rates of the anti-CD20 wild type antibody and 298Cys type chimeric antibody were compared with each other by a lactate dehydrogenase release assay. As a result, the cytotoxicity rate when the concentration of the added antibodies was 0.0001, 0.001, 0.01, 0.1, 1, and 10 ng/mL was 6.9%, 10.2%, 25.0%, 33.0%, 36.5%, and 37.9% respectively in the wild type antibody and 10.6%, 12.5%, 33.2%, 39.6%, 42.9%, and 45.2% respectively in the 298Cys type chimeric antibody. Therefore, the ratio of the ADCC activity (the ratio of the cytotoxicity rate of the mutant type antibody to that of the wild type antibody in two points in which the wild type antibody and the mutant type antibody have the same concentration) were shown to be 1.5, 1.2, 1.3, 1.2, 1.2 and 1.2 times, respectively (see Table 8).

[Table 8]

| | Conentration (μg/mL) | | | | | |
|---|---|---|---|---|---|---|
| Cytotoxicity (%) | 0.0001 | 0.001 | 0.01 | 0.1 | 1 | 10 |
| wild type anti-CD20 antibody | 6.9 | 10.2 | 25.0 | 33.0 | 36.5 | 37.9 |
| 298Cys type anti-CD20 antibody | 10.6 | 12.5 | 33.2 | 39.6 | 42.9 | 45.2 |
| 298Cys/wild type (times) | 1.5 | 1.2 | 1.3 | 1.2 | 1.2 | 1.2 |

Furthermore, from Table 8, two points in which the wild type antibody and the 298Cys type chimeric antibody have the same cytotoxicity rate were selected and comparison of the ADCC activity was carried out. For example, the 298Cys type chimeric antibody shows about 40% cytotoxicity at 0.1 μg/mL and the wild type antibody shows about 40% cytotoxicity at 10 μg/mL. When the comparison was carried out by using these two points, the antibody concentration necessary to obtaining the same ADCC activity was reduced to 1/100. Thus, it can be determined that the ADCC activity was increased about 100 times (the ratio of concentration of the mutant type antibody to that of the wild type antibody was increased by 100 times in two points in which the wild type antibody and the mutant type antibody have the same cytotoxicity rate).

Such an increase in the ADCC activity (about 10-100 times) was confirmed in the other concentration range. A person skilled in the art can calculate the increase of the ADCC activity according to the above-mentioned method from Fig. 4 and Table 8.

Furthermore, the cytotoxicity rates of the 294Cys type chimeric antibody and the wild type antibody were compared with each other by a lactate dehydrogenase release assay. As a result, the cytotoxicity rates when the concentration of the added antibodies were 0.00001, 0.0001, 0.001, 0.1, 1, and 10 μg/mL were 4.25, 6.92, 10.19, 33.04, 36.50 and 37.88% respectively in the wild type antibody and 8.41, 9.79, 11.37, 40.75, 74.98 and 83.68% respectively in the 294Cys type chimeric antibody. Therefore, it was shown that the ratio of the ADCC activity (the ratio of the cytotoxicity of the mutant type antibody to that of the wild type antibody in two points in which the wild type antibody and the mutant type antibody have the same concentration) was 2.0, 1.4, 1.1, 1.2, 2.1, and 2.2 times, respectively (see Table 9). Note here that the cytotoxicity rate of 294Cys type chimeric antibody showed such an extremely high value as 83.68% when the concentration was 10 μg/mL.

Furthermore, the cytotoxicity rates of the 301Cys type chimeric antibody and the wild type antibody were compared with each other by a lactate dehydrogenase release assay. As a result, the cytotoxicity rates when the concentration of the added antibodies were 0.00001, 0.0001, 0.1, 1, and 10 μg/mL were 4.25, 6.92, 33.04, 36.50 and 37.88% respectively in the wild type antibody and 7.33, 7.04, 35.68, 75.31, and 78.59% respectively in the 301Cys type chimeric antibody. Therefore, it was shown that the ratio of the ADCC activity (the ratio of the cytotoxicity of the mutant type antibody to that of the wild type antibody in two points in which the wild type antibody and the mutant type antibody have the same concentration) was 1.7, 1.02, 1.1, 2.1, and 2.1 times, respectively (see Table 9).

Note here that the cytotoxicity rate of 301Cys type chimeric antibody showed such an extremely high value as 78.59% when the concentration was 10 μg/mL.

[Table 9]

| | **concentration of anti-CD20 chimeric antibody** (μg/mL) | | | | | | |
|---|---|---|---|---|---|---|---|
| Cytotoxicity (%) | 0.00001 | 0.0001 | 0.001 | 0.01 | 0.1 | 1 | 10 |
| **wild type anti-CD20 antibody** | 4.25 | 6.92 | 10.19 | 25.02 | 33.04 | 36.50 | 37.88 |
| **294Cys type anti-CD20 antibody** | 8.41 | 9.79 | 11.37 | 22.06 | 40.75 | 74.98 | 83.68 |
| **301Cys type anti-CD20 antibody** | 7.33 | 7.04 | 4.92 | 10.41 | 35.68 | 75.31 | 78.59 |
| **294Cys/ wild type (times)** | 2.0 | 1.4 | 1.1 | 0.9 | 1.2 | 2.1 | 2.2 |
| **301Cys/ wild type (times)** | 1.7 | 1.0 | 0.5 | 0.4 | 1.1 | 2.1 | 2.1 |

[Example 2]

By the method similar to Example 1, 290, 291, 292, 302 and 303Cys type anti-CD20 chimeric antibodies were produced. The ADCC activities of these various Cys type anti-CD20 chimeric antibodies were evaluated. As a result, as compared with the ADCC activity of the wild type anti-CD20 chimeric antibody, 290, 291, 292, 302 and 303Cys type anti-CD20 chimeric antibodies showed an extremely high ADCC activity.
Hereinafter, a production method of wild type and five mutant types (290Cys, 291Cys, 292Cys, 302Cys, and 303Cys) of the anti-CD20 chimeric antibody, the ADCC activity measurement thereof and the reactivity with respect to a CD20 molecule are described.

1) Production of anti-CD20 Chimeric Antibody

A chimeric antibody is obtained as a purified chimeric antibody through the following steps A) to F):

A) cloning a gene necessary for production of a chimeric antibody,
B) introducing a mutation of the cloned gene,
C) constructing a chimeric antibody expression vector combining the cloned gene and the mutation-introduced gene,
D) carrying a gene transfer of a chimeric antibody expression vector into a CHO cell and screening a CHO cell highly expressing a chimeric antibody,
E) culturing a CHO cell highly expressing a chimeric antibody, and
F) carrying out column purification from a culture supernatant of a cell highly expressing a chimeric antibody.

In these steps, A) and C) to F) are the same as those in Example 1. Therefore, in this Example, step B) is described.

B) Introducing Mutation o Cloned Gene

In order to obtain 291 Cys chimeric antibody, 292Cys chimeric antibody, and 303Cys chimeric antibody, the mutation needs to be introduced into a certain position of the cloned human IgG1 H chain constant region. According to the number of human IgG1 H chain constant region in Sequence of proteins of Immunological Interest (NIH Publication No. 91-3242, 1991) shown by Elvin A.Kabat, in order to change Pro at a position 291 into Cys, Arg at a position 292 into Cys, and Val at a position 303 into Cys, mutation was introduced by PCR method by using a per2.1 vector into which a cloned human IgG1 H chain constant region had been inserted : pCR2.1-HC (wild type) as a template. This mutation introduction was described in detail as follows.

(1) Mutation Introduction into 290 (Lys → Cys)

| | |
|---|---|
| pCR2.1-HC (wild) (25 ng/μL) | 2 μL |
| 2.5 mM dNTPs | 4 μL |
| 290 Cys1 primer (20 μM) | 1.25 μL |
| 290 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequences of primers follows:

290 Cys1 primer: GTGCATAATGCCAAGACATGCCCGCGTGAGGAGCAGTAC (SEQ ID NO: 210)
290 Cys2 primer: GTACTGCTCCTCACGCGGGCATGTCTTGGCATTATGCAC (SEQ ID NO: 211)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 290 (Lys → Cys) had been introduced.

(2) Mutation Introduction into 291 (Pro → Cys)

| | |
|---|---|
| pCR2.1-HC (wild) (25 ng/μL) | 2 μL |
| 2.5 mM dNTPs | 4 μL |
| 291 Cys1 primer (20 μM) | 1.25 μL |
| 291 Cys2 primer (20 μM) | 1.25 μL |
| × 10 pfu polymerase Buffer | 5 μL |
| pfu polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

291 Cys1 primer: GTGCATAATGCCAAGACATGCCCGCGTGAGGAGCAGTAC (SEQ ID NO: 212)
291 Cys2 primer: GTACTGCTCCTCACGCGGGCATGTCTTGGCATTATGCAC (SEQ ID NO: 213)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 291 (Pro → Cys) had been introduced.

(3) Mutation Introduction into 292 (Arg → Cys)

| | |
|---|---|
| pCR2.1-HC (wild) (25 ng/μL) | 2 μL |
| 2.5 mM dNTPs | 4 μL |
| 292 Cys1 primer (20 μM) | 1.25 μL |
| 292 Cys2 primer (20 μM) | 1.25 μL |

(continued)

| | |
|---|---|
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

292 Cys1 primer: GCCAAGACAAAGCCGTGTGAGGAGCAGTACAAC (SEQ ID NO: 214)
292 Cys2 primer: GTTGTACTGCTCCTCACACGGCTTTGTCTTGGC (SEQ ID NO: 215)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 292 (Arg → Cys) had been introduced.

(4) Mutation Introduction into 302 (Val → Cys)

| | |
|---|---|
| pCR2.1-HC (wild) (25 ng/μL) | 2 μL |
| 2.5 mM dNTPs | 4 μL |
| 290 Cys1 primer (20 μM) | 1.25 μL |
| 290 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

302 Cys1 primer: GCCAAGACAAAGCCGTGTGAGGAGCAGTACAAC (SEQ ID NO: 216)
302 Cys2 primer: GTTGTACTGCTCCTCACACGGCTTTGTCTTGGC (SEQ ID NO: 217)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 302 (Val → Cys) had been introduced.

(5) Mutation Introduction into 303 (Val →Cys)

| | |
|---|---|
| pCR2.1-HC (wild) (25 ng/μL) | 2 μL |
| 2.5 mM dNTPs | 4 μL |
| 303 Cys1 primer (20 μM) | 1.25 μL |
| 303 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

303 Cys1 primer: GCCAAGACAAAGCCGTGTGAGGAGCAGTACAAC (SEQ ID NO: 218)
303 Cys2 primer: GTTGTACTGCTCCTCACACGGCTTTGTCTTGGC (SEQ ID NO: 219)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 303 (Val → Cys) had been introduced.

< Flow Cytometric Analysis of Various Kinds of Purified Chimeric Antibodies >

Flow Cytometric Analysis of various kinds of purified chimeric antibodies was carried out according to the same method as that described in Example 1.

< Evaluation of ADCC Activity of Various Kinds of Chimeric Antibody >

Evaluation of the Antibody-dependent Cellular Cytotoxicity (ADCC) activity of various kinds of chimeric antibodies was also carried out by the same method described in Example 1. The results of the ADCC activity of the wild type, 290Cys, 291Cys, 292Cys type anti-CD20 chimeric antibodies are shown in Fig. 5. Furthermore, the results of the ADCC activity of the wild type, 302Cys, 303Cys type anti-CD20 chimeric antibodies are shown in Fig. 6. From Figs. 5 and 6, the 291Cys, 292Cys, and 303Cys type chimeric antibodies showed higher ADCC activity in the entire region including a low concentration (at 0.1 μg/mL or less) as compared with that of the wild type. Furthermore, 290Cys and 302Cys showed higher ADCC activity as compared with that of the wild type.

[Table 10]

| | **concentration of anti-CD20 chimeric antibody** (μg/mL) | | | | | | |
|---|---|---|---|---|---|---|---|
| | 0.00001 | 0.0001 | 0.001 | 0.01 | 0.1 | 1 | 10 |
| Wild | 12.7 | 13.7 | 14.2 | 17.2 | 27.3 | 35.5 | 49.5 |
| 290Cys | 11.9 | 18.0 | 19.5 | 26.6 | 40.9 | 41.6 | 41.2 |
| 291Cys | 11.3 | 17.9 | 26.1 | 65.7 | 73.9 | 74.7 | 64.7 |
| 292Cys | 13.9 | 14.7 | 15.2 | 34.8 | 58.5 | 78.7 | 79.1 |
| **290Cys / wild type (times)** | 0.9 | 1.3 | 1.4 | 1.5 | 1.5 | 1.2 | 0.8 |
| **291Cys / wild type (times)** | 0.9 | 1.3 | 1.8 | 3.8 | 2.7 | 2.1 | 1.3 |
| **292Cys / wild type (times)** | 1.1 | 1.1 | 1.1 | 2.0 | 2.1 | 2.2 | 1.6 |

Furthermore, the cytotoxicity rates of the anti-CD20 wild type antibody and 290Cys type chimeric antibody were compared with each other by a lactate dehydrogenase release assay. As a result, the cytotoxicity rate when the concentration of the added antibodies was 0.0001, 0.001, 0.01, 0.1, and 1 μg/mL was 13.7%, 14.2%, 17.2%, 27.3% and 35.5% respectively in the wild type antibody and 18.0%, 19.5%, 26.6%, 40.9% and 41.6% respectively in the 290Cys type chimeric antibody. Therefore, the ratio of the ADCC activity (the ratio of the cytotoxicity rate of the mutant type antibody to the wild type antibody in two points in which the wild type antibody and the mutant type antibody have the same concentration) were shown to be 1.3, 1.4, 1.5, 1.5, and 1.2 times, respectively (see Table 10).

Furthermore, from Table 10, two points in which the wild type antibody and the 290Cys type chimeric antibody have the same cytotoxicity rate were selected and comparison of the ADCC activity was carried out. For example, the 290Cys type chimeric antibody shows about 18% cytotoxicity at 0.0001 μg/mL and the wild type antibody shows about 17% cytotoxicity at 0.01 μg/mL. When the comparison was carried out by using these two points, the antibody concentration necessary to obtaining the same ADCC activity was reduced to about 1/100. It can be determined that the ADCC activity was increased about 100 times (the ratio of concentration of the mutant type antibody to that of the wild type antibody was increased by 100 times in two points in which the wild type antibody and the mutant type antibody have the same cytotoxicity rate).

A person skilled in the art can calculate the increase of the ADCC activity according to the above-mentioned method from Fig. 5 and Table 10.

Furthermore, the cytotoxicity rates of anti-CD20 wild type antibody and 291Cys type chimeric antibody were compared with each other by a lactate dehydrogenase release assay. As a result, the cytotoxicity rates when the concentration of the added antibodies were 0.0001, 0.001, 0.01, 0.1, 1, and 10 μg/mL were 13.7%, 14.2%, 17.2%, 27.3%, 35.5%, and 49.5% respectively in the wild type antibody, and 17.9%, 26.1%, 65.7%, 73.9%, 74.7% and 64.7% respectively in the 291 Cys type chimeric antibody. Therefore, it was shown that the ratio of the ADCC activity (the the ratio of the cytotoxicity of the mutant type antibody to that of the wild type antibody in two points in which the wild type antibody and the mutant type antibody have the same concentration) was 1.3, 1.8, 3.8, 2.7, 2.1, and 1.3 times, respectively (see Table 10).

Furthermore, from Fig. 5, two points in which the wild type antibody and the 291 Cys type chimeric antibody have the same cytotoxicity rate were selected and comparison of the ADCC activity was carried out. For example, the 291Cys type chimeric antibody shows about 65.7% cytotoxicity at 0.01 μg/mL and the wild type antibody shows about 50% cytotoxicity at 10 μg/mL. When the comparison was carried out by using these two points, antibody concentration necessary to obtaining the same ADCC activity was reduced to 1/1000 or less. It can be determined that the ADCC activity was increased 1000 times or more (the ratio of concentration of the mutant type antibody to that of the wild type antibody was increased by 2000 times in two points in which the wild type antibody and the mutant type antibody have the same cytotoxicity rate).

A person skilled in the art can calculate the increase of the ADCC activity according to the above-mentioned method from Fig. 5 and Table 10.

Furthermore, the cytotoxicity rates of the anti-CD20 wild type antibody and 292Cys type chimeric antibody were compared with each other by a lactate dehydrogenase release assay. As a result, the cytotoxicity rates when the concentration of the added antibodies were 0.00001, 0.0001, 0.001, 0.01, 0.1, 1 and 10 μg/mL were 12.7, 13.7, 14.2,

17.2, 27.3, 35.5 and 49.5% respectively in the wild type antibody and 13.9, 14.7, 15.2, 34.8, 58.5, 78.7 and 79.1% respectively in the 292Cys type chimeric antibody. Therefore, it was shown that the ratio of the ADCC activity (the ratio of the cytotoxicity of the mutant type antibody to that of the wild type antibody in two points in which the wild type antibody and the mutant type antibody have the same concentration) was 1.1, 1.1, 1.1, 2.0, 2.1, 2.2 and 1.6 times, respectively (see Table 10).

Furthermore, from Fig. 5, two points in which the wild type antibody and the 292Cys type chimeric antibody have the same cytotoxicity rate were selected and comparison of the ADCC activity was carried out. For example, the 292Cys type chimeric antibody shows about 58.5% cytotoxicity at 0.1 μg/mL and the wild type antibody shows about 50% cytotoxicity at 10 μg/mL. When the comparison was carried out by using these two points, antibody concentration necessary to obtaining the same ADCC activity was reduced to 1/100. It can be determined that the ADCC activity was increased about 100 times (the ratio of concentration of the mutant type antibody to that of the wild type antibody was increased by 100 times in two points in which the wild type antibody and the mutant type antibody have the same cytotoxicity rate).

Such an increase in the ADCC activity (about 10-100 times) was confirmed in the other concentration range. A person skilled in the art can calculate the increase of the ADCC activity according to the above-mentioned method from Fig. 5 and Table 10.

[Table 11]

| | **concentration of anti-CD20 chimeric antibody** (μg/mL) | | | | | | |
|---|---|---|---|---|---|---|---|
| | 0.00001 | 0.0001 | 0.001 | 0.01 | 0.1 | 1 | 10 |
| **Wild** | 5.8 | 3.4 | 7.6 | 14.7 | 30.5 | 34.3 | 42.5 |
| **302Cys** | 7.9 | 9.6 | 11.3 | 19.6 | 39.9 | 42.9 | 42.9 |
| **303Cys** | 6.5 | 7.7 | 14.5 | 46.6 | 74.5 | 87.1 | 89.3 |
| **302Cys / wild type (times)** | 1.4 | 2.8 | 1.5 | 1.3 | 1.3 | 1.3 | 1.0 |
| **303Cys / wild type (times)** | 1.1 | 2.3 | 1.9 | 3.2 | 2.4 | 2.5 | 2.1 |

Furthermore, the cytotoxicity rates of the anti-CD20 wild type antibody and 302Cys type chimeric antibody were compared with each other by a lactate dehydrogenase release assay. As a result, the cytotoxicity rate when the ) concentration of the added antibodies was 0.00001, 0.0001, 0.001, 0.01, 0.1, 1 and 10 μg/mL was 5.8, 3.4, 7.6, 14.7, 30.5, 34.3 and 42.5% respectively in the wild type antibody and 7.9, 9.6, 11.3, 19.6, 39.9, 42.9 and 42.9% respectively in the 302Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 1.4, 2.8, 1.5, 1.3, 1.3, 1.3 and 1.0 times, respectively (see Table 11).

Furthermore, from Fig. 6, two points in which the wild type antibody and the 302Cys type chimeric antibody have the same cytotoxicity rate were selected and comparison of the ADCC activity was carried out. For example, the 302Cys type chimeric antibody shows about 40% cytotoxicity at 0.1 μg/mL and the wild type antibody shows about 40% cytotoxicity at 10 μg/mL. When the comparison was carried out by using these two points, antibody concentration necessary to obtaining the same ADCC activity was reduced to 1/100. It can be determined that the ADCC activity was increased about 100 times (the ratio of concentration of the mutant type antibody to that of the wild type antibody was increased by 100 times in two points in which the wild type antibody and the mutant type antibody have the same cytotoxicity rate).

Such an increase in the ADCC activity (about 10-100 times) was confirmed in the other concentration range. A person skilled in the art can calculate the increase of the ADCC activity according to the above-mentioned method from Fig. 6 and Table 11.

Furthermore, the cytotoxicity rates of the anti-CD20 wild type antibody and 303Cys type chimeric antibody were compared with each other by a lactate dehydrogenase release assay. As a result, the cytotoxicity rates when the concentration of the added antibodies were 0.00001, 0.0001, 0.001, 0.01, 0.1, 1 and 10 μg/mL were 5.8, 3.4, 7.6, 14.7, 30.5, 34.3 and 42.5% respectively in the wild type antibody and 6.5, 7.7, 14.5, 46.6, 74.5, 87.1 and 89.3% respectively in the 303Cys type chimeric antibody. Therefore, it was shown that the ratio of the ADCC activity (the ratio of the cytotoxicity of the mutant type antibody to that of the wild type antibody in two points in which the wild type antibody and the mutant type antibody have the same concentration) was 1.1, 2.3, 1.9, 3.2, 2.4, 2.5 and 2.1 times, respectively (see Table 11).

Furthermore, from Fig. 6, two points in which the wild type antibody and the 303Cys type chimeric antibody have the same cytotoxicity rate were selected and comparison of the ADCC activity was carried out. For example, the 303Cys type chimeric antibody shows about 40% cytotoxicity at 0.01 μg/mL and the wild type antibody shows about 40% cytotoxicity at 10 μg/mL. When the comparison was carried out by using these two points, antibody concentration necessary to obtaining the same ADCC activity was reduced to 1/1000. It can be determined that the ADCC activity was increased about 1000 times (the ratio of concentration of the mutant type antibody to that of the wild type antibody was increased by 1000 times in two points in which the wild type antibody and the mutant type antibody have the same cytotoxicity rate).
A person skilled in the art can calculate the increase of the ADCC activity according to the above-mentioned method from Fig. 6 and Table 11.

[Example 3]

(Production of Anti-EGFR Humanized Antibody)

In order to express wild type and 298Cys type anti-EGFR humanized antibodies in CHO cells, three kinds of expression vectors (anti-EGFR humanized antibody L chain expression vector, wild type anti-EGFR humanized antibody H chain expression vector, and 298Cys type anti-EGFR humanized antibody H chain expression vector) were constructed as an expression vector. Hereinafter, the construction of each expression vector is described in detail.

1) Construction of Anti-EGFR Humanized Antibody L Chain Expression Vector

A DNA fragment was obtained by PCR reaction using the below-mentioned primers using a gene containing a variable region and a constant region of a human-type anti-EGFR antibody L chain as a template.

EGFR-L1 primer:
ACCGCTCGAGATGGACATGAGGGTCCCCGCTCAGCTC (SEQ ID NO: 220)
EGFR-L2 primer:
ATAGTTTAGCGGCCGCTTACGAACATTCTGTAGGGGCCACTGTCTT (SEQ ID NO: 221)

A DNA fragment obtained by PCR reaction was purified by ethanol precipitation and then treated with restriction enzymes Xho I (TAKARA BIO, 1094A) and Not I (TAKARA BIO, Code 1166A). A BCMG-neo vector was also treated with restriction enzymes XhoI and NotI. Next, the PCR amplified DNA fragment and the BCMG-neo vector treated with the restriction enzymes were subjected to electrophoresis by using 1% agarose gel containing ethidium bromide. After electrophoresis, a DNA fragment having an intended size among the DNA fragments visualized by UV irradiation was cut out from the gel, a DNA fragment was extracted and purified from a gel by using SUPREC-01 (TAKARA BIO, code 9040). The PCR amplified fragment and the BCMG-neo vector, which had been cut out from a gel and purified, were subjected to ligation reaction using a DNA Ligation kit Ver.2.1 (TAKARA BIO, code 6022). A DNA solution after ligation reaction was used for transformation into Escherichia coli JM109. From the obtained transformant, a plasmid DNA was purified by an alkali SDS method. The purified plasmid was subjected to sequence analysis so as to confirm that an L chain (variable region and constant region) gene of the anti-EGFR humanized antibody was included. This was defined as an anti-EGFR humanized antibody L chain expression vector.
Base sequences of CDR1, CDR2 and CDR3 of the anti-EGFR humanized antibody L chain variable region are shown in SEQ ID NOs: 200, 202 and 204, respectively. Furthermore, amino acid sequences of CDR1, CDR2 and CDR3 of the anti-EGFR humanized antibody L chain variable region are shown in SEQ ID NOs: 201, 203 and 205, respectively.

2) Construction of Wild Type Anti-EGFR Humanized Antibody H Chain Expression Vector and 298Cys Type Anti-EGFR Humanized Antibody H Chain Expression Vector

A DNA fragment was obtained by PCR reaction using the below-mentioned primers using a gene containing a variable region of the human-type anti-EGFR antibody H chain as a template.

EGFR-H1 primer: ACCGCTCGAGATGAAACACCTGTGGTTCTTCCTC (SEQ ID NO: 222)
EGFR-H2 primer: CGAGACGGTGACCATTGTCCCTTG(5'-phosphorylated) (SEQ ID NO: 223)

A DNA fragment obtained by PCR reaction was purified by ethanol precipitation and then treated with restriction enzymes Xho I (TAKARA BIO, 1094A). Next, the PCR amplified DNA fragment treated with the restriction enzyme was subjected to electrophoresis by using 1% agarose gel containing ethidium bromide. After electrophoresis, a DNA fragment having an intended size among the DNA fragments visualized by UV irradiation was cut out from the gel, a DNA fragment was extracted and purified from a gel by using SUPREC-01 (TAKARA BIO, code 9040). This purified DNA fragment is an anti-EGFR humanized antibody H chain variable region gene.
Base sequences of CDR1, CDR2 and CDR3 of the anti-EGFR humanized antibody H chain variable region are shown in SEQ ID NOs: 194, 196 and 198, respectively. Furthermore, amino acid sequences of CDR1, CDR2 and CDR3 of the anti-EGFR humanized antibody H chain variable region are shown in SEQ ID NOs: 195, 197 and 199, respectively.
As the human IgG1 H chain constant region gene necessary to production of the anti-EGFR humanized antibody, the same one as the DNA fragment used in producing the anti-CD20 chimeric antibody was used. A DNA fragment was obtained by PCR amplification using the H3 primer and H4 primer by using a plasmid pCR2.1-HC (wild) as a template. The DNA fragment was treated with a restriction enzyme Not I and then purified. This DNA fragment was a wild type anti-EGFR humanized antibody H chain constant region gene. A DNA fragment obtained by PCR amplification using the H3 primer and H4 primer by using a plasmid pCR2.1-HC (298Cys) as a template was treated with a restriction enzyme NotI and then purified, which was a 298Cys type anti-EGFR humanized antibody H chain constant region gene.
DNA fragments obtained by cutting and purifying the anti-EGFR humanized antibody H chain variable region gene fragment, wild type anti-EGFR humanized antibody H chain constant region gene fragment and BCMG-neo vector prepared as mentioned above with Xho I and Not I were mixed with each other and subjected to ligation reaction by using a DNA Ligation kit Ver.2.1 (TAKARA BIO, code 6022). A DNA solution after the ligation reaction was used for transformation into Escherichia coli JM109. From the obtained transformant, a plasmid DNA was purified by an alkali SDS method. The purified plasmid was subjected to sequence analysis so as to confirm that a wild type H chain (variable region and constant region) gene of the anti-EGFR humanized antibody was included. This was defined as a wild type anti-EGFR humanized antibody H chain expression vector. Similarly, DNA fragments obtained by cutting and purifying the anti-EGFR humanized antibody H chain variable region gene fragment, 298Cys type anti-EGFR humanized antibody H chain constant region gene fragment and BCMG-neo vector with Xho I and Not I were mixed with each other and subjected to ligation reaction by using a DNA Ligation kit Ver.2.1 (TAKARA BIO, code 6022). A DNA solution after the ligation reaction was used for transformation into Escherichia coli JM109. From the obtained transformant, a plasmid DNA was purified by an alkali SDS method. The purified plasmid was subjected to sequence analysis so as to confirm that a 298Cys type H chain (variable region and constant region) gene of the anti-EGFR humanized antibody was included. This was defined as a 298Cys type anti-EGFR humanized antibody H chain expression vector.

3) Expression of Wild Type Anti-EGFR Humanized Antibody and 298Cys Type Anti- EGFR Humanized Antibody in CHO Cells and Purification

An anti-EGFR humanized antibody L chain expression vector and a wild type anti-EGFR humanized antibody H chain expression vector were transfected in CHO cells so as to allow a wild type anti-EGFR humanized antibody to be produced in the CHO cells. Similarly, an anti-EGFR humanized antibody L chain expression vector and a 298Cys type anti-EGFR humanized antibody H chain expression vector were transfected in CHO cells so as to allow a 298Cys type anti-EGFR humanized antibody to be produced in the CHO cells. CHO cells highly expressing the 298Cys type anti-EGFR humanized antibody were selected and the selected high-expression CHO cells were cultured in a serum-free medium CHO-S-SFMII medium. After culture, a culture supernatant was subjected to ProteinA column purification. Thus, a high purity anti-EGFR humanized antibody was obtained.

[Table 12]

| | **concentration** (μg/mL) | | | | | | |
|---|---|---|---|---|---|---|---|
| | 0.00001 | 0.0001 | 0.001 | 0.01 | 0.1 | 1 | 10 |
| **Wild** | 3.0 | 3.3 | 5.8 | 6.1 | 10.2 | 11.6 | 10.9 |
| **298Cys** | 4.6 | 6.7 | 8.7 | 11.0 | 18.8 | 20.6 | 20.2 |
| **298Cys/Wild(times)** | 1.5 | 2.0 | 1.5 | 1.8 | 1.8 | 1.8 | 1.9 |

Furthermore, the cytotoxicity rates of the anti-EGFR wild type antibody and the 298Cys type chimeric antibody were compared with each other by a lactate dehydrogenase release assay. As a result, the cytotoxicity rate when the concentration of the added antibodies was 0.00001, 0.0001, 0.001, 0.01, 0.1, 1 and 10 μg/mL was 3.0, 3.3, 5.8, 6.1, 10.2, 11.6 and 10.9%, respectively in the wild type antibody, and 4.6, 6.7, 8.7, 11.0, 18.8, 20.6 and 20.2%, respectively in the 298Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 1.5, 2.0, 1.5, 1.8, 1.8, 1.8 and 1.9 times, respectively (see Table 12).

Furthermore, from Fig. 7, two points in which the wild type antibody and the 298Cys type chimeric antibody have the same cytotoxicity rate were selected and comparison of the ADCC activity was carried out. For example, the 298Cys type chimeric antibody shows about 40% cytotoxicity at 0.01 μg/mL and the wild type antibody shows about 40% cytotoxicity at 10 μg/mL. When the comparison was carried out by using these two points, the antibody concentration necessary to obtaining the same ADCC activity was reduced to 1/1000. It can be determined that the ADCC activity was increased about 1000 times (the ratio of concentration of the mutant type antibody to that of the wild type antibody was increased by 1000 times in two points in which the wild type antibody and the mutant type antibody have the same cytotoxicity rate).
A person skilled in the art can calculate the increase of the ADCC activity according to the above-mentioned method from Fig. 7 and Table 12.

[Example 4]

By the same method as in Example 2, 286, 287, 288, 289, 305, 306, 307 and 308Cys type anti-CD20 chimeric antibodies were produced. The ADCC activities of these kinds of Cys type anti-CD20 chimeric antibodies were evaluated. As a result, as compared with the ADCC activity of the wild type anti-CD20 chimeric antibody, the 286, 287, 288, 289, 305, 306, 307 and 308Cys type anti-CD20 chimeric antibodies showed extremely high ADCC activity.
Hereinafter, a production method of wild type and five kinds mutant types (286 Cys type, 287 Cys type,288 Cys type, 289 Cys type, 305 Cys type, 306 Cys type, 307 Cys type and 308Cys type) of the anti-CD20 chimeric antibody and the ADCC activity measurement thereof and the reactivity with respect to a CD20 molecule are described.

<u>1) Production of anti-CD20 chimeric antibody</u>

A chimeric antibody is obtained as a purified chimeric antibody through the following steps A) to F).

A) cloning a gene necessary for production of a chimeric antibody,
B) introducing a mutation of the cloned gene,
C) constructing a chimeric antibody expression vector combining the cloned gene and the mutation-introduced gene,
D) carrying a gene transfer of a chimeric antibody expression vector into a CHO cell and screening a CHO cell highly expressing a chimeric antibody,
E) culturing a CHO cell highly expressing a chimeric antibody, and
F) carrying out column purification from a culture supernatant of a cell highly expressing a chimeric antibody.

In these steps, A) and C) to F) are the same as those in Example 1. Therefore, in this Example, step B) is described.

<u>B) Introducing a mutation of Cloned Gene</u>

In order to obtain 286Cys chimeric antibody, 287Cys chimeric antibody, 288Cys chimeric antibody, 289Cys chimeric antibody, 305Cys chimeric antibody, 306Cys chimeric antibody, 307Cys chimeric antibody, and 308Cys chimeric antibody, the mutation needs to be introduced into a certain position of the cloned human IgG1 H chain constant region. According to the number of human IgG1 H chain constant region in Sequence of proteins of Immunological Interest (NIH Publication No. 91-3242, 1991) shown by Elvin A.Kabat, in order to change Asn at a position 286 into Cys, Ala at a position 287 into Cys, Lys at a position 288 into Cys, Thr at a position 289 into Cys, Val at a position 305 into Cys, Leu at a position 306 into Cys, Thr at a position 307 into Cys, and Val at a position 308 into Cys, mutation was introduced by PCR method by using a pCR2.1 vector into which a cloned hunan IgG1 H chain constant region had been inserted : pCR2.1-HC (wild type) as a template. The detail of this mutation introduction was described as follows.

<u>286 (Asn → Cys) Mutation</u>

| | |
|---|---|
| pCR2.1-HC (wild) (25 ng/μL) | 2 μL |
| 2.5 mM dNTPs | 4 μL |
| 286 Cys1 primer (20 μM) | 1.25 μL |
| 286 Cys2 primer (20 μM) | 1.25 μL |
| × 10 pfu polymerase Buffer | 5 μL |
| pfu DNA polymerase | 1 μL |

(continued)

| | |
|---|---|
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequences of primers follows:

286Cys1 primer: gacggcgtggaggtgcattgtgccaagacaaagccgcgt (SEQ ID NO: 224)
286Cys2 primer: acgcggctttgtcttggcacaatgcacctccacgccgtc (SEQ ID NO: 225)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 286 (Asn → Cys) had been introduced.

287 (Ala → Cys) Mutation

| | |
|---|---|
| pCR2.1-HC (wild) (25 ng/μL) | 2 μL |
| 2.5 mM dNTPs | 4 μL |
| 287 Cys1 primer (20 μM) | 1.25 μL |
| 287 Cys2 primer (20 μM) | 1.25 μL |
| × 10 pfu polymerase Buffer | 5 μL |
| pfu polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

287Cys1 primer: ggcgtggaggtgcataattgcaagacaaagccgcgtaag (SEQ ID NO: 226)
287Cys2 primer: cttacgcggctttgtcttgcaattatgcacctccacgcc (SEQ ID NO: 227)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 287 (Ala → Cys) had been introduced.

288 (Lys → Cys) Mutation

| | |
|---|---|
| pCR2.1-HC (wild) (25 ng/μL) | 2 μL |
| 2.5 mM dNTPs | 4 μL |
| 288 Cys1 primer (20 μM) | 1.25 μL |
| 288 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

288Cys1 primer: gtggaggtgcataatgcctgtacaaagccgcgtgaggag (SEQ ID NO: 228)
288Cys2 primer: ctcctcacgcggctttgtacaggcattatgcacctccac (SEQ ID NO: 229)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 288 (Lys → Cys) had been introduced.

289 (Thr → Cys) Mutation

| | |
|---|---|
| pCR2.1-HC (wild) (25 ng/μL) | 2 μL |
| 2.5 mM dNTPs | 4 μL |
| 289 Cys1 primer (20 μM) | 1.25 μL |
| 289 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

289 Cys1 primer: gaggtgcataatgccaagtgcaagccgcgtgaggagcag (SEQ ID NO: 230)
289 Cys2 primer: ctgctcctcacgcggcttgcacttggcattatgcacctc (SEQ ID NO: 231)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 289 (Thr → Cys) had been introduced.

305 (Val → Cys) Mutation

| | |
|---|---|
| pCR2.1-HC (wild) (25 ng/μL) | 2 μL |
| 2.5 mM dNTPs | 4 μL |
| 305 Cys1 primer (20 μM) | 1.25 μL |
| 305 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

305 Cys1 primer: acgtaccgtgtggtcagctgcctcaccgtcctgcaccag (SEQ ID NO: 232)
305 Cys2 primer: ctggtgcaggacggtgaggcagctgaccacacggtacgt (SEQ ID NO: 233)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 305 (Val → Cys) had been introduced.

306 (Leu → Cys) Mutation

| | |
|---|---|
| pCR2.1-HC (wild) (25 ng/μL) | 2 μL |
| 2.5 mM dNTPs | 4 μL |
| 306 Cys1 primer (20 μM) | 1.25 μL |
| 306 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

306 Cys1 primer: taccgtgtggtcagcgtctgcaccgtcctgcaccaggac (SEQ ID NO: 234)
306 Cys2 primer: gtcctggtgcaggacggtgcagacgctgaccacacggta (SEQ ID NO: 235)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 306 (Leu → Cys) had been introduced.

307Cys (Leu → Cys) Mutation

| | |
|---|---|
| pCR2.1-HC (wild) (25 ng/μL) | 2 μL |
| 2.5 mM dNTPs | 4 μL |
| 307 Cys1 primer (20 μM) | 1.25 μL |
| 307 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min

4°C, unlimited time

Base sequence of primers follows:

307 Cys1 primer: cgtgtggtcagcgtcctctgcgtcctgcaccaggactgg (SEQ ID NO: 236)
307 Cys2 primer: ccagtcctggtgcaggacgcagaggacgctgaccacacg (SEQ ID NO: 237)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 307Cys (Th → Cys) had been introduced.

308 (Val → Cys) Mutation

| | |
|---|---|
| pCR2.1-HC (wild) (25 ng/μL) | 2 μL |
| 2.5 mM dNTPs | 4 μL |
| 308 Cys1 primer (20 μM) | 1.25 μL |
| 308 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequence of primers follows:

308 Cys1 primer: gtggtcagcgtcctcacctgcctgcaccaggactggctg (SEQ ID NO: 238)
308 Cys2 primer: cagccagtcctggtgcaggcaggtgaggacgctgaccac (SEQ ID NO: 239)

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 308 (Val → Cys) had been introduced.

< Flow Cytometric Analysis of Various Kinds of Purified Chimeric Antibodies >

Flow Cytometric Analysis of various kinds of purified chimeric antibodies was carried out according to the same method as that described in Example 1.

< Evaluation of ADCC Activity of Various Kinds of Chimeric Antibody >

< 286Cys mutant antibody>

Furthermore, the cytotoxicity rates of the anti-CD20 wild type antibody and 286Cys type chimeric antibody were compared with each other by a lactate dehydrogenase release assay. As a result, the cytotoxicity rate when the concentration of the added antibodies was 0.00001, 0.0001, 0.001, 0.01, 0.1, 1 and 10 μg/mL was 0.0, 0.9, 2.2, 8.7, 26.7, 33.5 and 34.8% respectively in the wild type antibody and 0.8, 2.8, 8.1, 26.9, 82.2, 88.5 and 87.5% respectively in the 286Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 41.0, 3.0, 3.7, 3.1, 3.1, 2.6 and 2.5 times, respectively (see Table 13).
[Table 13]

| | | **concentration of anti-CD20 chimeric antibody (μg/mL)** | | | | | |
|---|---|---|---|---|---|---|---|
| **cytotoxicity(%)** | 0.00001 | 0.0001 | 0.001 | 0.01 | 0.1 | 1 | 10 |
| Wild | 0.02 | 0.9 | 2.2 | 8.7 | 26.7 | 33.5 | 34.8 |
| 286Cys | 0.8 | 2.8 | 8.1 | 26.9 | 82.2 | 88.5 | 87.5 |
| 287Cys | 0.2 | 0.5 | 1.5 | 16.5 | 34.9 | 36.9 | 45.5 |
| 288Cys | 0.5 | 1.2 | 0.5 | 9.4 | 33.5 | 37.5 | 36.5 |
| 289Cys | 0.5 | 0.5 | 1.6 | 29.1 | 87.2 | 85.2 | 80.6 |
| **286Cys / wild type (times)** | 41.0 | 3.0 | 3.7 | 3.1 | 3.1 | 2.6 | 2.5 |
| **287Cys / wild type (times)** | 9.7 | 0.5 | 0.7 | 1.9 | 1.3 | 1.1 | 1.9 |
| **288Cys / wild type (times)** | 24.3 | 1.3 | 0.2 | 1.1 | 1.3 | 1.1 | 1.1 |
| **289Cys / wild type (times)** | 22.8 | 0.5 | 0.7 | 3.3 | 3.3 | 2.5 | 2.3 |

Furthermore, from Table 13, two points in which the wild type antibody and the 286Cys type chimeric antibody have the same cytotoxicity rate were selected and comparison of the ADCC activity was carried out. For example, the 286Cys type chimeric antibody shows about 26.9% cytotoxicity at 0.01 μg/mL and the wild type antibody shows about 26.7% cytotoxicity at 0.1 μg/mL. When the comparison was carried out by using these two points, antibody concentration necessary to obtaining the same ADCC activity was reduced to about 1/100. It can be determined that the ADCC activity was increased about 10 times (the ratio of concentration of the mutant type antibody to that of the wild type antibody was increased by about 10 times in two points in which the wild type antibody and the mutant type antibody have the same cytotoxicity rate).
A person skilled in the art can calculate the increase of the ADCC activity according to the above-mentioned method from Fig. 8.

< 287Cys mutant antibody >

Furthermore, the cytotoxicity rates of the anti-CD20 wild type antibody and 287Cys type chimeric antibody were compared with each other by a lactate dehydrogenase release assay. As a result, the cytotoxicity rate when the concentration of the added antibodies was 0.00001, 0.0001, 0.001, 0.01, 0.1, 1 and 10 μg/mL was 0.02, 0.9, 2.2, 8.7, 26.7, 33.5 and 34.8% respectively in the wild type antibody and 0.2, 0.5, 1.5, 16.5, 34.9, 36.9 and 45.5% respectively in the 287Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 9.7, 0.5, 0.7, 1.9, 1.3, 1.1, and 1.3 times, respectively (see Table 13).
Furthermore, from Table 13, two points in which the wild type antibody and the 287Cys type chimeric antibody have the same cytotoxicity rate were selected and comparison of the ADCC activity was carried out. For example, the 287Cys type chimeric antibody shows about 34.9% cytotoxicity at 0.1 μg/mL and the wild type antibody shows 34.8% cytotoxicity at 10 μg/mL. When the comparison was carried out by using these two points, antibody concentration necessary to obtaining the same ADCC activity was reduced to about 1/100. It can be determined that the ADCC activity was increased about 100 times (the ratio of concentration of the mutant type antibody to that of the wild type antibody was increased by about 100 times in two points in which the wild type antibody and the mutant type antibody have the same cytotoxicity rate).
A person skilled in the art can calculate the increase of the ADCC activity according to the above-mentioned method from Fig. 8.

< 288Cys mutant antibody>

Furthermore, the cytotoxicity rates of the anti-CD20 wild type antibody and 288Cys type chimeric antibody were compared with each other by a lactate dehydrogenase release assay. As a result, the cytotoxicity rate when the concentration of the added antibodies was 0.00001, 0.0001, 0.001, 0.01, 0.1, 1 and 10 μg/mL was 0.02, 0.9, 2.2, 8.7, 26.7, 33.5 and 34.8% respectively in the wild type antibody and 0.5, 1.2, 0.5, 9.4, 33.5, 37.5 and 36.5% respectively in the 288Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 24.3, 1.3, 0.2, 1.1, 1.3, 1.1, and 1.1 times, respectively (see Table 13).
Furthermore, from Table 13, two points in which the wild type antibody and the 288Cys type chimeric antibody have the same cytotoxicity rate were selected and comparison of the ADCC activity was carried out. For example, the 288Cys type chimeric antibody shows about 33.5% cytotoxicity at 0.1 μg/mL and the wild type antibody shows about 33.5% cytotoxicity at 1 μg/mL. When the comparison was carried out by using these two points, antibody concentration necessary to obtaining the same ADCC activity was reduced to about 1/100. It can be determined that the ADCC activity was increased about 10 times (the ratio of concentration of the mutant type antibody to that of the wild type antibody was increased by about 10 times in two points in which the wild type antibody and the mutant type antibody have the same cytotoxicity rate).
A person skilled in the art can calculate the increase of the ADCC activity according to the above-mentioned method from Fig. 8.

<289Cys mutant antibody >

Furthermore, the cytotoxicity rates of the anti-CD20 wild type antibody and 289Cys type chimeric antibody were compared with each other by a lactate dehydrogenase release assay. As a result, the cytotoxicity rate when the concentration of the added antibodies was 0.00001, 0.00001, 0.0001, 0.001, 0.01, 0.1, 1, and 10 μg/mL was 0.02, 0.9, 2.2, 8.7, 26.7, 33.5 and 34.8% respectively in the wild type antibody and 0.5, 0.5, 1.6, 29.1, 87.2, 85.2 and 80.6% respectively in the 289Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 22.8, 0.5, 0.7, 3.3, 3.3, 2.5 and 2.3 times, respectively (see Table 13).
Furthermore, from Table 13, two points in which the wild type antibody and the 289Cys type chimeric antibody have the same cytotoxicity rate were selected and comparison of the ADCC activity was carried out. For example, the 289Cys type chimeric antibody shows about 29.1 % cytotoxicity at 0.01 μg/mL and the wild type antibody shows about 26.7% cytotoxicity at 0.1 μg/mL. When the comparison was carried out by using these two points, antibody concentration necessary to obtaining the same ADCC activity was reduced to about 1/10. It can be determined that the ADCC activity was increased about 10 times (the ratio of concentration of the mutant type antibody to that of the wild type antibody was increased by about 10 times in two points in which the wild type antibody and the mutant type antibody have the same cytotoxicity rate).
A person skilled in the art can calculate the increase of the ADCC activity according to the above-mentioned method from Fig. 8.

<305Cys mutant antibody >

Furthermore, the cytotoxicity rates of the anti-CD20 wild type antibody and 305Cys type chimeric antibody were compared with each other by a lactate dehydrogenase release assay. As a result, the cytotoxicity rate when the concentration of the added antibodies was 0.0000001, 0.000001, 0.00001, 0.0001, 0.001, 0.01, 0.1, 1 and 10 μg/mL was 8.1, 4.7, 4.3, 3.4, 5.6, 14.1, 40.5, 52.3 and 63.3% respectively in the wild type antibody and 8.1, 5.2, 4.2, 5.5, 15.2, 38.7, 53.8, 55.8 and 52.7% respectively in the 305Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 1.0, 1.1, 1.0, 1.6, 2.7, 2.7, 1.3, 1.1 and 0.8 times, respectively (see Table 14).

[Table 14]

| | concentration of anti-CD20 chimeric antibody(μg/ml) | | | | | | | | |
|---|---|---|---|---|---|---|---|---|---|
| **cytotoxicity(%)** | 0.0000001 | 0.000001 | 0.00001 | 0.0001 | 0.001 | 0.01 | 0.1 | 1 | 10 |
| wild | 8.1 | 4.7 | 4.3 | 3.4 | 5.6 | 14.1 | 40.5 | 52.3 | 63.3 |
| 305 | 8.1 | 5.2 | 4.2 | 5.5 | 15.2 | 38.7 | 53.8 | 55.8 | 52.7 |
| 306 | 4.3 | 8.5 | 6.0 | 6.6 | 15.2 | 42.1 | 53.2 | 53.4 | 49.3 |
| 307 | 3.1 | 4.0 | 3.0 | 3.5 | 13.1 | 42.1 | 57.8 | 74.0 | 86.9 |
| 308 | 10.6 | 3.9 | 7.8 | 4.5 | 12.6 | 35.5 | 55.8 | 60.3 | 72.5 |
| **305Cys / wild type (times)** | 1.0 | 1.0 | 1.0 | 1.0 | 1.0 | 1.0 | 1.0 | 1.0 | 1.0 |
| **306Cys / wild type (times)** | 0.5 | 1.6 | 1.4 | 1.2 | 1.0 | 1.1 | 1.0 | 1.0 | 0.9 |
| **307Cys / wild type (times)** | 0.4 | 0.8 | 0.7 | 0.6 | 0.9 | 1.1 | 1.1 | 1.3 | 1.6 |
| **308Cys / wild type (times)** | 1.3 | 0.8 | 1.9 | 0.8 | 0.8 | 0.9 | 1.0 | 1.1 | 1.4 |

Furthermore, from Table 14, two points in which the wild type antibody and the 305Cys type chimeric antibody have the same cytotoxicity rate were selected and comparison of the ADCC activity was carried out. For example, the 305Cys type chimeric antibody shows about 15.2% cytotoxicity at 0.01 μg/mL and the wild type antibody shows about 14.1% cytotoxicity at 0.1 μg/mL. When the comparison was carried out by using these two points, antibody concentration necessary to obtaining the same ADCC activity was reduced to about 1/100. It can be determined that the ADCC activity was increased about 100 times (the ratio of concentration of the mutant type antibody to that of the wild type antibody was increased by about 100 times in two points in which the wild type antibody and the mutant type antibody have the same cytotoxicity rate).

A person skilled in the art can calculate the increase of the ADCC activity according to the above-mentioned method from Fig. 9.

<306Cys mutant antibody >

Furthermore, the cytotoxicity rates of the anti-CD20 wild type antibody and 306Cys type chimeric antibody were compared with each other by a lactate dehydrogenase release assay. As a result, the cytotoxicity rate when the concentration of the added antibodies was 0.0000001, 0.000001, 0.00001, 0.0001, 0.001, 0.01, 0.1, 1 and 10 μg/mL was 8.1, 4.7, 4.3, 3.4, 5.6, 14.1, 40.5, 52.3 and 63.3% respectively in the wild type antibody and 4.3, 8.5, 6.0, 6.6, 15.2, 42.1, 53.2, 53.4 and 49.3% respectively in the 306Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 0.5, 1.8, 1.4, 1.9, 2.7, 3.0, 1.3, 1.0 and 0.8 times, respectively (see Table 14).

Furthermore, from Table 14, two points in which the wild type antibody and the 306Cys type chimeric antibody have the same cytotoxicity rate were selected and comparison of the ADCC activity was carried out. For example, the 306Cys type chimeric antibody shows about 15.2% cytotoxicity at 0.01 μg/mL and the wild type antibody shows about 14.1% cytotoxicity at 0.1 μg/mL. When the comparison was carried out by using these two points, antibody concentration necessary to obtaining the same ADCC activity was reduced to about 1/100. It can be determined that the ADCC activity was increased about 100 times (the ratio of concentration of the mutant type antibody to that of the wild type antibody was increased by about 100 times in two points in which the wild type antibody and the mutant type antibody have the same cytotoxicity rate).

A person skilled in the art can calculate the increase of the ADCC activity according to the above-mentioned method from Fig. 9.

< 307Cys mutant antibody >

Furthermore, the cytotoxicity rates of the anti-CD20 wild type antibody and 307Cys type chimeric antibody were compared with each other by a lactate dehydrogenase release assay. As a result, the cytotoxicity rate when the concentration of the added antibodies was 0.0000001, 0.000001, 0.00001, 0.0001, 0.001, 0.01, 0.1, 1 and 10 μg/mL was 8.1, 4.7, 4.3, 3.4, 5.6, 14.1, 40.5, 52.3, 63.3% respectively in the wild type antibody and 3.1, 4.0, 3.0, 3.5, 13.1, 42.1, 57.8, 74.0 and 86.9% respectively in the 307Cys type chimeric antibody. Therefore, the ratio of the ADCC activity (the the ratio of the cytotoxicity of the mutant type antibody to that of the wild type antibody in two points in which the wild type antibody and the mutant type antibody have the same concentration) were shown to be 0.4, 0.8, 0.7, 1.0, 2.3, 3.0, 1.4, 1.4 and 1.4 times, respectively (see Table 14).

Furthermore, from Table 14, two points in which the wild type antibody and the 307Cys type chimeric antibody have the same cytotoxicity rate were selected and comparison of the ADCC activity was carried out. For example, the 307Cys type chimeric antibody shows about 57.8% cytotoxicity at 0.1 μg/mL and the wild type antibody shows about 63.3% cytotoxicity at 10 μg/mL. When the comparison was carried out by using these two points, antibody concentration necessary to obtaining the same ADCC activity was reduced to about 1/100. It can be determined that the ADCC activity was increased about 100 times (the ratio of concentration of the mutant type antibody to that of the wild type antibody was increased by about 100 times in two points in which the wild type antibody and the mutant type antibody have the same cytotoxicity rate).
A person skilled in the art can calculate the increase of the ADCC activity according to the above-mentioned method from Fig. 9.

<308Cys mutant antibody >

Furthermore, the cytotoxicity rates of the anti-CD20 wild type antibody and 308Cys type chimeric antibody were compared with each other by a lactate dehydrogenase release assay. As a result, the cytotoxicity rate when the concentration of the added antibodies was 0.0000001, 0.000001, 0.00001, 0.0001, 0.001, 0.01, 0.1, 1 and 10 μg/mL was 8.1, 4.7, 4.3, 3.4, 5.6, 14.1, 40.5, 52.3, 63.3% respectively in the wild type antibody and 10.6, 3.9, 7.8, 4.5, 12.6, 35.5, 55.8, 60.3, 72.5% respectively in the 308Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 1.3, 0.8, 1.8, 1.3, 2.3, 2.5, 1.4, 1.2, and 1.1 times, respectively (see Table 14).

Furthermore, from Table 14, two points in which the wild type antibody and the 308Cys type chimeric antibody have the same cytotoxicity rate were selected and comparison of the ADCC activity was carried out. For example, the 308Cys type chimeric antibody shows about 55.8% cytotoxicity at 0.1 μg/mL and the wild type antibody shows 52.3% cytotoxicity at 1 μg/mL. When the comparison was carried out by using these two points, antibody concentration necessary to obtaining the same ADCC activity was reduced to about 1/10. It can be determined that the ADCC activity was increased about 10 times (the ratio of concentration of the mutant type antibody to that of the wild type antibody was increased by about 10 times in two points in which the wild type antibody and the mutant type antibody have the same cytotoxicity rate).
A person skilled in the art can calculate the increase of the ADCC activity according to the above-mentioned method from Fig. 9.

[Example 5]

By the same method as in Example 2, 309Cys type anti-CD20 chimeric antibody was produced and the ADCC activities of these Cys type anti-CD20 chimeric antibodies were evaluated. As a result, as compared with the ADCC activity of the wild type anti-CD20 chimeric antibody, ADCC activities of 309Cys type anti-CD20 chimeric antibodies were extremely higher.
Hereinafter, a production method of wild type and one mutant type (309Cys type) anti-CD20 chimeric antibodies, ADCC activity measurement thereof and the reactivity with respect to a CD20 molecule are described.

1) Production of anti-CD20 chimeric antibody

A chimeric antibody is obtained as a purified chimeric antibody through the following steps A) to F).

A) cloning a gene necessary for production of a chimeric antibody,
B) introducing a mutation of the cloned gene,
C) constructing a chimeric antibody expression vector combining the cloned gene and the mutation-introduced gene,

D) carrying a gene transfer of a chimeric antibody expression vector into a CHO cell and screening a CHO cell highly expressing a chimeric antibody,
E) culturing a CHO cell highly expressing a chimeric antibody, and
F) carrying out column purification from a culture supernatant of a cell highly expressing a chimeric antibody.

In these steps, A) and C) to F) are the same as those in Example 1. Therefore, in this Example, step B) is described.

B) Introducing a mutation of Cloned Gene

In order to obtain 309Cys chimeric antibody, the mutation needs to be introduced into a certain position of the cloned human IgG1 H chain constant region. According to the number of human IgG1 H chain constant region in Sequence of proteins of Immunological Interest (NIH Publication No. 91-3242, 1991) shown by Elvin A.Kabat, in order to change Leu at a position 309 into Cys, mutation was introduced by PCR method by using a pCR2.1 vector into which a cloned human IgG1 H chain constant region had been inserted : pCR2.1-HC (wild type) as a template. The detail of this mutation introduction was described as follows.

Mutation Introduction into 309 (Leu → Cys)

| | |
|---|---|
| pCR2.1-HC (wild) (25 ng/μL) | 2 μL |
| 2.5 mM dNTPs | 4 μL |
| 309 Cys1 primer (20 μM) | 1.25 μL |
| 309 Cys2 primer (20 μM) | 1.25 μL |
| × 10 pfu polymerase Buffer | 5 μL |
| pfu DNA polymerase | 1 μL |
| sterile water | 35.5 μL |
| total | 50 μL |

(PCR amplification reaction)

95°C, 30 sec
95°C, 30 sec; 55°C, 1 min; 68°C, 13 min (12 cycles)
68°C, 4 min
4°C, unlimited time

Base sequences of primers follows:

309Cys1 primer: gtcagcgtcctcaccgtctgtcaccaggactggctgaat
309Cys2 primer: attcagccagtcctggtgacagacggtgaggacgctgac

After the above-mentioned PCR reaction, 1 μL of DpnI (New England BioLabs) was added and incubated at 37°C for two hours. By using a liquid after the incubation was completed, Escherichia coli JM109 was transformed. A plasmid was purified from the transformed Escherichia coli. By confirming a DNA sequence of the purified plasmid, a pCR2.1 vector into which a human IgG1 H chain constant region had been inserted was obtained. In the human IgG1 H chain constant region, mutant 309 (Leu → Cys) had been introduced.

< Flow Cytometric Analysis of Various Kinds of Purified Chimeric Antibodies >

Flow Cytometric Analysis of various kinds of purified chimeric antibodies was carried out according to the same method as that described in Example 1.

< Evaluation of Activity of Various Kinds of Chimeric Antibody >

< 309Cys mutant antibody >

Furthermore, the cytotoxicity rates of the anti-CD20 wild type antibody and 309Cys type chimeric antibody were compared with each other by a lactate dehydrogenase release assay. As a result, the cytotoxicity rate when the concentration of the added antibodies was 0.0001, 0.001, 0.01, 0.1, 1 μg/mL was 1.8, 2.4, 2.2, 7.4, 28.4, 35.7, 37.2 and 44.3% respectively in the wild type antibody and 3.4, 7.7, 11.8, 28.5, 39.7, 45.7, 39.8 and 41.5% respectively in the 309Cys type chimeric antibody. Therefore, the ratio of the ADCC activity (the ratio of the cytotoxicity of the mutant type antibody to that of the wild type antibody in two points in which the wild type antibody and the mutant type antibody have the same concentration) were shown to be 1.9, 3.2, 5.3, 3.9, 1.4, 1.3, 1.1 and 0.9 times, respectively (see Table 15).
[Table 15]

| | **concentration of anti-CD20 chimeric antibody(μg/ml)** | | | | | | | |
|---|---|---|---|---|---|---|---|---|
| **cytotoxicity(%)** | 0.000001 | 0.00001 | 0.0001 | 0.001 | 0.01 | 0.1 | 1 | 10 |
| **Wild type** | 1.8 | 2.4 | 2.2 | 7.4 | 28.4 | 35.7 | 37.2 | 44.3 |
| **309Cys type** | 3.4 | 7.7 | 11.8 | 28.5 | 39.7 | 45.7 | 39.8 | 41.5 |
| **309Cys/Wild type (times)** | 1.9 | 3.2 | 5.3 | 3.9 | 1.4 | 1.3 | 1.1 | 0.9 |

Furthermore, from Table 15, two points in which the wild type antibody and the 309Cys type chimeric antibody have the same cytotoxicity rate were selected and comparison of the ADCC activity was carried out. For example, the 309Cys type chimeric antibody shows about 39.7% cytotoxicity at 0.01 μg/mL and the wild type antibody shows about 37.2% cytotoxicity at 1 μg/mL. When the comparison was carried out by using these two points, the antibody concentration necessary to obtaining the same ADCC activity was reduced to about 1/100. It can be determined that the ADCC activity was increased about 100 times (the ratio of concentration of the mutant type antibody to that of the wild type antibody was increased by 100 times in two points in which the wild type antibody and the mutant type antibody have the same cytotoxicity rate).
A person skilled in the art can calculate the increase of the ADCC activity according to the above-mentioned method from Fig. 10.

## Claims

1. An antibody comprising an H chain constant region in which an amino acid residue at at least one position selected from the group consisting of positions 286, 287, 288, 289, 290, 291, 292, 294, 298, 301, 302, 303, 305, 306, 307, 308 and 309 is substituted with cysteine.

2. The antibody according to claim 1, wherein the H chain constant region is any one constant region selected from the group consisting of human Cγ1, Cγ2, Cγ3, and Cγ4.

**3.** The antibody according to claim 1 or 2, wherein an ADCC activity is increased as compared with an ADCC activity before substitution.

**4.** The antibody according to any one of claims 1 to 3, wherein the antibody has an improved stability, solubility or binding affinity to an Fc receptor.

**5.** The antibody according to any one of claims 1 to 4, wherein a sugar chain is provided.

**6.** The antibody according to claim 5, wherein an effector function is improved.

**7.** The antibody according to any one of claims 1 to 6, wherein the antibody binds to FcγRI, FcγRII, FcγRIII, or FcRn.

**8.** The antibody according to any one of claims 1 to 7, wherein the antibody has specificity with respect to a target antigen selected from the group consisting of CD20, CD22, CD33, CD52, Her2/neu, EGFR, EpCAM, MUC1, GD3, CEA, CA125, HLA-DR, TNF alpha and VEGF.

**9.** An antibody, which is a human antibody having a specificity to CD20, wherein an amino acid residue at at least one position selected from the group consisting of positions 286, 287, 288, 289, 290, 291, 292, 294, 298, 301, 302, 303, 305, 306, 307, 308, and 309 is substituted with cysteine.

**10.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 95 as CH;
(2) an antibody comprising an H chain having an amino acid sequence set forth in SEQ ID NO: 93;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 97 as an Fc region; and
(4) an antibody comprising an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**11.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 101 as CH;
(2) an antibody comprising an H chain having an amino acid sequence set forth in SEQ ID NO: 99;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 103 as an Fc region; and
(4) an antibody comprising an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**12.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 107 as CH;
(2) an antibody comprising an H chain having an amino acid sequence set forth in SEQ ID NO: 105;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 109 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**13.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 54 as CH;
(2) an antibody comprising an H chain having an amino acid sequence set forth in SEQ ID NO: 45;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 73 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**14.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 83 as CH;
(2) an antibody comprising an H chain having an amino acid sequence set forth in SEQ ID NO: 81;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**15.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 89 as CH;
(2) an antibody comprising an H chain having an amino acid sequence set forth in SEQ ID NO: 87;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 91 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**16.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 131 as CH;
(2) an antibody comprising an H chain having an amino acid sequence set forth in SEQ ID NO: 129;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 133 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**17.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 137 as CH;
(2) an antibody comprising an H chain having an amino acid sequence set forth in SEQ ID NO: 135;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 139 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**18.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 143 as CH;
(2) an antibody comprising an H chain having an amino acid sequence set forth in SEQ ID NO: 141;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 145 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**19.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 149 as CH;
(2) an antibody comprising an H chain having an amino acid sequence set forth in SEQ ID NO: 147;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 151 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**20.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 155 as CH;
(2) an antibody comprising an H chain having an amino acid sequence set forth in SEQ ID NO: 153;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 157 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**21.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 161 as CH;
(2) an antibody comprising an H chain having an amino acid sequence set forth in SEQ ID NO: 159;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 163 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**22.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 167 as CH;
(2) an antibody comprising an H chain having an amino acid sequence set forth in SEQ ID NO: 165;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 169 as an Fc region; and

(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**23.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 173 as CH;
(2) an antibody comprising an H chain having an amino acid sequence set forth in SEQ ID NO: 171;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 175 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**24.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 179 as CH;
(2) an antibody comprising an H chain having an amino acid sequence set forth in SEQ ID NO: 177;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 181 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**25.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 185 as CH;
(2) an antibody comprising an H chain having an amino acid sequence set forth in SEQ ID NO: 183;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 187 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**26.** An antibody described in any one of the following (1) to (4);

(1) an antibody including an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 191 as CH;
(2) an antibody comprising an H chain having an amino acid sequence set forth in SEQ ID NO: 189;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 193 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**27.** An antibody described in any one of the following (1) to (4);

(1) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 241 as CH;
(2) an antibody comprising an H chain having an amino acid sequence set forth in SEQ ID NO: 243;
(3) an antibody comprising an H chain having an amino acid sequence set forth in SEQ ID NO: 47 as CDR1, an amino acid sequence set forth in SEQ ID NO: 49 as CDR2, an amino acid sequence set forth in SEQ ID NO: 51 as CDR3, and an amino acid sequence set forth in SEQ ID NO: 245 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 58 as CDR1, an amino acid sequence set forth in SEQ ID NO: 60 as CDR2, an amino acid sequence set forth in SEQ ID NO: 62 as CDR3, and an amino acid sequence set forth in SEQ ID NO: 64 as CL; and
(ii) an L chain having an amino acid sequence set forth in SEQ ID NO: 56.

**28.** An antibody described in any one of the following (1) to (4);

(1) an antibody comprising an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 83 as CH;
(2) an antibody comprising an H chain having an amino acid sequence set forth in SEQ ID NO: 209;
(3) an antibody comprising an H chain having an amino acid sequence set forth in SEQ ID NO: 195 as CDR1, an amino acid sequence set forth in SEQ ID NO: 197 as CDR2, an amino acid sequence set forth in SEQ ID NO: 199 as CDR3, and an amino acid sequence set forth in SEQ ID NO: 85 as an Fc region; and
(4) an antibody having an H chain described in the above-mentioned (1), (2) or (3), and a pair of L chains described in the following (i) or (ii);

(i) an L chain having an amino acid sequence set forth in SEQ ID NO: 203 as CDR1, an amino acid sequence set forth in SEQ ID NO: 205 as CDR2, an amino acid sequence set forth in SEQ ID NO: 207 as CDR3, and an amino acid sequence set forth in SEQ ID NO: 247 as CL; and
(ii) an L chain having an amino acid sequence set forth in SEQ ID NO: 201.

**29.** A method for producing an antibody having an enhanced ADCC activity, the method including substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region.

**30.** A method for producing an antibody having an enhanced ADCC activity, the method including the following steps (a) and (b);

(a) a step of expressing DNA encoding an H chain in which an amino acid residue at at least one position selected from the group consisting of positions 286, 287, 288, 289, 290, 291, 292, 294, 298, 301, 302, 303, 305, 306, 307, 308, and 309 is substituted with cysteine in an H chain constant region, and a DNA encoding an L chain; and
(b) a step of collecting an expression product in the step (a).

**31.** The method according to claim 29 or 30, wherein the H chain constant region is any one of constant regions selected from the group consisting of human Cγ1, Cγ2, Cγ3, and Cγ4.

**32.** A pharmaceutical composition comprising an antibody according to any of claims 1 to 28 and a pharmaceutically acceptable carrier.

**33.** A method for treating non-human mammalian, the method including administering an antibody according to any of claims 1 to 28 to a subject.

**34.** A nucleic acid encoding an antibody according to any of claims 1 to 28.

**35.** A vector including a nucleic acid according to claim 34.

**36.** A host cell having a vector according to claim 35.

**37.** A host organism having a vector according to claim 35.

**38.** A method for enhancing an ADCC activity, the method including substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region.

**39.** The method according to claim 38, wherein the H chain constant region is any one constant region selected from the group consisting of human Cγ1, Cγ2, Cγ3, and Cγ4.

**40.** A method for determining whether or not an ADCC activity of an antibody in a step (a) is enhanced, wherein it is determined that the ADCC activity of the antibody in a step (a) is enhanced when the ADCC activity measured in the step (a) is higher than an ADCC activity of the antibody before substitution;

(a) a step of providing a mutant of an antibody having an ADCC activity in which an amino acid residue at at least one position selected from the group consisting of positions 286, 287, 288, 289, 290, 291, 292, 294, 298, 301, 302, 303, 305, 306, 307, 308, and 309 is substituted with cysteine in an H chain constant region, and
(b) a step for measuring the ADCC activity of the step (a).

**41.** A method for screening an antibody having an enhanced ADCC activity, the method comprising the following steps (a) to (d);

(a) providing an antibody having an ADCC activity;
(b) substituting an amino acid residue at at least one position selected from the group consisting of positions 286, 287, 288, 289, 290, 291, 292, 294, 298, 301, 302, 303, 305, 306, 307, 308, and 309 with cysteine in an H chain constant region of the antibody of the step (a);
(c) determining whether or not an ADCC activity of the antibody obtained in the step (b) is enhanced by the method according to claim 40; and
(d) selecting an antibody that has been determined in the step (c) that the ADCC activity is enhanced.

FIG. 1

```
  1 ATGGATTTTCAGGTGCAGATTATCAGCTTCCTGCTAATCAGTGCTTCAGTCATAATGTCC  60
    METAspPheGlnValGlnIleIleSerPheLeuLeuIleSerAlaSerValIleMETSer

 61 AGAGGACAAATTGTTCTCTCCCAGTCTCCAGCAATCCTGTCTGCATCTCCAGGGGAGAAG  120
    ArgGlyGlnIleValLeuSerGlnSerProAlaIleLeuSerAlaSerProGlyGluLys
                                 CDR1
121 GTCACAATGACTTGC AGGGCCAGCTCAAGTGTAAGTTACATGCAC TGGTACCAGCAGAAG  180
    ValThrMETThrCys ArgAlaSerSerSerValSerTyrMETHis TrpTyrGlnGlnLys
                                                     CDR2
181 CCAGGATCCTCCCCCAAACCCTGGATTTAT GCCCCATCCAACCTGGCTTCT GGAGTCCCT  240
    ProGlySerSerProLysProTrpIleTyr AlaProSerAsnLeuAlaSer GlyValPro

241 GCTCGCTTCAGTGGCAGTGGGTCTGGGACCTCTTACTCTCTCACAATCAGCAGAGTGGAG  300
    AlaArgPheSerGlySerGlySerGlyThrSerTyrSerLeuThrIleSerArgValGlu
                                                         CDR3
301 GCTGAAGATGCTGCCACTTATTACTGC CAGCAGTGGAGTTTTAACCCACCCACG TTCGGT  360
    AlaGluAspAlaAlaThrTyrTyrCys GlnGlnTrpSerPheAsnProProThr PheGly

361 GCTGGGACCAAGCTGGAGCTGAAACGGGCTGATGCTGCACCAACTGTATCCATCTTCCCA  420
    AlaGlyThrLysLeuGluLeuLysArgAlaAspAlaAlaProThrValSerIlePhePro

421 CCATCCAGT  429                       mouse L chain constant region (κ)
    ProSerSer
```

FIG. 2

```
  1  ATGGGATGGAGCTGGGTCTTTCTCTTCCTCCTGTCAGTAACTACAGGTGTCCACTCCCAG   60
     METGlyTrpSerTrpValPheLeuPheLeuLeuSerValThrThrGlyValHisSerGln

 61  GCTTATCTACAGCAGTCTGGGGCTGAGCTGGTGAGGCCTGGGGCCTCAGTGAAGATGTCC  120
     AlaTyrLeuGlnGlnSerGlyAlaGluLeuValArgProGlyAlaSerValLysMETSer
                                      CDR1
121  TGCAAGGCTTCTGGCTACACATTTACC AGTTACAATATGCAC TGGGTAAAGCAGACACCT  180
     CysLysAlaSerGlyTyrThrPheThr SerTyrAsnMETHis TrpValLysGlnThrPro
                                                          CDR2
181  AGACAGGGCCTGGAATGGATTGGA GCTATTTATCCAGGAAATGGTGATACTTCCTACAAT  240
     ArgGlnGlyLeuGluTrpIleGly AlaIleTyrProGlyAsnGlyAspThrSerTyrAsn

241  CAGAAGTTCAAGGGC AAGGCCACACTGACTGTAGACAAATCCTCCAGCACAGCCTACATG  300
     GlnLysPheLysGly LysAlaThrLeuThrValAspLysSerSerSerThrAlaTyrMET

301  CAGCTCAGCAGCCTGACATCTGAAGACTCTGCGGTCTATTTCTGTGCAAGA GTGGTGTAC  360
     GlnLeuSerSerLeuThrSerGluAspSerAlaValTyrPheCysAlaArg ValValTyr
                  CDR3
361  TATAGTAACTCTTACTGGTACTTCGATGTC TGGGGCACAGGGACCACGGTCACCGTCTCC  420
     TyrSerAsnSerTyrTrpTyrPheAspVal TrpGlyThrGlyThrThrValThrValSer

421  TCAGCCAAAACAACACCCCCATCAGTCTATCCACTG  456
     SerAlaLysThrThrProProSerValTyrProLeu
```

mouse H chain constant region

FIG. 3

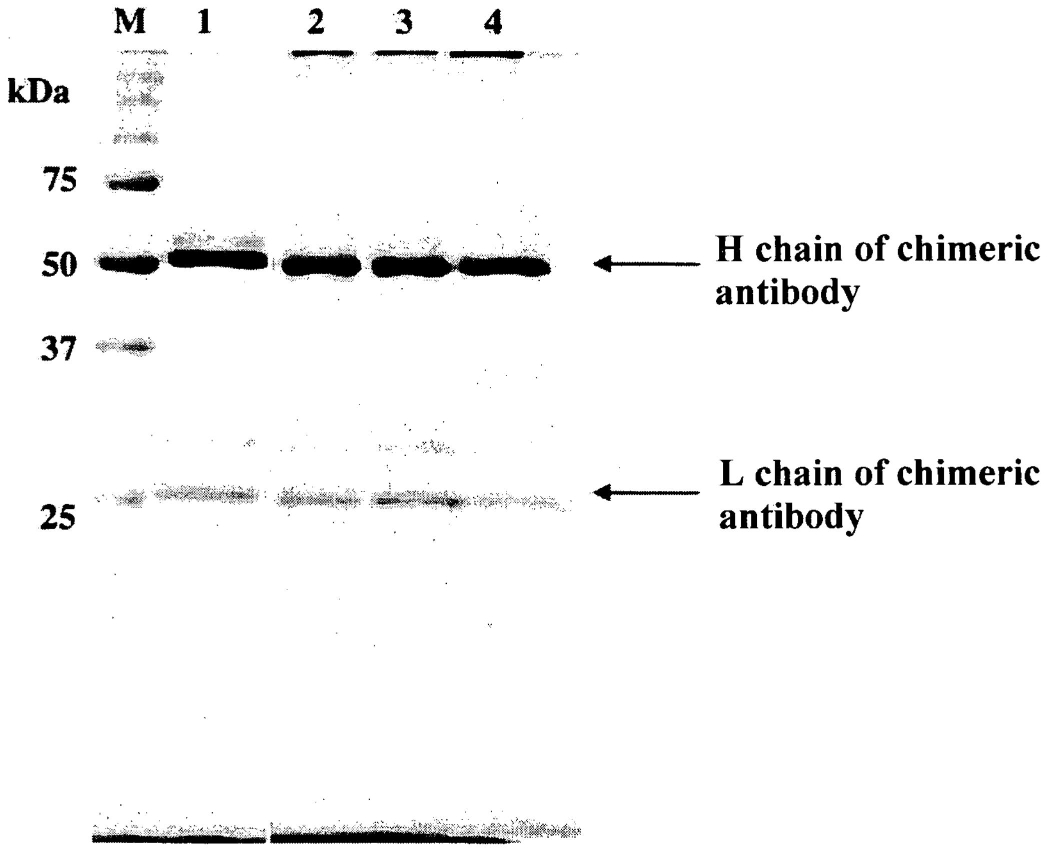


lane M : marker protein
lane 1 : purified wild type anti-CD20 chimeric antibody
lane 2 : purified 294Cys anti-CD20 chimeric antibody
lane 3 : purified 298Cys anti-CD20 chimeric antibody
lane 4 : purified 301Cys anti-CD20 chimeric antibody

FIG. 4

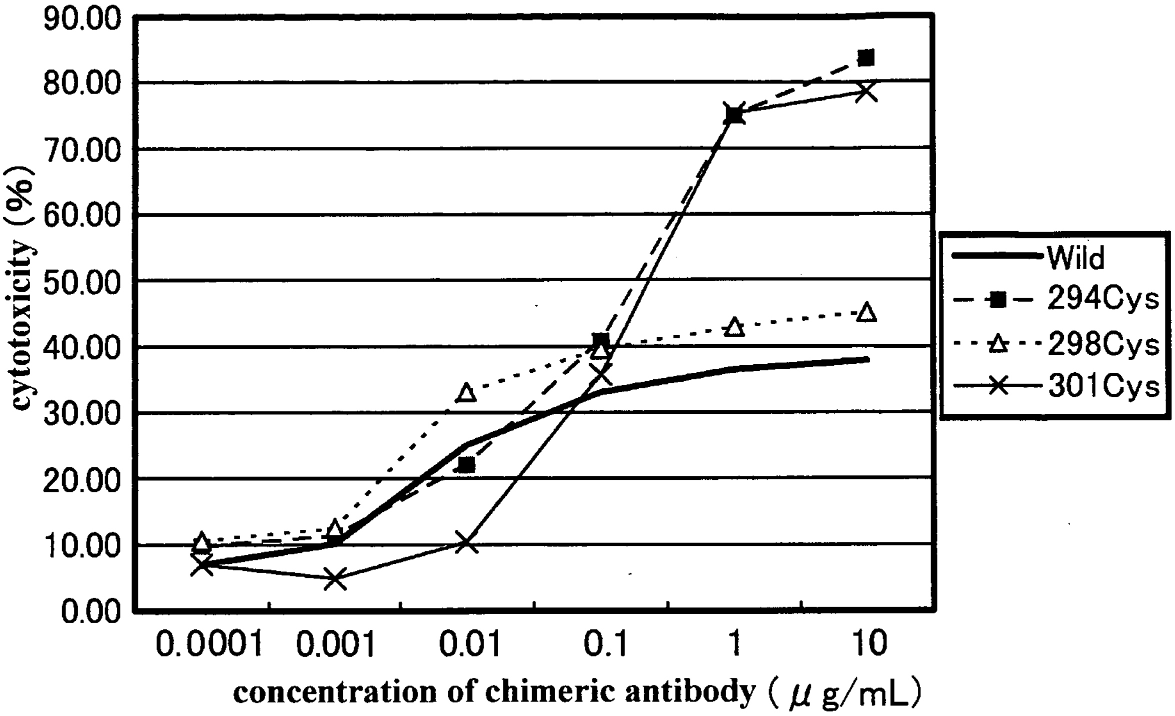
90.00
80.00
70.00
60.00
50.00
40.00
30.00
20.00
10.00
0.00
cytotoxicity (%)
Wild
294Cys
298Cys
301Cys
0.0001
0.001
0.01
0.1
1
10
concentration of chimeric antibody ( μ g/mL)

FIG. 5

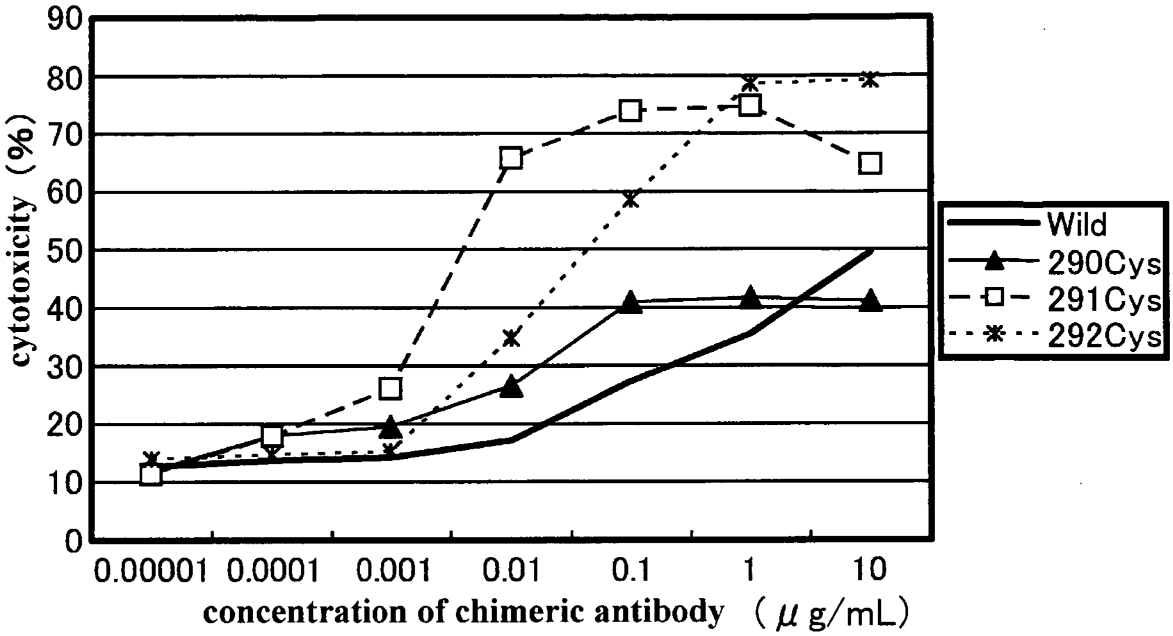
90
80
70
60
50
40
30
20
10
0
cytotoxicity (%)
0.00001 0.0001 0.001 0.01 0.1 1 10
concentration of chimeric antibody (μg/mL)
Wild
290Cys
291Cys
292Cys

FIG. 6

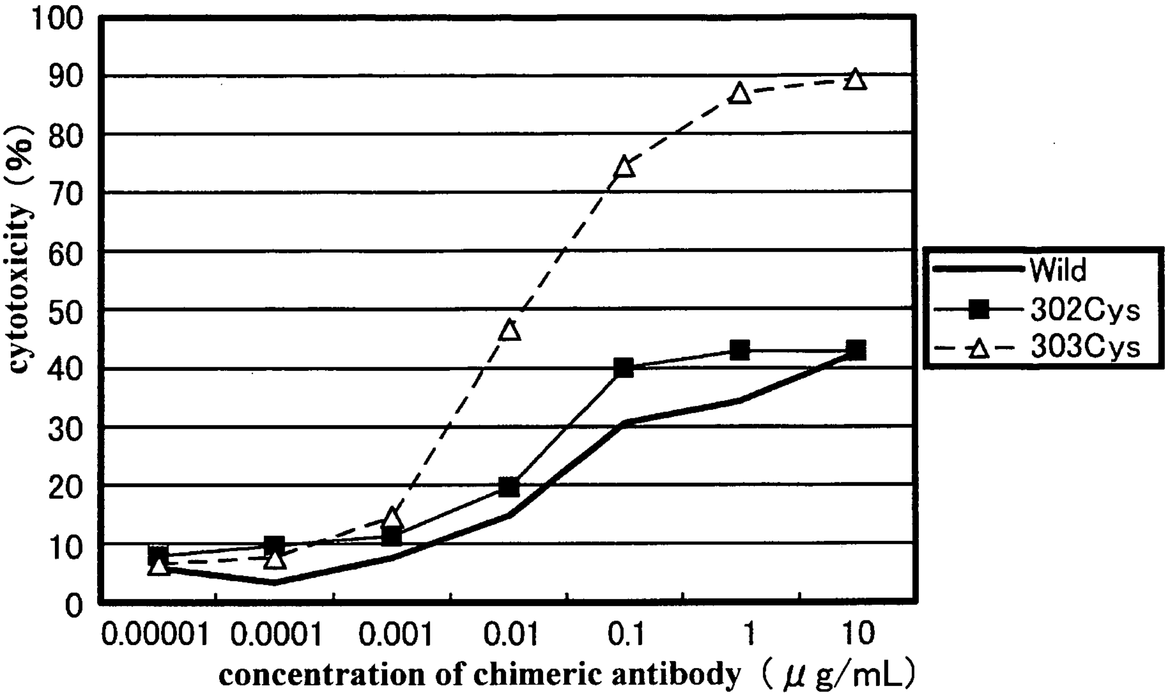
100
90
80
70
60
50
40
30
20
10
0
cytotoxicity (%)
0.00001
0.0001
0.001
0.01
0.1
1
10
concentration of chimeric antibody (μg/mL)
Wild
302Cys
303Cys

FIG. 7

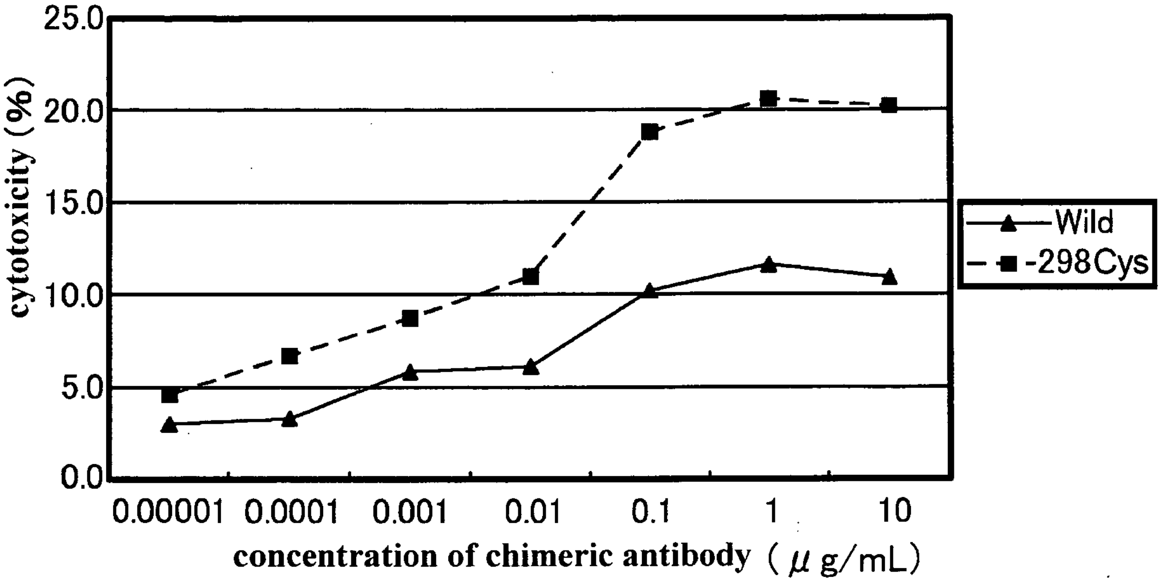
25.0
20.0
15.0
10.0
5.0
0.0
cytotoxicity (%)
0.00001
0.0001
0.001
0.01
0.1
1
10
concentration of chimeric antibody ( μ g/mL)
Wild
-298Cys

FIG. 8

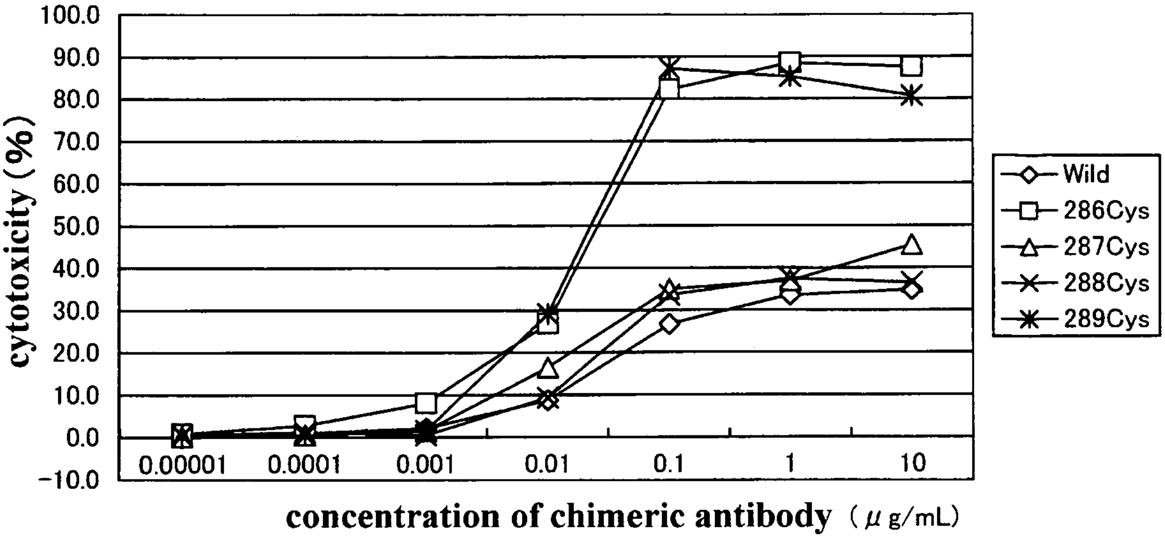
100.0
90.0
80.0
70.0
60.0
50.0
40.0
30.0
20.0
10.0
0.0
-10.0
cytotoxicity (%)
0.00001
0.0001
0.001
0.01
0.1
1
10
concentration of chimeric antibody (μg/mL)
Wild
286Cys
287Cys
288Cys
289Cys

FIG. 9

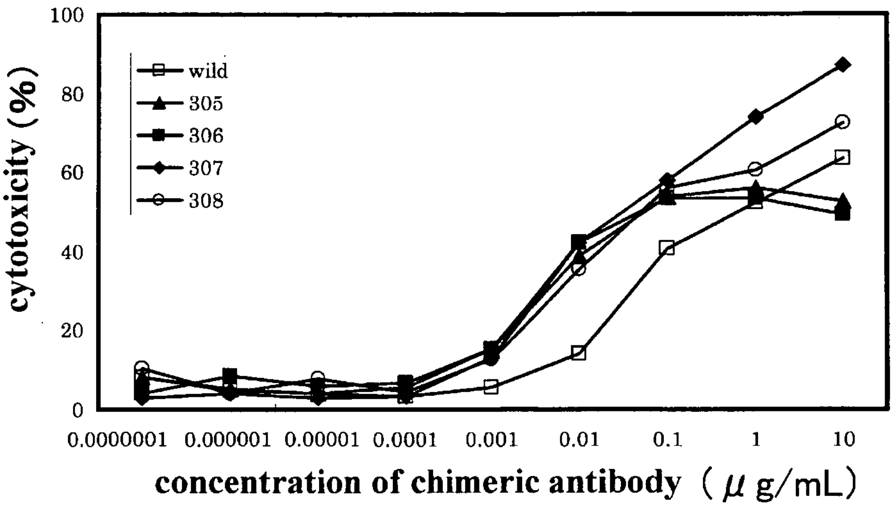
100
80
60
40
20
0
cytotoxicity (%)
wild
305
306
307
308
0.0000001
0.000001
0.00001
0.0001
0.001
0.01
0.1
1
10
concentration of chimeric antibody (μg/mL)

FIG. 10

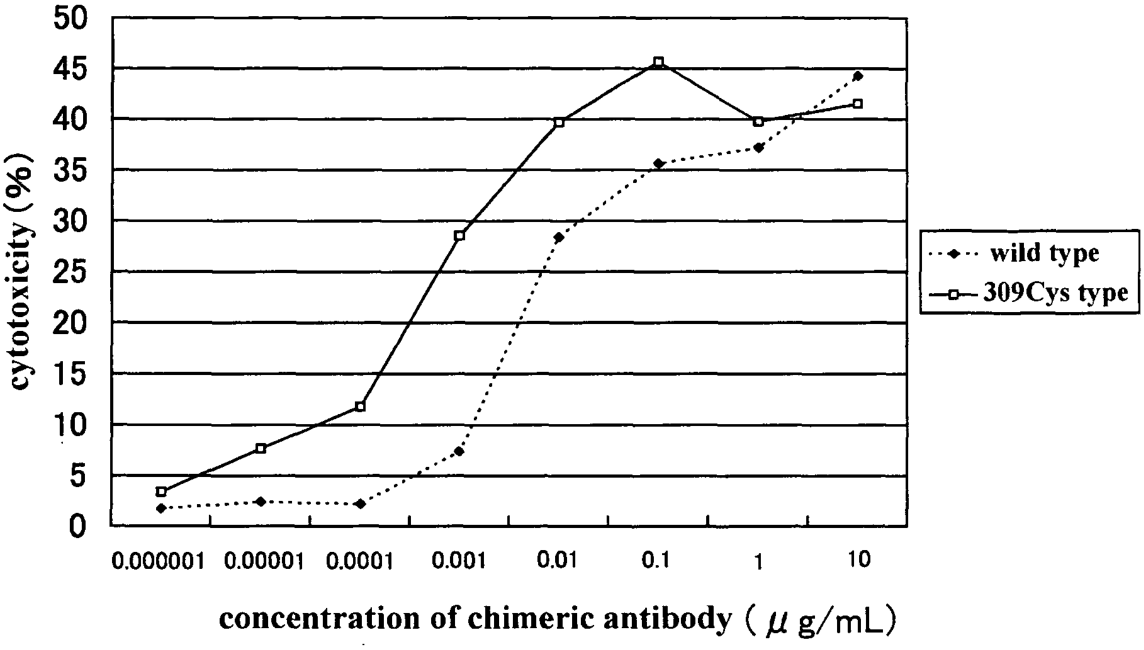
cytotoxicity (%)
50
45
40
35
30
25
20
15
10
5
0
0.000001
0.00001
0.0001
0.001
0.01
0.1
1
10
concentration of chimeric antibody ( μ g/mL)
wild type
309Cys type

**INTERNATIONAL SEARCH REPORT**

International application No.
PCT/JP2007/067979

A. CLASSIFICATION OF SUBJECT MATTER
*C07K16/28*(2006.01)i, *A61K39/395*(2006.01)i, *C12N15/02*(2006.01)i, *C12N15/09*(2006.01)i, *C12P21/08*(2006.01)i

According to International Patent Classification (IPC) or to both national classification and IPC

B. FIELDS SEARCHED

Minimum documentation searched (classification system followed by classification symbols)
C07K16/28, A61K39/395, C12N15/02, C12N15/09, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| E,X | WO 2007/119796 A1 (Medical & Biological Laboratories Co., Ltd.), 25 October, 2007 (25.10.07), (Family: none) | 1-41 |
| Y | WO 2006/019447 A1 (XENCOR, INC.), 23 February, 2006 (23.02.06), Figs. 24b, 28 & AU 2005/272993 A1 & IN 2006/01475 P3 & KR 2007/029190 A | 1-41 |
| Y | WO 2004/063351 A2 (MACROGENICS, INC.), 29 July, 2004 (29.07.04), Table 12 & US 2005/037000 A1 & US 2005/064514 A1 & EP 1587540 A2 & JP 2006-524039 A & AU 2004/204494 A1 & IN 2005/01849 P4 | 1-41 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 December, 2007 (06.12.07) | 18 December, 2007 (18.12.07) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/067979

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2004/074455 A2 (APPLIED MOLECULAR EVOLUTION), 02 September, 2004 (02.09.04), Figs. 10, 11<br>& US 2004/002587 A1 & EP 1606314 A2<br>& US 2007/141052 A1 | 1-41 |
| Y | WO 2005/070963 A1 (APPLIED MOLECULAR EVOLUTION, INC.), 04 August, 2005 (04.08.05), Table 2<br>& EP 1706424 A1 & NO 2006/03624 A<br>& AU 2005/206473 A1 & CN 1918178 A | 1-41 |
| Y | US 2006/0134709 A1 (Jeffery Stavenhagen), 22 June, 2006 (22.06.06), Table 15<br>& WO 2007/024249 A2 & EP 1810035 A2 | 1-41 |
| Y | JP 2006-512087 A (Protein Design Labs Inc.), 13 April, 2006 (13.04.06), Claims 13, 15<br>& WO 2004/035752 A2 & US 2005/014934 A1<br>& US 2005/276799 A1 & EP 1562972 A2<br>& AU 2003/286467 A1 | 1-41 |
| Y | JP 10-508482 A (The Welcome Foundation Ltd.), 25 August, 1998 (25.08.98), Claims 3 to 5<br>& WO 96/14339 A1 & EP 789713 A1<br>& AU 95/38114 A & ZA 95/09336 A | 1-41 |
| Y | P.C.CARON et al., Engineererd Humanized Dimeric Forms of IgG Are More Effective Antibodies, J.Exp.Med., 1992, Vol.176, p.1191-1195 | 1-41 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004029207 A
- WO 2000042072 A
- WO 2004063351 A
- WO 2004099249 A
- WO 2006019447 A
- JP 2006512407 PCT
- JP 2003512019 PCT
- WO 2006104989 A
- WO 2006105062 A
- US 5585089 A
- US 5693761 A
- US 5693762 A
- US 6180370 B
- EP 451216 A
- EP 682040 A
- EP 2828340 A
- JP H159878 B
- WO 9312227 A
- WO 9203918 A
- WO 9402602 A
- WO 9425585 A
- WO 9634096 A
- WO 9633735 A
- US 5223409 A
- US 5403484 A
- US 5571689 A
- US 5663143 A
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 9201047 A
- WO 9220791 A
- WO 9306213 A
- WO 9311236 A
- WO 9319172 A
- WO 9501438 A
- WO 9515388 A
- US 5565332 A
- US 5573905 A
- WO 2003085119 A


### Non-patent literature cited in the description


- *Nature Genetics,* 1997, vol. 16, 113-114
- *J. Biol. Chem.,* 2003, vol. 278, 3466-3473
- **Shields R L et al.** *J. Biol. Chem.,* 2002, vol. 277, 26733-26740
- *Nature Biotechnology,* 1999, vol. 17, 176-180
- *J. Biol. Chem.,* 2001, vol. 276, 6591-6604
- *J. Biol. Chem.,* 2002, vol. 277, 26733-26740
- Sequence of Proteins of Immunological Interest. National Institutes of Health, 1991
- **Harlow ; Lane.** Antibodies: A Laboratory Manual. Cold Spring Harbor Laboratory Press, 1988
- **P. T. Johons et al.** *Nature,* 1986, vol. 321, 522
- **A. Wright ; S. L. Morrison.** *J. Immunol.,* 1998, vol. 160, 3393-3402
- **K. Motmans et al.** *Eur. J. Cancer Prev.,* 1996, vol. 5, 512-519
- **R. P. Junghans et al.** *Cancer Res.,* 1990, vol. 50, 1495-1502
- **R. W. Malone et al.** *Proc. Natl. Acad. Sci. USA,* 1989, vol. 86, 6077
- **P. L. Felgner et al.** *Proc. Natl. Acad. Sci. USA,* 1987, vol. 84, 7413
- **F. L. Graham ; A. J. van der Eb.** *Virology,* 1973, vol. 52, 456-467
- **Riechmann L. et al.** *Nature,* 1988, vol. 332, 323-3Z7

- **Tempst, PR. et al.** *Protein Engineering,* 1994, vol. 7, 1501-1507
- **Ellis JH. et al.** *J. Immunol,* 1995, vol. 155, 925-937
- **Queen C. et al.** *Proc Natl Acad SciUSA,* 1989, vol. 86, 10029-10033
- **Rozak MJ. et al.** *J Biol Chem,* 1996, vol. 271, 22611-22618
- **Shearman CW. et al.** *J.Immunol,* 1991, vol. 147, 4366-4373
- **Sato K. et al.** *Mol Immunol,* 1994, vol. 31, 371-381
- **Kobinger F. et al.** *Protein Engineering,* 1993, vol. 6, 971-980
- **Kettleborough CA. et al.** *Protein Engineering,* 1991, vol. 4, 773-783
- **McCafferty et al.** *Nature,* 1990, vol. 348, 552-554
- **Clackson et al.** *Nature,* 1991, vol. 352, 624-628
- **Marks et al.** *J. Mol. Biol.,* 1991, vol. 222, 581-597
- **Marks et al.** *Bio/Technology,* 1992, vol. 10, 779-783
- **Waterhouse et al.** *Nuc. Acids. Res.,* 1993, vol. 21, 2265-2266
- **Scott, J. K. ; Smith G. P.** *Science,* 1990, vol. 249, 386
- **Cwirla, S.E. et al.** *Proc. Natl. Acad. Sci. USA,* 1990, vol. 87, 6378
- **Lowman, H. B. et al.** *Biochemistry,* 1991, vol. 30, 10832
- **Clackson, T. et al.** *Nature,* 1991, vol. 352, 624
- **Marks, J. D. et al.** *J. Mol. Biol.,* 1991, vol. 222, 581
- **Kang, A. S. et al.** *Proc. Natl. Acad. Sci. USA,* 1991, vol. 88, 8363
- **Smith, G. P.** *Current Opin. Biotechnol.,* 1991, vol. 2, 668
- **Ren J. et al.** *Gene,* 1998, vol. 215, 439
- **Zhu et al.** *Cancer Researc,* 1998, vol. 58 (15), 3209-3214
- **Jiang et al.** *Infection & Immunity,* 1997, vol. 65 (11), 4770-4777
- **Ren et al.** *Gene,* 1997, vol. 195 (2), 303-311
- **Ren.** *Protein Sci.,* 1996, vol. 5, 1833
- **Efimov et al.** *VirusGenes,* 1995, vol. 10, 173
- **Smith ; Scott.** *Methods in Enzymology,* 1993, vol. 217, 228-257
- **Li et al.** *Mol Biotech.,* 1998, vol. 9, 187
- **McCafferty et al.** *Nature,* 1990, vol. 348, 552-553
- **Johnson, Kevin S. ; Chiswell, David J.** *Current Opinion in Structural Biology,* 1993, vol. 3, 564-571
- **Griffith.** *EMBO J.,* 1993, vol. 12, 725-734
- **Hopp, T. P. et al.** *BioTechnology,* 1988, vol. 6, 1204-1210
- **Ravetch ; Kinet.** *Annu. Rev. Immunol,* 1991, vol. 9, 457-92
- **Capel et al.** *Immunomethods,* 1994, vol. 4, 25-34
- **de Haas et al.** *J Lab. Clin. Med.,* 1995, vol. 126, 330-41
- **Bredius et al.** *Immunology,* 1994, vol. 83, 624-630
- **Tax et al.** *J. Immunol.,* 1984, vol. 133 (3), 1185-1189
- **Nagarajan et al.** *J. Biol. Chem.,* 1995, vol. 270 (43), 25762-25770
- **Warmerdam et al.** *J. Immunol.,* 1991, vol. 147 (4), 1338-1343
- **Ward et al.** *Nature,* 1989, vol. 341, 544-546
- *FASEB J.,* 1992, vol. 6, 2422-2427
- **Better et al.** *Science,* 1988, vol. 240, 1041-1043
- **Lei, S. P. et al.** *J. Bacteriol.,* 1987, vol. 169, 4379
- *Nucleic Acids. Res.,* 1990, vol. 18 (17), 5322
- **Mulligan et al.** *Nature,* 1979, vol. 277, 108
- **Mizushima et al.** *Nucleic Acids Res.,* 1990, vol. 18, 5322
- *J. Exp. Med.,* 1995, vol. 108, 945
- **Valle et al.** *Nature,* 1981, vol. 291, 358-340
- **Ebert, K.M. et al.** *Bio/Technology,* 1994, vol. 12, 699-702
- **Hashimoto-Gotoh, T ; Mizuno, T ; Ogasahara, Y ; Nakagawa, M.** An oligodeoxyribonucleotide-directed dual amber method for site-directed mutagenesis. *Gene,* 1995, vol. 152, 271-275
- **Zoller, MJ ; Smith, M.** Oligonucleotide-directed mutagenesis of DNA fragments cloned into M13 vectors. *Methods Enzymol.,* 1983, vol. 100, 468-500
- **Kramer, W ; Drutsa, V ; Jansen, HW ; Kramer, B ; Pflugfelder, M ; Fritz, HJ.** The gapped duplex DNA approach to oligonucleotide-directed mutation construction. *Nucleic Acids Res.,* 1984, vol. 12, 9441-9456
- **Kramer W ; Fritz HJ.** Oligonucleotide-directed construction of mutations via gapped duplex DNA Methods. *Enzymol.,* 1987, vol. 154, 350-367
- **Kunkel, TA.** Rapid and efficient site-specific mutagenesis without phenotypic selection. *Proc Natl Acad Sci U S A.,* 1985, vol. 82, 488-492

[Sequence listing]

SEQUENCE LISTING

<110> MEDICAL AND BIOLOGICAL LABORATORIES CO., LTD.

<120> Antibody with altered effector function

<130> P07066P

<160> 247

<170> PatentIn version 3.4

<210> 1
<211> 30
<212> DNA
<213> Artificial

<220>
<223> An artificially synthesized primer sequence

<400> 1
atgaagttgc ctgttaggct gttggtgctg 30

<210> 2
<211> 30
<212> DNA
<213> Artificial

<220>
<223> An artificially synthesized primer sequence

<400> 2
atggagwcag acacactcct gytatgggtg 30

<210> 3
<211> 30
<212> DNA
<213> Artificial

<220>
<223> An artificially synthesized primer sequence

<400> 3
atgagtgtgc tcactcaggt cctggsgttg 30

<210> 4
<211> 33
<212> DNA
<213> Artificial

<220>
<223> An artificially synthesized primer sequence

<400> 4
atgaggrccc ctgctcagwt tyttggmwtc ttg 33

<210> 5
<211> 30
<212> DNA
<213> Artificial

<220>
<223> An artificially synthesized primer sequence

<400> 5
atggatttwc aggtgcagat twtcagcttc 30

<210> 6
<211> 27
<212> DNA
<213> Artificial

<220>
<223> An artificially synthesized primer sequence

<400> 6
atgaggtkcy ytgytsagyt yctgrgg 27

<210> 7
<211> 31
<212> DNA
<213> Artificial

<220>
<223> An artificially synthesized primer sequence

<400> 7
atgggcwtca agatggagtc acakwyycwg g 31

<210> 8
<211> 31
<212> DNA
<213> Artificial

<220>
<223> An artificially synthesized primer sequence

<400> 8
atgtggggay ctktttycmm tttttcaatt g 31

<210> 9
<211> 25
<212> DNA
<213> Artificial

<220>
<223> An artificially synthesized primer sequence

<400> 9
atggtrtccw casctcagtt ccttg 25

<210> 10
<211> 27
<212> DNA
<213> Artificial

<220>
<223> An artificially synthesized primer sequence

<400> 10
atgtatatat gtttgttgtc tatttct 27

<210> 11
<211> 28
<212> DNA
<213> Artificial

<220>
<223> An artificially synthesized primer sequence

<400> 11
atggaagccc cagctcagct tctcttcc 28

<210> 12
<211> 20
<212> DNA
<213> Artificial

<220>
<223> An artificially synthesized primer sequence

<400> 12
actggatggt gggaagatgg 20

<210> 13
<211> 27
<212> DNA
<213> Artificial

<220>
<223> An artificially synthesized primer sequence

<400> 13
atgaaatgca gctggggcat sttcttc 27

<210> 14
<211> 26
<212> DNA
<213> Artificial

<220>
<223> An artificially synthesized primer sequence

<400> 14
atgggatgga gctrtatcat sytctt 26

<210> 15
<211> 27
<212> DNA
<213> Artificial

<220>
<223> An artificially synthesized primer sequence

<400> 15
atgaagwtgt ggttaaactg ggttttt 27

<210> 16
<211> 25
<212> DNA

<213> Artificial

<220>
<223> An artificially synthesized primer sequence

<400> 16
atgractttg ggytcagctt grttt 25

<210> 17
<211> 30
<212> DNA
<213> Artificial

<220>
<223> An artificially synthesized primer sequence

<400> 17
atggactcca ggctcaattt agttttcctt 30

<210> 18
<211> 27
<212> DNA
<213> Artificial

<220>
<223> An artificially synthesized primer sequence

<400> 18
atggctgtcy trgsgctrct cttctgc 27

<210> 19
<211> 26
<212> DNA
<213> Artificial

<220>
<223> An artificially synthesized primer sequence

<400> 19
atggratgga gckggrtctt tmtctt 26

<210> 20
<211> 23
<212> DNA
<213> Artificial

<220>
<223> An artificially synthesized primer sequence

<400> 20
atgagagtgc tgattctttt gtg 23

<210> 21
<211> 30
<212> DNA
<213> Artificial

<220>
<223> An artificially synthesized primer sequence

<400> 21
atggmttggg tgtggamctt gctattcctg 30

<210> 22
<211> 27
<212> DNA
<213> Artificial

<220>
<223> An artificially synthesized primer sequence

<400> 22
atgggcagac ttacattctc attcctg 27

<210> 23
<211> 28
<212> DNA
<213> Artificial

<220>
<223> An artificially synthesized primer sequence

<400> 23
atggattttg ggctgatttt ttttattg 28

<210> 24
<211> 27
<212> DNA
<213> Artificial

<220>
<223> An artificially synthesized primer sequence

<400> 24
atgatggtgt taagtcttct gtacctg 27

<210> 25
<211> 21
<212> DNA
<213> Artificial

<220>
<223> An artificially synthesized primer sequence

<400> 25
cagtggatag actgatgggg g 21

<210> 26
<211> 24
<212> DNA
<213> Artificial

<220>
<223> An artificially synthesized primer sequence

<400> 26
actgtggctg caccatctgt cttc 24

<210> 27

<211> 27
<212> DNA
<213> Artificial

<220>
<223> An artificially synthesized primer sequence

<400> 27
ttaacactct cccctgttga agctctt 27

<210> 28
<211> 24
<212> DNA
<213> Artificial

<220>
<223> An artificially synthesized primer sequence

<400> 28
gcctccacca agggcccatc ggtc 24

<210> 29
<211> 27
<212> DNA
<213> Artificial

<220>
<223> An artificially synthesized primer sequence

<400> 29
ttatttaccc ggagacaggg agaggct 27

<210> 30
<400> 30
000

<210> 31
<400> 31
000

<210> 32
<400> 32
000

<210> 33
<400> 33
000

<210> 34
<400> 34
000

<210> 35
<400> 35
000

<210> 36
<211> 37
<212> DNA
<213> Artificial

<220>
<223> An artificially synthesized primer sequence

<400> 36
accgctcgag atggattttc aggtgcagat tatcagc 37

<210> 37
<211> 27
<212> DNA
<213> Artificial

<220>
<223> An artificially synthesized primer sequence

<400> 37
tttcagctcc agcttggtcc cagcacc 27

<210> 38
<211> 27
<212> DNA
<213> Artificial

<220>
<223> An artificially synthesized primer sequence

<400> 38
actgtggctg caccatctgt cttcatc 27

<210> 39
<211> 46
<212> DNA
<213> Artificial

<220>
<223> An artificially synthesized primer sequence

<400> 39
atagtttagc ggccgcttaa cactctcccc tgttgaagct ctttgt 46

<210> 40
<211> 37
<212> DNA
<213> Artificial

<220>
<223> An artificially synthesized primer sequence

<400> 40
accgctcgag atgggatgga gctgggtctt tctcttc 37

<210> 41
<211> 24
<212> DNA
<213> Artificial

<220>
<223> An artificially synthesized primer sequence

<400> 41
tgaggagacg gtgaccgtgg tccc 24

<210> 42
<211> 24
<212> DNA
<213> Artificial

<220>
<223> An artificially synthesized primer sequence

<400> 42
gcctccacca agggcccatc ggtc 24

<210> 43
<211> 46
<212> DNA
<213> Artificial

<220>
<223> An artificially synthesized primer sequence

<400> 43
atagtttagc ggccgcttat ttacccggag acagggagag gctctt 46

<210> 44
<211> 1413
<212> DNA
<213> Artificial

<220>
<223> An artificially synthesized nucleotide sequence

<400> 44
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga gtgccagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 45
<211> 471
<212> PRT
<213> Artificial

<220>
<223> An artificially synthesized peptide sequence

<400> 45

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Cys Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 46
<211> 15
<212> DNA
<213> Mus musculus

<400> 46
agttacaata tgcac 15

<210> 47
<211> 5
<212> PRT
<213> Mus musculus

<400> 47

Ser Tyr Asn Met His
1 5

<210> 48
<211> 51
<212> DNA
<213> Mus musculus

<400> 48
gctatttatc caggaaatgg tgatacttcc tacaatcaga agttcaaggg c 51

<210> 49
<211> 17
<212> PRT
<213> Mus musculus

<400> 49

Ala Ile Tyr Pro Gly Asn Gly Asp Thr Ser Tyr Asn Gln Lys Phe Lys
1 5 10 15

Gly

<210> 50
<211> 39
<212> DNA
<213> Mus musculus

<400> 50
gtggtgtact atagtaactc ttactggtac ttcgatgtc 39

<210> 51
<211> 13
<212> PRT
<213> Mus musculus

<400> 51

Val Val Tyr Tyr Ser Asn Ser Tyr Trp Tyr Phe Asp Val
1 5 10

<210> 52
<211> 990
<212> DNA
<213> Artificial

<220>
<223> An artificially synthesized nucleotide sequence

<400> 52
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagtg ccagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 53
<211> 330
<212> PRT
<213> Homo sapiens

<400> 53

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 54
<211> 330
<212> PRT
<213> Artificial

<220>
<223> An artificially synthesized peptide sequence

<400> 54

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Ala Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Cys Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 55
<211> 702
<212> DNA
<213> Artificial

<220>
<223> An artificially synthesized nucleotide sequence

<400> 55
atggattttc aggtgcagat tatcagcttc ctgctaatca gtgcttcagt cataatgtcc 60

agaggacaaa ttgttctctc ccagtctcca gcaatcctgt ctgcatctcc aggggagaag 120

gtcacaatga cttgcagggc cagctcaagt gtaagttaca tgcactggta ccagcagaag 180

```
ccaggatcct cccccaaacc ctggatttat gccccatcca acctggcttc tggagtccct     240
gctcgcttca gtggcagtgg gtctgggacc tcttactctc tcacaatcag cagagtggag     300
gctgaagatg ctgccactta ttactgccag cagtggagtt ttaacccacc cacgttcggt     360
gctgggacca agctggagct gaaaactgtg gctgcaccat ctgtcttcat cttcccgcca     420
tctgatgagc agttgaaatc tggaactgcc tctgttgtgt gcctgctgaa taacttctat     480
cccagagagg ccaaagtaca gtggaaggtg gataacgccc tccaatcggg taactcccag     540
gagagtgtca cagagcagga cagcaaggac agcacctaca gcctcagcag caccctgacg     600
ctgagcaaag cagactacga gaaacacaaa gtctacgcct gcgaagtcac ccatcagggc     660
ctgagctcgc ccgtcacaaa gagcttcaac aggggagagt gt                        702
```

```
<210>  56
<211>  234
<212>  PRT
<213>  Artificial

<220>
<223>  An artificially synthesized peptide sequence

<400>  56

Met Asp Phe Gln Val Gln Ile Ile Ser Phe Leu Leu Ile Ser Ala Ser
1               5                   10                  15

Val Ile Met Ser Arg Gly Gln Ile Val Leu Ser Gln Ser Pro Ala Ile
            20                  25                  30

Leu Ser Ala Ser Pro Gly Glu Lys Val Thr Met Thr Cys Arg Ala Ser
        35                  40                  45

Ser Ser Val Ser Tyr Met His Trp Tyr Gln Gln Lys Pro Gly Ser Ser
    50                  55                  60

Pro Lys Pro Trp Ile Tyr Ala Pro Ser Asn Leu Ala Ser Gly Val Pro
65                  70                  75                  80

Ala Arg Phe Ser Gly Ser Gly Ser Gly Thr Ser Tyr Ser Leu Thr Ile
                85                  90                  95

Ser Arg Val Glu Ala Glu Asp Ala Ala Thr Tyr Tyr Cys Gln Gln Trp
            100                 105                 110

Ser Phe Asn Pro Pro Thr Phe Gly Ala Gly Thr Lys Leu Glu Leu Lys
        115                 120                 125

Thr Val Ala Ala Pro Ser Val Phe Ile Phe Pro Pro Ser Asp Glu Gln
    130                 135                 140
```

```
Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  57
<211>  30
<212>  DNA
<213>  Mus musculus

<400>  57
agggccagct caagtgtaag ttacatgcac                                        30


<210>  58
<211>  10
<212>  PRT
<213>  Mus musculus

<400>  58

Arg Ala Ser Ser Ser Val Ser Tyr Met His
1               5                   10


<210>  59
<211>  21
<212>  DNA
<213>  Mus musculus

<400>  59
gccccatcca acctggcttc t                                                 21


<210>  60
<211>  7
<212>  PRT
<213>  Mus musculus

<400>  60

Ala Pro Ser Asn Leu Ala Ser
1               5


<210>  61
```

<211> 27
<212> DNA
<213> Mus musculus

<400> 61
cagcagtgga gttttaaccc acccacg 27

<210> 62
<211> 9
<212> PRT
<213> Mus musculus

<400> 62

Gln Gln Trp Ser Phe Asn Pro Pro Thr
1 5

<210> 63
<211> 318
<212> DNA
<213> Homo sapiens

<400> 63
actgtggctg caccatctgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgt 318

<210> 64
<211> 106
<212> PRT
<213> Homo sapiens

<400> 64

Thr Val Ala Ala Pro Ser Val Phe Ile Phe Pro Pro Ser Asp Glu Gln
1 5 10 15

Leu Lys Ser Gly Thr Ala Ser Val Val Cys Leu Leu Asn Asn Phe Tyr
20 25 30

Pro Arg Glu Ala Lys Val Gln Trp Lys Val Asp Asn Ala Leu Gln Ser
35 40 45

Gly Asn Ser Gln Glu Ser Val Thr Glu Gln Asp Ser Lys Asp Ser Thr
50 55 60

Tyr Ser Leu Ser Ser Thr Leu Thr Leu Ser Lys Ala Asp Tyr Glu Lys
65 70 75 80

```
His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  65
<211>  978
<212>  DNA
<213>  Homo sapiens

<400>  65
gcctccacca agggcccatc ggtcttcccc ctggcgccct gctccaggag cacctccgag      60

agcacagccg ccctgggctg cctggtcaag gactacttcc ccgaaccggt gacggtgtcg     120

tggaactcag gcgctctgac cagcggcgtg cacaccttcc cagctgtcct acagtcctca     180

ggactctact ccctcagcag cgtggtgacc gtgccctcca gcaacttcgg cacccagacc     240

tacacctgca acgtagatca caagcccagc aacaccaagg tggacaagac agttgagcgc     300

aaatgttgtg tcgagtgccc accgtgccca gcaccacctg tggcaggacc gtcagtcttc     360

ctcttccccc caaaacccaa ggacaccctc atgatctccc ggacccctga ggtcacgtgc     420

gtggtggtgg acgtgagcca cgaagacccc gaggtccagt tcaactggta cgtggacggc     480

gtggaggtgc ataatgccaa gacaaagcca cgggaggagc agttcaacag cacgttccgt     540

gtggtcagcg tcctcaccgt tgtgcaccag gactggctga acggcaagga gtacaagtgc     600

aaggtctcca acaaaggcct cccagccccc atcgagaaaa ccatctccaa aaccaaaggg     660

cagccccgag aaccacaggt gtacaccctg cccccatccc gggaggagat gaccaagaac     720

caggtcagcc tgacctgcct ggtcaaaggc ttctacccca gcgacatcgc cgtggagtgg     780

gagagcaatg ggcagccgga gaacaactac aagaccacac ctcccatgct ggactccgac     840

ggctccttct tcctctacag caagctcacc gtggacaaga gcaggtggca gcaggggaac     900

gtcttctcat gctccgtgat gcatgaggct ctgcacaacc actacacgca gaagagcctc     960

tccctgtctc cgggtaaa                                                   978

<210>  66
<211>  326
<212>  PRT
<213>  Homo sapiens

<400>  66

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
```

35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Cys Cys Val Glu Cys Pro Pro Cys Pro Ala Pro
100 105 110

Pro Val Ala Gly Pro Ser Val Phe Leu Phe Pro Pro Lys Pro Lys Asp
115 120 125

Thr Leu Met Ile Ser Arg Thr Pro Glu Val Thr Cys Val Val Val Asp
130 135 140

Val Ser His Glu Asp Pro Glu Val Gln Phe Asn Trp Tyr Val Asp Gly
145 150 155 160

Val Glu Val His Asn Ala Lys Thr Lys Pro Arg Glu Glu Gln Phe Asn
165 170 175

Ser Thr Phe Arg Val Val Ser Val Leu Thr Val Val His Gln Asp Trp
180 185 190

Leu Asn Gly Lys Glu Tyr Lys Cys Lys Val Ser Asn Lys Gly Leu Pro
195 200 205

Ala Pro Ile Glu Lys Thr Ile Ser Lys Thr Lys Gly Gln Pro Arg Glu
210 215 220

Pro Gln Val Tyr Thr Leu Pro Pro Ser Arg Glu Glu Met Thr Lys Asn
225 230 235 240

Gln Val Ser Leu Thr Cys Leu Val Lys Gly Phe Tyr Pro Ser Asp Ile
245 250 255

Ala Val Glu Trp Glu Ser Asn Gly Gln Pro Glu Asn Asn Tyr Lys Thr
260 265 270

Thr Pro Pro Met Leu Asp Ser Asp Gly Ser Phe Phe Leu Tyr Ser Lys
275 280 285

Leu Thr Val Asp Lys Ser Arg Trp Gln Gln Gly Asn Val Phe Ser Cys
290 295 300

Ser Val Met His Glu Ala Leu His Asn His Tyr Thr Gln Lys Ser Leu
305 310 315 320

Ser Leu Ser Pro Gly Lys
325

<210> 67
<211> 1131
<212> DNA
<213> Homo sapiens

<400> 67
gcttccacca agggcccatc ggtcttcccc ctggcgccct gctccaggag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacacctgca acgtgaatca caagcccagc aacaccaagg tggacaagag agttgagctc 300

aaaaccccac ttggtgacac aactcacaca tgcccacggt gcccagagcc caaatcttgt 360

gacacacctc ccccgtgccc acggtgccca gagcccaaat cttgtgacac acctccccca 420

tgcccacggt gcccagagcc caaatcttgt gacacacctc ccccgtgccc aaggtgccca 480

gcacctgaac tcctgggagg accgtcagtc ttcctcttcc ccccaaaacc caaggatacc 540

cttatgattt cccggacccc tgaggtcacg tgcgtggtgg tggacgtgag ccacgaagac 600

cccgaggtcc agttcaagtg gtacgtggac ggcgtggagg tgcataatgc caagacaaag 660

ccgcgggagg agcagtacaa cagcacgttc cgtgtggtca gcgtcctcac cgtcctgcac 720

caggactggc tgaacggcaa ggagtacaag tgcaaggtct ccaacaaagc cctcccagcc 780

cccatcgaga aaaccatctc caaaaccaaa ggacagcccc gagaaccaca ggtgtacacc 840

ctgcccccat cccgggagga gatgaccaag aaccaggtca gcctgacctg cctggtcaaa 900

ggcttctacc ccagcgacat cgccgtggag tgggagagca gcgggcagcc ggagaacaac 960

tacaagacca cgcctcccat gctggactcc gacggctcct tcttcctcta cagcaagctc 1020

accgtggaca agagcaggtg gcagcagggg aacatcttct catgctccgt gatgcatgag 1080

gctctgcaca accgcttcac gcagaagagc ctctccctgt ctccgggtaa a 1131

<210> 68
<211> 377
<212> PRT
<213> Homo sapiens

<400> 68

Ala Ser Thr Lys Gly Pro Ser Val Phe Pro Leu Ala Pro Cys Ser Arg
1 5 10 15

```
Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270
```

```
Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Lys Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375


<210>  69
<211>  981
<212>  DNA
<213>  Homo sapiens

<400>  69
gcttccacca agggcccatc cgtcttcccc ctggcgccct gctccaggag cacctccgag      60

agcacagccg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca acgtagatca caagcccagc aacaccaagg tggacaagag agttgagtcc     300

aaatatggtc ccccatgccc atcatgccca gcacctgagt tcctgggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat     480

ggcgtggagg tgcataatgc caagacaaag ccgcgggagg agcagttcaa cagcacgtac     540

cgtgtggtca gcgtcctcac cgtcctgcac caggactggc tgaacggcaa ggagtacaag     600

tgcaaggtct ccaacaaagg cctcccgtcc tccatcgaga aaaccatctc caaagccaaa     660

gggcagcccc gagagccaca ggtgtacacc ctgcccccat cccaggagga gatgaccaag     720

aaccaggtca gcctgacctg cctggtcaaa ggcttctacc ccagcgacat cgccgtggag     780

tgggagagca atgggcagcc ggagaacaac tacaagacca cgcctcccgt gctggactcc     840

gacggctcct tcttcctcta cagcaggcta accgtggaca agagcaggtg gcaggagggg     900

aatgtcttct catgctccgt gatgcatgag gctctgcaca accactacac acagaagagc     960
```

ctctccctgt ctctgggtaa a 981

<210> 70
<211> 327
<212> PRT
<213> Homo sapiens

<400> 70

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220
```

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 71
<211> 990
<212> DNA
<213> Homo sapiens

<400> 71
gcctccacca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agcagagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgtgagga gcagtacaac 540
agcacgtacc gtgtggtcag cgtcctcacc gtcctgcacc aggactggct gaatggcaag 600
gagtacaagt gcaaggtctc caacaaagcc ctcccagccc ccatcgagaa aaccatctcc 660
aaagccaaag ggcagccccg agaaccacag gtgtacaccc tgcccccatc ccgggatgag 720
ctgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900

cagcaggga acgtcttctc atgctccgtg atgcatgagg ctctgcacaa ccactacacg 960

cagaagagcc tctccctgtc tccgggtaaa 990

<210> 72
<211> 651
<212> DNA
<213> Artificial

<220>
<223> An artificially synthesized nucleotide sequence

<400> 72
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagt gccagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 73
<211> 217
<212> PRT
<213> Artificial

<220>
<223> An artificially synthesized peptide sequence

<400> 73

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Cys
    50                  55                  60

Gln Tyr Asn Ser Thr Tyr Arg Val Val Ser Val Leu Thr Val Leu His

65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 74
<211> 27
<212> DNA
<213> Artificial

<220>
<223> An artificially synthesized primer sequence

<400> 74
aagccgcgtg agtgccagta caacagc 27

<210> 75
<211> 27
<212> DNA
<213> Artificial

<220>
<223> An artificially synthesized primer sequence

<400> 75
gctgttgtac tggcactcac gcggctt 27

<210> 76
<211> 27

<212> DNA
<213> Artificial

<220>
<223> An artificially synthesized primer sequence

<400> 76
gagcagtaca actgcacgta ccgtgtg 27

<210> 77
<211> 27
<212> DNA
<213> Artificial

<220>
<223> An artificially synthesized primer sequence

<400> 77
cacacggtac gtgcagttgt actgctc 27

<210> 78
<211> 33
<212> DNA
<213> Artificial

<220>
<223> An artificially synthesized primer sequence

<400> 78
tacaacagca cgtactgtgt ggtcagcgtc ctc 33

<210> 79
<211> 33
<212> DNA
<213> Artificial

<220>
<223> An artificially synthesized primer sequence

<400> 79
gaggacgctg accacacagt acgtgctgtt gta 33

<210> 80
<211> 1413
<212> DNA
<213> Artificial

<220>
<223> an artificially synthesized nucleotide sequence

<400> 80
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360
tatagtaact cttactggta cttcgatgtc tggggcacag ggaccacggt caccgtctcc 420
tcagcctcca ccaagggccc atcggtcttc cccctggcac cctcctccaa gagcacctct 480
gggggcacag cggccctggg ctgcctggtc aaggactact tccccgaacc ggtgacggtg 540
tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagcagag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900
tggtacgtgg acggcgtgga ggtgcataat gccaagacaa agccgcgtga ggagcagtac 960
aactgcacgt accgtgtggt cagcgtcctc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 81
<211> 471
<212> PRT
<213> Artificial

<220>
<223> An artificially synthesized peptide sequence

<400> 81

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

```
Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Cys Thr Tyr Arg Val Val Ser Val Leu Thr Val Leu His Gln Asp
```

325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 82
<211> 990
<212> DNA
<213> Artificial

<220>
<223> an artificially synthesized nucleotide sequence

<400> 82
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagga gcagtacaac 540

tgcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 83
<211> 330
<212> PRT
<213> Artificial

<220>
<223> An artificially synthesized peptide sequence

<400> 83

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Ala Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp

145 150 155 160

Tyr Val Asp Gly Val Glu Val His Asn Ala Lys Thr Lys Pro Arg Glu
165 170 175

Glu Gln Tyr Asn Cys Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 84
<211> 651
<212> DNA
<213> Artificial

<220>
<223> an artificially synthesized nucleotide sequence

<400> 84
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa ctgcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 85
<211> 217
<212> PRT
<213> Artificial

<220>
<223> An artificially synthesized peptide sequence

<400> 85

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Cys Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu

165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 86
<211> 1413
<212> DNA
<213> Artificial

<220>
<223> an artificially synthesized nucleotide sequence

<400> 86
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960

aacagcacgt actgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 87
<211> 471
<212> PRT
<213> Artificial

<220>
<223> An artificially synthesized peptide sequence

<400> 87

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

```
Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Cys Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445
```

```
Cys Ser Val Met His Glu Ala Leu His Asn His Tyr Thr Gln Lys Ser
    450                 455                 460

Leu Ser Leu Ser Pro Gly Lys
465                 470
```

<210> 88
<211> 990
<212> DNA
<213> Artificial

<220>
<223> an artificially synthesized nucleotide sequence

<400> 88

```
gcctccacca agggcccatc ggtcttcccc ctggcaccct cctccaagag cacctctggg     60

ggcacagcgg ccctgggctg cctggtcaag gactacttcc ccgaaccggt gacggtgtcg    120

tggaactcag gcgccctgac cagcggcgtg cacaccttcc cggctgtcct acagtcctca    180

ggactctact ccctcagcag cgtggtgacc gtgccctcca gcagcttggg cacccagacc    240

tacatctgca acgtgaatca caagcccagc aacaccaagg tggacaagaa agcagagccc    300

aaatcttgtg acaaaactca cacatgccca ccgtgcccag cacctgaact cctgggggga    360

ccgtcagtct tcctcttccc cccaaaaccc aaggacaccc tcatgatctc ccggacccct    420

gaggtcacat gcgtggtggt ggacgtgagc cacgaagacc ctgaggtcaa gttcaactgg    480

tacgtggacg gcgtggaggt gcataatgcc aagacaaagc cgcgtgagga gcagtacaac    540

agcacgtact gtgtggtcag cgtcctcacc gtcctgcacc aggactggct gaatggcaag    600

gagtacaagt gcaaggtctc caacaaagcc ctcccagccc ccatcgagaa aaccatctcc    660

aaagccaaag ggcagccccg agaaccacag gtgtacaccc tgcccccatc ccgggatgag    720

ctgaccaaga accaggtcag cctgacctgc ctggtcaaag gcttctatcc cagcgacatc    780

gccgtggagt gggagagcaa tgggcagccg gagaacaact acaagaccac gcctcccgtg    840

ctggactccg acggctcctt cttcctctac agcaagctca ccgtggacaa gagcaggtgg    900

cagcagggga acgtcttctc atgctccgtg atgcatgagg ctctgcacaa ccactacacg    960

cagaagagcc tctccctgtc tccgggtaaa                                     990
```

<210> 89
<211> 330
<212> PRT
<213> Artificial

<220>
<223> An artificially synthesized peptide sequence

<400> 89

```
Ala Ser Thr Lys Gly Pro Ser Val Phe Pro Leu Ala Pro Ser Ser Lys
1               5                   10                  15
```

```
Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Cys Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270
```

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 90
<211> 651
<212> DNA
<213> Artificial

<220>
<223> an artificially synthesized nucleotide sequence

<400> 90
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac tgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 91
<211> 217
<212> PRT
<213> Artificial

<220>
<223> An artificially synthesized peptide sequence

<400> 91

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Cys Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 92
<211> 1413
<212> DNA
<213> Artificial

<220>
<223> an artificially synthesized nucleotide sequence

<400> 92
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

```
cagaagttca agggcaaggc cacactgact gtagacaaat cctccagcac agcctacatg    300
cagctcagca gcctgacatc tgaagactct gcggtctatt tctgtgcaag agtggtgtac    360
tatagtaact cttactggta cttcgatgtc tggggcacag ggaccacggt caccgtctcc    420
tcagcctcca ccaagggccc atcggtcttc cccctggcac cctcctccaa gagcacctct    480
gggggcacag cggccctggg ctgcctggtc aaggactact tccccgaacc ggtgacggtg    540
tcgtggaact caggcgccct gaccagcggc gtgcacacct tcccggctgt cctacagtcc    600
tcaggactct actccctcag cagcgtggtg accgtgccct ccagcagctt gggcacccag    660
acctacatct gcaacgtgaa tcacaagccc agcaacacca aggtggacaa gaaagcagag    720
cccaaatctt gtgacaaaac tcacacatgc ccaccgtgcc cagcacctga actcctgggg    780
ggaccgtcag tcttcctctt ccccccaaaa cccaaggaca ccctcatgat ctcccggacc    840
cctgaggtca catgcgtggt ggtggacgtg agccacgaag accctgaggt caagttcaac    900
tggtacgtgg acggcgtgga ggtgcataat gccaagacat gcccgcgtga ggagcagtac    960
aacagcacgt accgtgtggt cagcgtcctc accgtcctgc accaggactg gctgaatggc   1020
aaggagtaca agtgcaaggt ctccaacaaa gccctcccag cccccatcga gaaaaccatc   1080
tccaaagcca aagggcagcc ccgagaacca caggtgtaca ccctgccccc atcccgggat   1140
gagctgacca agaaccaggt cagcctgacc tgcctggtca aaggcttcta tcccagcgac   1200
atcgccgtgg agtgggagag caatgggcag ccggagaaca actacaagac cacgcctccc   1260
gtgctggact ccgacggctc cttcttcctc tacagcaagc tcaccgtgga caagagcagg   1320
tggcagcagg ggaacgtctt ctcatgctcc gtgatgcatg aggctctgca caaccactac   1380
acgcagaaga gcctctccct gtctccgggt aaa                                1413
```

<210> 93
<211> 471
<212> PRT
<213> Artificial

<220>
<223> An artificially synthesized peptide sequence

<400> 93

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
```

50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Cys Pro Arg Glu Glu Gln Tyr
305 310 315 320

```
Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470


<210>  94
<211>  990
<212>  DNA
<213>  Artificial

<220>
<223>  an artificially synthesized nucleotide sequence

<400>  94
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agcagagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420
```

```
gaggtcacat gcgtggtggt ggacgtgagc cacgaagacc ctgaggtcaa gttcaactgg   480
tacgtggacg gcgtggaggt gcataatgcc aagacatgcc cgcgtgagga gcagtacaac   540
agcacgtacc gtgtggtcag cgtcctcacc gtcctgcacc aggactggct gaatggcaag   600
gagtacaagt gcaaggtctc caacaaagcc ctcccagccc ccatcgagaa aaccatctcc   660
aaagccaaag ggcagccccg agaaccacag gtgtacaccc tgcccccatc ccgggatgag   720
ctgaccaaga accaggtcag cctgacctgc ctggtcaaag gcttctatcc cagcgacatc   780
gccgtggagt gggagagcaa tgggcagccg gagaacaact acaagaccac gcctcccgtg   840
ctggactccg acggctcctt cttcctctac agcaagctca ccgtggacaa gagcaggtgg   900
cagcagggga acgtcttctc atgctccgtg atgcatgagg ctctgcacaa ccactacacg   960
cagaagagcc tctccctgtc tccgggtaaa                                    990
```

<210> 95
<211> 330
<212> PRT
<213> Artificial

<220>
<223> An artificially synthesized peptide sequence

<400> 95

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140
```

Val Val Val Asp Val Ser His Glu Asp Pro Glu Val Lys Phe Asn Trp
145 150 155 160

Tyr Val Asp Gly Val Glu Val His Asn Ala Lys Thr Cys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 96
<211> 651
<212> DNA
<213> Artificial

<220>
<223> an artificially synthesized nucleotide sequence

<400> 96
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacatgc 180

ccgcgtgagg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 97
<211> 217
<212> PRT
<213> Artificial

<220>
<223> An artificially synthesized peptide sequence

<400> 97

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Cys Pro Arg Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 98
<211> 1413
<212> DNA
<213> Artificial

<220>
<223> an artificially synthesized nucleotide sequence

<400> 98
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agtgccgtga ggagcagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 99
<211> 471
<212> PRT
<213> Artificial

<220>
<223> An artificially synthesized peptide sequence

<400> 99

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175
```

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Cys Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser

435 440 445

Cys Ser Val Met His Glu Ala Leu His Asn His Tyr Thr Gln Lys Ser
450 455 460

Leu Ser Leu Ser Pro Gly Lys
465 470

<210> 100
<211> 990
<212> DNA
<213> Artificial

<220>
<223> an artificially synthesized nucleotide sequence

<400> 100
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagt gccgtgagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 101
<211> 330
<212> PRT
<213> Artificial

<220>
<223> An artificially synthesized peptide sequence

<400> 101

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Cys Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
```

260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 102
<211> 651
<212> DNA
<213> Artificial

<220>
<223> an artificially synthesized nucleotide sequence

<400> 102
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

tgccgtgagg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 103
<211> 217
<212> PRT
<213> Artificial

<220>
<223> An artificially synthesized peptide sequence

<400> 103

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Cys Arg Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 104
<211> 1413
<212> DNA
<213> Artificial

<220>
<223> an artificially synthesized nucleotide sequence

<400> 104
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

```
tgcaaggctt ctggctacac atttaccagt tacaatatgc actgggtaaa gcagacacct    180
agacagggcc tggaatggat tggagctatt tatccaggaa atggtgatac ttcctacaat    240
cagaagttca agggcaaggc cacactgact gtagacaaat cctccagcac agcctacatg    300
cagctcagca gcctgacatc tgaagactct gcggtctatt tctgtgcaag agtggtgtac    360
tatagtaact cttactggta cttcgatgtc tggggcacag ggaccacggt caccgtctcc    420
tcagcctcca ccaagggccc atcggtcttc cccctggcac cctcctccaa gagcacctct    480
gggggcacag cggccctggg ctgcctggtc aaggactact tccccgaacc ggtgacggtg    540
tcgtggaact caggcgccct gaccagcggc gtgcacacct tcccggctgt cctacagtcc    600
tcaggactct actccctcag cagcgtggtg accgtgccct ccagcagctt gggcacccag    660
acctacatct gcaacgtgaa tcacaagccc agcaacacca aggtggacaa gaaagcagag    720
cccaaatctt gtgacaaaac tcacacatgc ccaccgtgcc cagcacctga actcctgggg    780
ggaccgtcag tcttcctctt ccccccaaaa cccaaggaca ccctcatgat ctcccggacc    840
cctgaggtca catgcgtggt ggtggacgtg agccacgaag accctgaggt caagttcaac    900
tggtacgtgg acggcgtgga ggtgcataat gccaagacaa agccgtgtga ggagcagtac    960
aacagcacgt accgtgtggt cagcgtcctc accgtcctgc accaggactg gctgaatggc   1020
aaggagtaca agtgcaaggt ctccaacaaa gccctcccag cccccatcga gaaaaccatc   1080
tccaaagcca aagggcagcc ccgagaacca caggtgtaca ccctgccccc atcccgggat   1140
gagctgacca agaaccaggt cagcctgacc tgcctggtca aaggcttcta tcccagcgac   1200
atcgccgtgg agtgggagag caatgggcag ccggagaaca actacaagac cacgcctccc   1260
gtgctggact ccgacggctc cttcttcctc tacagcaagc tcaccgtgga caagagcagg   1320
tggcagcagg ggaacgtctt ctcatgctcc gtgatgcatg aggctctgca caaccactac   1380
acgcagaaga gcctctccct gtctccgggt aaa                                1413
```

<210> 105
<211> 471
<212> PRT
<213> Artificial

<220>
<223> An artificially synthesized peptide sequence

<400> 105

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45
```

```
Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300
```

Gly Val Glu Val His Asn Ala Lys Thr Lys Pro Cys Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 106
<211> 990
<212> DNA
<213> Artificial

<220>
<223> an artificially synthesized nucleotide sequence

<400> 106
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

```
ccgtcagtct tcctcttccc cccaaaaccc aaggacaccc tcatgatctc ccggacccct    420
gaggtcacat gcgtggtggt ggacgtgagc cacgaagacc ctgaggtcaa gttcaactgg    480
tacgtggacg gcgtggaggt gcataatgcc aagacaaagc cgtgtgagga gcagtacaac    540
agcacgtacc gtgtggtcag cgtcctcacc gtcctgcacc aggactggct gaatggcaag    600
gagtacaagt gcaaggtctc caacaaagcc ctcccagccc ccatcgagaa aaccatctcc    660
aaagccaaag ggcagccccg agaaccacag gtgtacaccc tgcccccatc ccgggatgag    720
ctgaccaaga accaggtcag cctgacctgc ctggtcaaag gcttctatcc cagcgacatc    780
gccgtggagt gggagagcaa tgggcagccg gagaacaact acaagaccac gcctcccgtg    840
ctggactccg acggctcctt cttcctctac agcaagctca ccgtggacaa gagcaggtgg    900
cagcagggga acgtcttctc atgctccgtg atgcatgagg ctctgcacaa ccactacacg    960
cagaagagcc tctccctgtc tccgggtaaa                                     990


<210>  107
<211>  330
<212>  PRT
<213>  Artificial

<220>
<223>  An artificially synthesized peptide sequence

<400>  107

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125
```

```
Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Cys Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  108
<211>  651
<212>  DNA
<213>  Artificial

<220>
<223>  an artificially synthesized nucleotide sequence

<400>  108
gcacctgaac tcctgggggg accgtcagtc ttcctcttcc ccccaaaacc caaggacacc      60

ctcatgatct cccggacccc tgaggtcaca tgcgtggtgg tggacgtgag ccacgaagac     120
```

cctgaggtca agttcaactg gtacgtggac ggcgtggagg tgcataatgc caagacaaag 180

ccgtgtgagg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 109
<211> 217
<212> PRT
<213> Artificial

<220>
<223> An artificially synthesized peptide sequence

<400> 109

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Cys Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 110
<211> 1413
<212> DNA
<213> Artificial

<220>
<223> an artificially synthesized nucleotide sequence

<400> 110
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgttg cgagcagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 111
<211> 471
<212> PRT
<213> Artificial

<220>
<223> An artificially synthesized peptide sequence

<400> 111

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu

```
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Cys Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430
```

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 112
<211> 990
<212> DNA
<213> Artificial

<220>
<223> an artificially synthesized nucleotide sequence

<400> 112
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgttgcga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 113
<211> 330
<212> PRT
<213> Artificial

<220>
<223> An artificially synthesized peptide sequence

<400> 113

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Ala Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Cys
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 114
<211> 651
<212> DNA
<213> Artificial

<220>
<223> an artificially synthesized nucleotide sequence

<400> 114
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgttgcg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 115
<211> 434
<212> PRT
<213> Artificial

<220>
<223> An artificially synthesized peptide sequence

<400> 115

Ala Pro Glu Leu Leu Gly Gly Pro Ser Val Phe Leu Phe Pro Pro Lys

```
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Cys Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys     210                 215


<210>  116
<211>  1413
<212>  DNA
<213>  Artificial

<220>
<223>  an artificially synthesized nucleotide sequence

<400>  116
atgggatgga gctgggtctt tctcttcctc ctgtcagtaa ctacaggtgt ccactcccag      60
```

gcttatctac agcagtctgg ggctgagctg gtgaggcctg gggcctcagt gaagatgtcc 120
tgcaaggctt ctggctacac atttaccagt tacaatatgc actgggtaaa gcagacacct 180
agacagggcc tggaatggat tggagctatt tatccaggaa atggtgatac ttcctacaat 240
cagaagttca agggcaaggc cacactgact gtagacaaat cctccagcac agcctacatg 300
cagctcagca gcctgacatc tgaagactct gcggtctatt tctgtgcaag agtggtgtac 360
tatagtaact cttactggta cttcgatgtc tggggcacag ggaccacggt caccgtctcc 420
tcagcctcca ccaagggccc atcggtcttc cccctggcac cctcctccaa gagcacctct 480
gggggcacag cggccctggg ctgcctggtc aaggactact tccccgaacc ggtgacggtg 540
tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagcagag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900
tggtacgtgg acggcgtgga ggtgcataat gccaagacaa agccgcgtga ggagcagtac 960
aacagctgct accgtgtggt cagcgtcctc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 117
<211> 471
<212> PRT
<213> Artificial

<220>
<223> An artificially synthesized peptide sequence

<400> 117

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Cys Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 118
<211> 990
<212> DNA
<213> Artificial

<220>
<223> an artificially synthesized nucleotide sequence

<400> 118
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

```
tacatctgca acgtgaatca caagcccagc aacaccaagg tggacaagaa agcagagccc    300
aaatcttgtg acaaaactca cacatgccca ccgtgcccag cacctgaact cctgggggga    360
ccgtcagtct tcctcttccc cccaaaaccc aaggacaccc tcatgatctc ccggacccct    420
gaggtcacat gcgtggtggt ggacgtgagc cacgaagacc ctgaggtcaa gttcaactgg    480
tacgtggacg gcgtggaggt gcataatgcc aagacaaagc cgcgtgagga gcagtacaac    540
agctgctacc gtgtggtcag cgtcctcacc gtcctgcacc aggactggct gaatggcaag    600
gagtacaagt gcaaggtctc caacaaagcc ctcccagccc ccatcgagaa aaccatctcc    660
aaagccaaag ggcagccccg agaaccacag gtgtacaccc tgcccccatc ccgggatgag    720
ctgaccaaga accaggtcag cctgacctgc ctggtcaaag gcttctatcc cagcgacatc    780
gccgtggagt gggagagcaa tgggcagccg gagaacaact acaagaccac gcctcccgtg    840
ctggactccg acggctcctt cttcctctac agcaagctca ccgtggacaa gagcaggtgg    900
cagcagggga acgtcttctc atgctccgtg atgcatgagg ctctgcacaa ccactacacg    960
cagaagagcc tctccctgtc tccgggtaaa                                      990


<210>  119
<211>  330
<212>  PRT
<213>  Artificial

<220>
<223>  An artificially synthesized peptide sequence

<400>  119

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110
```

```
Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Cys Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  120
<211>  651
<212>  DNA
<213>  Artificial

<220>
<223>  an artificially synthesized nucleotide sequence

<400>  120
```

gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagctgctac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 121
<211> 217
<212> PRT
<213> Artificial

<220>
<223> An artificially synthesized peptide sequence

<400> 121

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Cys Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215

<210> 122
<211> 1413
<212> DNA
<213> Artificial

<220>
<223> an artificially synthesized nucleotide sequence

<400> 122
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960

aacagcacgt gccgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 123
<211> 471
<212> PRT
<213> Artificial

<220>
<223> An artificially synthesized peptide sequence

<400> 123

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

```
Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Cys Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415
```

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 124
<211> 990
<212> DNA
<213> Artificial

<220>
<223> an artificially synthesized nucleotide sequence

<400> 124
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagga gcagtacaac 540

agcacgtgcc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 125
<211> 330
<212> PRT
<213> Artificial

<220>
<223> An artificially synthesized peptide sequence

<400> 125

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Cys Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 126
<211> 651
<212> DNA
<213> Artificial

<220>
<223> an artificially synthesized nucleotide sequence.

<400> 126
gcacctgaac tcctgggggg accgtcagtc ttcctcttcc ccccaaaacc caaggacacc 60
ctcatgatct cccggacccc tgaggtcaca tgcgtggtgg tggacgtgag ccacgaagac 120
cctgaggtca agttcaactg gtacgtggac ggcgtggagg tgcataatgc caagacaaag 180
ccgcgtgagg agcagtacaa cagcacgtgc cgtgtggtca gcgtcctcac cgtcctgcac 240
caggactggc tgaatggcaa ggagtacaag tgcaaggtct ccaacaaagc cctcccagcc 300
cccatcgaga aaaccatctc caaagccaaa gggcagcccc gagaaccaca ggtgtacacc 360
ctgcccccat cccgggatga gctgaccaag aaccaggtca gcctgacctg cctggtcaaa 420
ggcttctatc ccagcgacat cgccgtggag tgggagagca atgggcagcc ggagaacaac 480
tacaagacca cgcctcccgt gctggactcc gacggctcct tcttcctcta cagcaagctc 540
accgtggaca agagcaggtg gcagcagggg aacgtcttct catgctccgt gatgcatgag 600
gctctgcaca accactacac gcagaagagc ctctccctgt ctccgggtaa a 651

<210> 127
<211> 217
<212> PRT
<213> Artificial

<220>
<223> An artificially synthesized peptide sequence

<400> 127

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Cys Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210> 128
<211> 1413
<212> DNA
<213> Artificial

<220>
<223> an artificially synthesized nucleotide sequence

<400> 128
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960

aacagcacgt accgttgcgt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 129
<211> 471
<212> PRT
<213> Artificial

<220>
<223> An artificially synthesized peptide sequence

<400> 129

Met Gly Trp Ser Trp Val Phe Leu Phe Leu Leu Ser Val Thr Thr Gly
1 5 10 15

```
Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
```

275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Cys Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 130
<211> 990
<212> DNA
<213> Artificial

<220>
<223> an artificially synthesized nucleotide sequence

<400> 130
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

```
ggactctact ccctcagcag cgtggtgacc gtgccctcca gcagcttggg cacccagacc    240
tacatctgca acgtgaatca caagcccagc aacaccaagg tggacaagaa agcagagccc    300
aaatcttgtg acaaaactca cacatgccca ccgtgcccag cacctgaact cctgggggga    360
ccgtcagtct tcctcttccc cccaaaaccc aaggacaccc tcatgatctc ccggacccct    420
gaggtcacat gcgtggtggt ggacgtgagc cacgaagacc ctgaggtcaa gttcaactgg    480
tacgtggacg gcgtggaggt gcataatgcc aagacaaagc cgcgtgagga gcagtacaac    540
agcacgtacc gttgcgtcag cgtcctcacc gtcctgcacc aggactggct gaatggcaag    600
gagtacaagt gcaaggtctc caacaaagcc ctcccagccc ccatcgagaa aaccatctcc    660
aaagccaaag ggcagccccg agaaccacag gtgtacaccc tgcccccatc ccgggatgag    720
ctgaccaaga accaggtcag cctgacctgc ctggtcaaag gcttctatcc cagcgacatc    780
gccgtggagt gggagagcaa tgggcagccg gagaacaact acaagaccac gcctcccgtg    840
ctggactccg acggctcctt cttcctctac agcaagctca ccgtggacaa gagcaggtgg    900
cagcagggga acgtcttctc atgctccgtg atgcatgagg ctctgcacaa ccactacacg    960
cagaagagcc tctccctgtc tccgggtaaa                                      990
```

<210> 131
<211> 330
<212> PRT
<213> Artificial

<220>
<223> An artificially synthesized peptide sequence

<400> 131

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
```

```
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Cys Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 132
<211> 651
<212> DNA
<213> Artificial

<220>

<223> an artificially synthesized nucleotide sequence

<400> 132
gcacctgaac tcctgggggg accgtcagtc ttcctcttcc ccccaaaacc caaggacacc 60
ctcatgatct cccggacccc tgaggtcaca tgcgtggtgg tggacgtgag ccacgaagac 120
cctgaggtca agttcaactg gtacgtggac ggcgtggagg tgcataatgc caagacaaag 180
ccgcgtgagg agcagtacaa cagcacgtac cgttgcgtca gcgtcctcac cgtcctgcac 240
caggactggc tgaatggcaa ggagtacaag tgcaaggtct ccaacaaagc cctcccagcc 300
cccatcgaga aaaccatctc caaagccaaa gggcagcccc gagaaccaca ggtgtacacc 360
ctgcccccat cccgggatga gctgaccaag aaccaggtca gcctgacctg cctggtcaaa 420
ggcttctatc ccagcgacat cgccgtggag tgggagagca atgggcagcc ggagaacaac 480
tacaagacca cgcctcccgt gctggactcc gacggctcct tcttcctcta cagcaagctc 540
accgtggaca agagcaggtg gcagcagggg aacgtcttct catgctccgt gatgcatgag 600
gctctgcaca accactacac gcagaagagc ctctccctgt ctccgggtaa a 651

<210> 133
<211> 217
<212> PRT
<213> Artificial

<220>
<223> An artificially synthesized peptide sequence

<400> 133

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Cys Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu

115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 134
<211> 1413
<212> DNA
<213> Artificial

<220>
<223> an artificially synthesized nucleotide sequence

<400> 134
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960
aacagcacgt accgtgtgtg cagcgtcctc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 135
<211> 471
<212> PRT
<213> Artificial

<220>
<223> An artificially synthesized peptide sequence

<400> 135

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

```
Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Cys Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400
```

```
Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 136
<211> 990
<212> DNA
<213> Artificial

<220>
<223> an artificially synthesized nucleotide sequence

<400> 136

```
gcctccacca agggcccatc ggtcttcccc ctggcaccct cctccaagag cacctctggg      60
ggcacagcgg ccctgggctg cctggtcaag gactacttcc ccgaaccggt gacggtgtcg     120
tggaactcag gcgccctgac cagcggcgtg cacaccttcc cggctgtcct acagtcctca     180
ggactctact ccctcagcag cgtggtgacc gtgccctcca gcagcttggg cacccagacc     240
tacatctgca acgtgaatca caagcccagc aacaccaagg tggacaagaa agcagagccc     300
aaatcttgtg acaaaactca cacatgccca ccgtgcccag cacctgaact cctgggggga     360
ccgtcagtct tcctcttccc cccaaaaccc aaggacaccc tcatgatctc ccggacccct     420
gaggtcacat gcgtggtggt ggacgtgagc cacgaagacc ctgaggtcaa gttcaactgg     480
tacgtggacg gcgtggaggt gcataatgcc aagacaaagc cgcgtgagga gcagtacaac     540
agcacgtacc gtgtgtgcag cgtcctcacc gtcctgcacc aggactggct gaatggcaag     600
gagtacaagt gcaaggtctc caacaaagcc ctcccagccc ccatcgagaa aaccatctcc     660
aaagccaaag ggcagccccg agaaccacag gtgtacaccc tgcccccatc ccgggatgag     720
ctgaccaaga accaggtcag cctgacctgc ctggtcaaag gcttctatcc cagcgacatc     780
gccgtggagt gggagagcaa tgggcagccg gagaacaact acaagaccac gcctcccgtg     840
ctggactccg acggctcctt cttcctctac agcaagctca ccgtggacaa gagcaggtgg     900
cagcagggga acgtcttctc atgctccgtg atgcatgagg ctctgcacaa ccactacacg     960
cagaagagcc tctccctgtc tccgggtaaa                                      990
```

<210> 137

<211> 330
<212> PRT
<213> Artificial

<220>
<223> An artificially synthesized peptide sequence

<400> 137

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Cys Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220
```

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 138
<211> 651
<212> DNA
<213> Artificial

<220>
<223> an artificially synthesized nucleotide sequence

<400> 138
gcacctgaac tcctgggggg accgtcagtc ttcctcttcc ccccaaaacc caaggacacc 60
ctcatgatct cccggacccc tgaggtcaca tgcgtggtgg tggacgtgag ccacgaagac 120
cctgaggtca agttcaactg gtacgtggac ggcgtggagg tgcataatgc caagacaaag 180
ccgcgtgagg agcagtacaa cagcacgtac cgtgtgtgca gcgtcctcac cgtcctgcac 240
caggactggc tgaatggcaa ggagtacaag tgcaaggtct ccaacaaagc cctcccagcc 300
cccatcgaga aaaccatctc caaagccaaa gggcagcccc gagaaccaca ggtgtacacc 360
ctgcccccat cccgggatga gctgaccaag aaccaggtca gcctgacctg cctggtcaaa 420
ggcttctatc ccagcgacat cgccgtggag tgggagagca atgggcagcc ggagaacaac 480
tacaagacca cgcctcccgt gctggactcc gacggctcct tcttcctcta cagcaagctc 540
accgtggaca agagcaggtg gcagcagggg aacgtcttct catgctccgt gatgcatgag 600
gctctgcaca accactacac gcagaagagc ctctccctgt ctccgggtaa a 651

<210> 139
<211> 217
<212> PRT
<213> Artificial

<220>
<223>  An artificially synthesized peptide sequence

<400>  139

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Cys Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210>  140
<211>  1413
<212>  DNA
<213>  Artificial

<220>
<223> an artificially synthesized nucleotide sequence

<400> 140
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960

aacagcacgt accgtgtggt ctgcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 141
<211> 471
<212> PRT
<213> Artificial

<220>
<223> An artificially synthesized peptide sequence

<400> 141

Met Gly Trp Ser Trp Val Phe Leu Phe Leu Leu Ser Val Thr Thr Gly

1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

```
Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Cys Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 142
<211> 990
<212> DNA
<213> Artificial

<220>
<223> an artificially synthesized nucleotide sequence

<400> 142
gcctccacca agggcccatc ggtcttcccc ctggcaccct cctccaagag cacctctggg 60

```
ggcacagcgg ccctgggctg cctggtcaag gactacttcc ccgaaccggt gacggtgtcg    120
tggaactcag gcgccctgac cagcggcgtg cacaccttcc cggctgtcct acagtcctca    180
ggactctact ccctcagcag cgtggtgacc gtgccctcca gcagcttggg cacccagacc    240
tacatctgca acgtgaatca caagcccagc aacaccaagg tggacaagaa agcagagccc    300
aaatcttgtg acaaaactca cacatgccca ccgtgcccag cacctgaact cctgggggga    360
ccgtcagtct tcctcttccc cccaaaaccc aaggacaccc tcatgatctc ccggacccct    420
gaggtcacat gcgtggtggt ggacgtgagc cacgaagacc ctgaggtcaa gttcaactgg    480
tacgtggacg gcgtggaggt gcataatgcc aagacaaagc cgcgtgagga gcagtacaac    540
agcacgtacc gtgtggtctg cgtcctcacc gtcctgcacc aggactggct gaatggcaag    600
gagtacaagt gcaaggtctc caacaaagcc ctcccagccc ccatcgagaa aaccatctcc    660
aaagccaaag ggcagccccg agaaccacag gtgtacaccc tgcccccatc ccgggatgag    720
ctgaccaaga accaggtcag cctgacctgc ctggtcaaag gcttctatcc cagcgacatc    780
gccgtggagt gggagagcaa tgggcagccg gagaacaact acaagaccac gcctcccgtg    840
ctggactccg acggctcctt cttcctctac agcaagctca ccgtggacaa gagcaggtgg    900
cagcagggga acgtcttctc atgctccgtg atgcatgagg ctctgcacaa ccactacacg    960
cagaagagcc tctccctgtc tccgggtaaa                                     990
```

```
<210>  143
<211>  330
<212>  PRT
<213>  Artificial

<220>
<223>  An artificially synthesized peptide sequence

<400>  143

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95
```

```
Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Cys Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 144
<211> 651
<212> DNA

<213> Artificial

<220>
<223> an artificially synthesized nucleotide sequence

<400> 144
gcacctgaac tcctgggggg accgtcagtc ttcctcttcc ccccaaaacc caaggacacc 60
ctcatgatct cccggacccc tgaggtcaca tgcgtggtgg tggacgtgag ccacgaagac 120
cctgaggtca agttcaactg gtacgtggac ggcgtggagg tgcataatgc caagacaaag 180
ccgcgtgagg agcagtacaa cagcacgtac cgtgtggtct gcgtcctcac cgtcctgcac 240
caggactggc tgaatggcaa ggagtacaag tgcaaggtct ccaacaaagc cctcccagcc 300
cccatcgaga aaaccatctc caaagccaaa gggcagcccc gagaaccaca ggtgtacacc 360
ctgcccccat cccgggatga gctgaccaag aaccaggtca gcctgacctg cctggtcaaa 420
ggcttctatc ccagcgacat cgccgtggag tgggagagca atgggcagcc ggagaacaac 480
tacaagacca cgcctcccgt gctggactcc gacggctcct tcttcctcta cagcaagctc 540
accgtggaca agagcaggtg gcagcagggg aacgtcttct catgctccgt gatgcatgag 600
gctctgcaca accactacac gcagaagagc ctctccctgt ctccgggtaa a 651

<210> 145
<211> 217
<212> PRT
<213> Artificial

<220>
<223> An artificially synthesized peptide sequence

<400> 145

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Val Val Cys Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 146
<211> 1413
<212> DNA
<213> Artificial

<220>
<223> An artificially synthesized nucleotide sequence

<400> 146
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

```
ggaccgtcag tcttcctctt ccccccaaaa cccaaggaca ccctcatgat ctcccggacc    840
cctgaggtca catgcgtggt ggtggacgtg agccacgaag accctgaggt caagttcaac    900
tggtacgtgg acggcgtgga ggtgcattgt gccaagacaa agccgcgtga ggagcagtac    960
aacagcacgt accgtgtggt cagcgtcctc accgtcctgc accaggactg gctgaatggc   1020
aaggagtaca agtgcaaggt ctccaacaaa gccctcccag cccccatcga gaaaaccatc   1080
tccaaagcca aagggcagcc ccgagaacca caggtgtaca ccctgccccc atcccgggat   1140
gagctgacca agaaccaggt cagcctgacc tgcctggtca aaggcttcta tcccagcgac   1200
atcgccgtgg agtgggagag caatgggcag ccggagaaca actacaagac cacgcctccc   1260
gtgctggact ccgacggctc cttcttcctc tacagcaagc tcaccgtgga caagagcagg   1320
tggcagcagg ggaacgtctt ctcatgctcc gtgatgcatg aggctctgca caaccactac   1380
acgcagaaga gcctctccct gtctccgggt aaa                                1413
```

```
<210>  147
<211>  471
<212>  PRT
<213>  Artificial

<220>
<223>  An artificially synthesized peptide sequence

<400>  147

Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125
```

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Cys Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp

385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 148
<211> 990
<212> DNA
<213> Artificial

<220>
<223> An artificially synthesized nucleotide sequence

<400> 148
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcattgtgcc aagacaaagc cgcgtgagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 149
<211> 330
<212> PRT
<213> Artificial

<220>
<223> An artificially synthesized peptide sequence

<400> 149

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Cys Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
```

210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 150
<211> 651
<212> DNA
<213> Artificial

<220>
<223> An artificially synthesized nucleotide sequence

<400> 150
gcacctgaac tcctgggggg accgtcagtc ttcctcttcc ccccaaaacc caaggacacc 60
ctcatgatct cccggacccc tgaggtcaca tgcgtggtgg tggacgtgag ccacgaagac 120
cctgaggtca agttcaactg gtacgtggac ggcgtggagg tgcattgtgc caagacaaag 180
ccgcgtgagg agcagtacaa cagcacgtac cgtgtggtca gcgtcctcac cgtcctgcac 240
caggactggc tgaatggcaa ggagtacaag tgcaaggtct ccaacaaagc cctcccagcc 300
cccatcgaga aaaccatctc caaagccaaa gggcagcccc gagaaccaca ggtgtacacc 360
ctgcccccat cccgggatga gctgaccaag aaccaggtca gcctgacctg cctggtcaaa 420
ggcttctatc ccagcgacat cgccgtggag tgggagagca atgggcagcc ggagaacaac 480
tacaagacca cgcctcccgt gctggactcc gacggctcct tcttcctcta cagcaagctc 540
accgtggaca agagcaggtg gcagcagggg aacgtcttct catgctccgt gatgcatgag 600
gctctgcaca accactacac gcagaagagc ctctccctgt ctccgggtaa a 651

<210> 151
<211> 235

<212> PRT
<213> Artificial

<220>
<223> An artificially synthesized peptide sequence

<400> 151

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Cys Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210> 152

<211> 1413
<212> DNA
<213> Artificial

<220>
<223> An artificially synthesized nucleotide sequence

<400> 152
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat tgcaagacaa agccgcgtga ggagcagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 153
<211> 471
<212> PRT
<213> Artificial

<220>
<223> An artificially synthesized peptide sequence

<400> 153

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Cys Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 154
<211> 990
<212> DNA
<213> Artificial

<220>
<223> An artificially synthesized nucleotide sequence

<400> 154
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataattgc aagacaaagc cgcgtgagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 155
<211> 330
<212> PRT
<213> Artificial

<220>
<223> An artificially synthesized peptide sequence

<400> 155

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

```
Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Cys Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 156
<211> 651
<212> DNA
<213> Artificial

<220>
<223> An artificially synthesized nucleotide sequence

<400> 156
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataattg caagacaaag 180

ccgcgtgagg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 157
<211> 217
<212> PRT
<213> Artificial

<220>
<223> An artificially synthesized peptide sequence

<400> 157

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Cys Lys Thr Lys Pro Arg Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 158
<211> 1413
<212> DNA
<213> Artificial

<220>
<223> An artificially synthesized nucleotide sequence

<400> 158
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gcctgtacaa agccgcgtga ggagcagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 159
<211> 471
<212> PRT
<213> Artificial

<220>
<223> An artificially synthesized peptide sequence

<400> 159

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe

115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Cys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 160
<211> 990
<212> DNA
<213> Artificial

<220>
<223> An artificially synthesized nucleotide sequence

<400> 160
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc tgtacaaagc cgcgtgagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 161
<211> 330
<212> PRT
<213> Artificial

<220>
<223> An artificially synthesized peptide sequence

<400> 161

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Ala Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Cys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

```
Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 162
<211> 651
<212> DNA
<213> Artificial

<220>
<223> An artificially synthesized nucleotide sequence

<400> 162

```
gcacctgaac tcctgggggg accgtcagtc ttcctcttcc ccccaaaacc caaggacacc      60
ctcatgatct cccggacccc tgaggtcaca tgcgtggtgg tggacgtgag ccacgaagac     120
cctgaggtca agttcaactg gtacgtggac ggcgtggagg tgcataatgc ctgtacaaag     180
ccgcgtgagg agcagtacaa cagcacgtac cgtgtggtca gcgtcctcac cgtcctgcac     240
caggactggc tgaatggcaa ggagtacaag tgcaaggtct ccaacaaagc cctcccagcc     300
cccatcgaga aaaccatctc caaagccaaa gggcagcccc gagaaccaca ggtgtacacc     360
ctgcccccat cccgggatga gctgaccaag aaccaggtca gcctgacctg cctggtcaaa     420
ggcttctatc ccagcgacat cgccgtggag tgggagagca atgggcagcc ggagaacaac     480
tacaagacca cgcctcccgt gctggactcc gacggctcct tcttcctcta cagcaagctc     540
accgtggaca agagcaggtg gcagcagggg aacgtcttct catgctccgt gatgcatgag     600
gctctgcaca accactacac gcagaagagc ctctccctgt ctccgggtaa a              651
```

<210> 163
<211> 217
<212> PRT
<213> Artificial

<220>
<223> An artificially synthesized peptide sequence

<400> 163

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Cys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210> 164
<211> 1413
<212> DNA
<213> Artificial

<220>
<223> An artificially synthesized nucleotide sequence

<400> 164
atgggatgga gctgggtctt tctcttcctc ctgtcagtaa ctacaggtgt ccactcccag 60
gcttatctac agcagtctgg ggctgagctg gtgaggcctg gggcctcagt gaagatgtcc 120
tgcaaggctt ctggctacac atttaccagt tacaatatgc actgggtaaa gcagacacct 180
agacagggcc tggaatggat tggagctatt tatccaggaa atggtgatac ttcctacaat 240
cagaagttca agggcaaggc cacactgact gtagacaaat cctccagcac agcctacatg 300
cagctcagca gcctgacatc tgaagactct gcggtctatt tctgtgcaag agtggtgtac 360
tatagtaact cttactggta cttcgatgtc tggggcacag ggaccacggt caccgtctcc 420
tcagcctcca ccaagggccc atcggtcttc cccctggcac cctcctccaa gagcacctct 480
gggggcacag cggccctggg ctgcctggtc aaggactact tccccgaacc ggtgacggtg 540
tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagcagag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900
tggtacgtgg acggcgtgga ggtgcataat gccaagtgca agccgcgtga ggagcagtac 960
aacagcacgt accgtgtggt cagcgtcctc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 165
<211> 471
<212> PRT
<213> Artificial

<220>
<223> An artificially synthesized peptide sequence

<400> 165

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp TyrPhe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

```
Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Cys Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470


<210>  166
<211>  990
<212>  DNA
<213>  Artificial
```

<220>
<223>  An artificially synthesized nucleotide sequence

<400>  166
gcctccacca agggcccatc ggtcttcccc ctggcaccct cctccaagag cacctctggg    60

ggcacagcgg ccctgggctg cctggtcaag gactacttcc ccgaaccggt gacggtgtcg   120

tggaactcag gcgccctgac cagcggcgtg cacaccttcc cggctgtcct acagtcctca   180

ggactctact ccctcagcag cgtggtgacc gtgccctcca gcagcttggg cacccagacc   240

tacatctgca acgtgaatca caagcccagc aacaccaagg tggacaagaa agcagagccc   300

aaatcttgtg acaaaactca cacatgccca ccgtgcccag cacctgaact cctgggggga   360

ccgtcagtct tcctcttccc cccaaaaccc aaggacaccc tcatgatctc ccggacccct   420

gaggtcacat gcgtggtggt ggacgtgagc cacgaagacc ctgaggtcaa gttcaactgg   480

tacgtggacg gcgtggaggt gcataatgcc aagtgcaagc cgcgtgagga gcagtacaac   540

agcacgtacc gtgtggtcag cgtcctcacc gtcctgcacc aggactggct gaatggcaag   600

gagtacaagt gcaaggtctc caacaaagcc ctcccagccc ccatcgagaa aaccatctcc   660

aaagccaaag ggcagccccg agaaccacag gtgtacaccc tgcccccatc ccgggatgag   720

ctgaccaaga accaggtcag cctgacctgc ctggtcaaag gcttctatcc cagcgacatc   780

gccgtggagt gggagagcaa tgggcagccg gagaacaact acaagaccac gcctcccgtg   840

ctggactccg acggctcctt cttcctctac agcaagctca ccgtggacaa gagcaggtgg   900

cagcagggga acgtcttctc atgctccgtg atgcatgagg ctctgcacaa ccactacacg   960

cagaagagcc tctccctgtc tccgggtaaa                                    990

<210>  167
<211>  330
<212>  PRT
<213>  Artificial

<220>
<223>  An artificially synthesized peptide sequence

<400>  167

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Ala Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Cys Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys

325 330

<210> 168
<211> 651
<212> DNA
<213> Artificial

<220>
<223> An artificially synthesized nucleotide sequence

<400> 168
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagtgcaag 180

ccgcgtgagg agcagtacaa cagcacgtac cgtgtggtca gcgtcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 169
<211> 217
<212> PRT
<213> Artificial

<220>
<223> An artificially synthesized peptide sequence

<400> 169

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Cys Lys Pro Arg Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 170
<211> 1413
<212> DNA
<213> Artificial

<220>
<223> An artificially synthesized nucleotide sequence

<400> 170
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

```
tcaggactct actccctcag cagcgtggtg accgtgccct ccagcagctt gggcacccag      660

acctacatct gcaacgtgaa tcacaagccc agcaacacca aggtggacaa gaaagcagag      720

cccaaatctt gtgacaaaac tcacacatgc ccaccgtgcc cagcacctga actcctgggg      780

ggaccgtcag tcttcctctt ccccccaaaa cccaaggaca ccctcatgat ctcccggacc      840

cctgaggtca catgcgtggt ggtggacgtg agccacgaag accctgaggt caagttcaac      900

tggtacgtgg acggcgtgga ggtgcataat gccaagacaa agccgcgtga ggagcagtac      960

aacagcacgt accgtgtggt cagctgcctc accgtcctgc accaggactg gctgaatggc     1020

aaggagtaca agtgcaaggt ctccaacaaa gccctcccag cccccatcga gaaaaccatc     1080

tccaaagcca aagggcagcc ccgagaacca caggtgtaca ccctgccccc atcccgggat     1140

gagctgacca agaaccaggt cagcctgacc tgcctggtca aaggcttcta tcccagcgac     1200

atcgccgtgg agtgggagag caatgggcag ccggagaaca actacaagac cacgcctccc     1260

gtgctggact ccgacggctc cttcttcctc tacagcaagc tcaccgtgga caagagcagg     1320

tggcagcagg ggaacgtctt ctcatgctcc gtgatgcatg aggctctgca caaccactac     1380

acgcagaaga gcctctccct gtctccgggt aaa                                  1413


<210>  171
<211>  471
<212>  PRT
<213>  Artificial

<220>
<223>  An artificially synthesized peptide sequence

<400>  171

Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110
```

Tyr Phe Cys Ala Arg Val Val Tyr Tyr Ser Asn Ser Tyr Trp Tyr Phe
115 120 125

Asp Val Trp Gly Thr Gly Thr Thr Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp TyrPhe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Ala Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Cys Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210> 172
<211> 990
<212> DNA
<213> Artificial

<220>
<223> An artificially synthesized nucleotide sequence

<400> 172
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagga gcagtacaac 540

agcacgtacc gtgtggtcag ctgcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 173
<211> 330
<212> PRT
<213> Artificial

<220>
<223> An artificially synthesized peptide sequence

<400> 173

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Cys Leu Thr Val Leu
            180                 185                 190
```

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 174
<211> 651
<212> DNA
<213> Artificial

<220>
<223> An artificially synthesized nucleotide sequence

<400> 174
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac cgtgtggtca gctgcctcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 175
<211> 217
<212> PRT
<213> Artificial

<220>
<223> An artificially synthesized peptide sequence

<400> 175

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Val Val Ser Cys Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
    210                 215

<210> 176
<211> 1413
<212> DNA
<213> Artificial

<220>
<223> An artificially synthesized nucleotide sequence

<400> 176
atgggatgga gctgggtctt tctcttcctc ctgtcagtaa ctacaggtgt ccactcccag 60
gcttatctac agcagtctgg ggctgagctg gtgaggcctg gggcctcagt gaagatgtcc 120
tgcaaggctt ctggctacac atttaccagt tacaatatgc actgggtaaa gcagacacct 180
agacagggcc tggaatggat tggagctatt tatccaggaa atggtgatac ttcctacaat 240
cagaagttca agggcaaggc cacactgact gtagacaaat cctccagcac agcctacatg 300
cagctcagca gcctgacatc tgaagactct gcggtctatt tctgtgcaag agtggtgtac 360
tatagtaact cttactggta cttcgatgtc tggggcacag ggaccacggt caccgtctcc 420
tcagcctcca ccaagggccc atcggtcttc cccctggcac cctcctccaa gagcacctct 480
gggggcacag cggccctggg ctgcctggtc aaggactact tccccgaacc ggtgacggtg 540
tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagcagag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900
tggtacgtgg acggcgtgga ggtgcataat gccaagacaa agccgcgtga ggagcagtac 960
aacagcacgt accgtgtggt cagcgtctgc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 177
<211> 471

<212> PRT
<213> Artificial

<220>
<223> An artificially synthesized peptide sequence

<400> 177

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
```

```
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Cys Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  178
<211>  990
```

<212> DNA
<213> Artificial

<220>
<223> An artificially synthesized nucleotide sequence

<400> 178
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagga gcagtacaac 540

agcacgtacc gtgtggtcag cgtctgcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 179
<211> 330
<212> PRT
<213> Artificial

<220>
<223> An artificially synthesized peptide sequence

<400> 179

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

```
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Cys Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320
```

Gln Lys Ser Leu Ser Leu Ser Pro Gly Lys
325 330

<210> 180
<211> 651
<212> DNA
<213> Artificial

<220>
<223> An artificially synthesized nucleotide sequence

<400> 180
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac cgtgtggtca gcgtctgcac cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 181
<211> 217
<212> PRT
<213> Artificial

<220>
<223> An artificially synthesized peptide sequence

<400> 181

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Val Val Ser Val Cys Thr Val Leu His

65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 182
<211> 1413
<212> DNA
<213> Artificial

<220>
<223> An artificially synthesized nucleotide sequence

<400> 182
atgggatgga gctgggtctt tctcttcctc ctgtcagtaa ctacaggtgt ccactcccag 60
gcttatctac agcagtctgg ggctgagctg gtgaggcctg gggcctcagt gaagatgtcc 120
tgcaaggctt ctggctacac atttaccagt tacaatatgc actgggtaaa gcagacacct 180
agacagggcc tggaatggat tggagctatt tatccaggaa atggtgatac ttcctacaat 240
cagaagttca agggcaaggc cacactgact gtagacaaat cctccagcac agcctacatg 300
cagctcagca gcctgacatc tgaagactct gcggtctatt tctgtgcaag agtggtgtac 360
tatagtaact cttactggta cttcgatgtc tggggcacag ggaccacggt caccgtctcc 420
tcagcctcca ccaagggccc atcggtcttc cccctggcac cctcctccaa gagcacctct 480
gggggcacag cggccctggg ctgcctggtc aaggactact tccccgaacc ggtgacggtg 540

tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagcagag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900
tggtacgtgg acggcgtgga ggtgcataat gccaagacaa agccgcgtga ggagcagtac 960
aacagcacgt accgtgtggt cagcgtcctc tgcgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaa 1413

<210> 183
<211> 471
<212> PRT
<213> Artificial

<220>
<223> An artificially synthesized peptide sequence

<400> 183

Met Gly Trp Ser Trp Val Phe Leu Phe Leu Leu Ser Val Thr Thr Gly
1 5 10 15

Val His Ser Gln Ala Tyr Leu Gln Gln Ser Gly Ala Glu Leu Val Arg
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Arg Gln Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Val Asp Lys Ser Ser Ser
85 90 95

```
Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Cys Val Leu His Gln Asp
                325                 330                 335

Trp Leu Asn Gly Lys Glu Tyr Lys Cys Lys Val Ser Asn Lys Ala Leu
            340                 345                 350
```

```
Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 184
<211> 990
<212> DNA
<213> Artificial

<220>
<223> An artificially synthesized nucleotide sequence

<400> 184

```
gcctccacca agggcccatc ggtcttcccc ctggcaccct cctccaagag cacctctggg      60
ggcacagcgg ccctgggctg cctggtcaag gactacttcc ccgaaccggt gacggtgtcg     120
tggaactcag gcgccctgac cagcggcgtg cacaccttcc cggctgtcct acagtcctca     180
ggactctact ccctcagcag cgtggtgacc gtgccctcca gcagcttggg cacccagacc     240
tacatctgca acgtgaatca caagcccagc aacaccaagg tggacaagaa agcagagccc     300
aaatcttgtg acaaaactca cacatgccca ccgtgcccag cacctgaact cctgggggga     360
ccgtcagtct tcctcttccc cccaaaaccc aaggacaccc tcatgatctc ccggacccct     420
gaggtcacat gcgtggtggt ggacgtgagc cacgaagacc ctgaggtcaa gttcaactgg     480
tacgtggacg gcgtggaggt gcataatgcc aagacaaagc cgcgtgagga gcagtacaac     540
agcacgtacc gtgtggtcag cgtcctctgc gtcctgcacc aggactggct gaatggcaag     600
gagtacaagt gcaaggtctc caacaaagcc ctcccagccc ccatcgagaa aaccatctcc     660
aaagccaaag ggcagccccg agaaccacag gtgtacaccc tgcccccatc ccgggatgag     720
```

```
ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa                                      990


<210>  185
<211>  330
<212>  PRT
<213>  Artificial

<220>
<223>  An artificially synthesized peptide sequence

<400>  185

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175
```

Glu Gln Tyr Asn Ser Thr Tyr Arg Val Val Ser Val Leu Cys Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 186
<211> 651
<212> DNA
<213> Artificial

<220>
<223> An artificially synthesized nucleotide sequence

<400> 186
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac cgtgtggtca gcgtcctctg cgtcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

```
tacaagacca cgcctcccgt gctggactcc gacggctcct tcttcctcta cagcaagctc    540

accgtggaca agagcaggtg gcagcagggg aacgtcttct catgctccgt gatgcatgag    600

gctctgcaca accactacac gcagaagagc ctctccctgt ctccgggtaa a             651


<210>  187
<211>  217
<212>  PRT
<213>  Artificial

<220>
<223>  An artificially synthesized peptide sequence

<400>  187

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Cys Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190
```

Phe Ser Cys Ser Val Met His Glu Ala Leu His Asn His Tyr Thr Gln
195 200 205

Lys Ser Leu Ser Leu Ser Pro Gly Lys
210 215

<210> 188
<211> 1413
<212> DNA
<213> Artificial

<220>
<223> An artificially synthesized nucleotide sequence

<400> 188
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcgtga ggagcagtac 960

aacagcacgt accgtgtggt cagcgtcctc acctgcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 189
<211> 471
<212> PRT
<213> Artificial

<220>
<223> An artificially synthesized peptide sequence

<400> 189

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220
```

```
Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Cys Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 190
<211> 990
<212> DNA
<213> Artificial

<220>
<223> An artificially synthesized nucleotide sequence

<400> 190
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgtgagga gcagtacaac 540

agcacgtacc gtgtggtcag cgtcctcacc tgcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 191
<211> 330
<212> PRT
<213> Artificial

<220>
<223> An artificially synthesized peptide sequence

<400> 191

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

```
Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Cys Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300
```

Val Phe Ser Cys Ser Val Met His Glu Ala Leu His Asn His Tyr Thr
305 310 315 320

Gln Lys Ser Leu Ser Leu Ser Pro Gly Lys
325 330

<210> 192
<211> 651
<212> DNA
<213> Artificial

<220>
<223> An artificially synthesized nucleotide sequence

<400> 192
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac cgtgtggtca gcgtcctcac ctgcctgcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 193
<211> 217
<212> PRT
<213> Artificial

<220>
<223> An artificially synthesized peptide sequence

<400> 193

Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

```
Gln Tyr Asn Ser Thr Tyr Arg Val Val Ser Val Leu Thr Cys Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Asp Glu Leu
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215

<210>  194
<211>  21
<212>  DNA
<213>  Homo sapiens

<400>  194
agtggtggtt actactggag c                                                  21

<210>  195
<211>  7
<212>  PRT
<213>  Homo sapiens

<400>  195

Ser Gly Gly Tyr Tyr Trp Ser
1               5

<210>  196
<211>  48
<212>  DNA
```

<213> Homo sapiens

<400> 196
tacatctatt acagtgggag cacctactac aacccgtccc tcaagagt 48

<210> 197
<211> 16
<212> PRT
<213> Homo sapiens

<400> 197

Tyr Ile Tyr Tyr Ser Gly Ser Thr Tyr Tyr Asn Pro Ser Leu Lys Ser
1 5 10 15

<210> 198
<211> 30
<212> DNA
<213> Homo sapiens

<400> 198
acaccgtggg agctactagc ttttgatatc 30

<210> 199
<211> 10
<212> PRT
<213> Homo sapiens

<400> 199

Thr Pro Trp Glu Leu Leu Ala Phe Asp Ile
1 5 10

<210> 200
<211> 705
<212> DNA
<213> Homo sapiens

<400> 200
atggacatga gggtccccgc tcagctcctg gggctcctgc tactctggct ccgcggtgcc 60

agatcctatg agctgactca gccaccctca gtgtcagtgg ccccaggaaa gacgaccagg 120

attacctgtg ggggaaacaa cattggaagt aaaagtgcgc actggtacca gcagaagcca 180

ggccaggccc ctgtgctggt catctattat gatagcgacc ggccctcagg gatccctgag 240

cgattctctg gctccaactc tgggaacacg gccaccctga ccatcagcag ggtcgaagcc 300

ggggatgagg ccgactatta ctgtcaggtg tgggatagta gtagtgatca ttgggtgttc 360

ggcggaggga ccaagctgac cgtcctaggt cagcccaagg ctgccccctc ggtcactctg 420

ttcccgccct cctctgagga gcttcaagcc aacaaggcca cactggtgtg tctcataagt 480

gacttctacc cgggagccgt gacagtggcc tggaaggcag atagcagccc cgtcaaggcg 540

ggagtggaga ccaccacacc ctccaaacaa agcaacaaca agtacgcggc cagcagctac 600

ctgagcctga cgcctgagca gtggaagtcc cacaaaagct acagctgcca ggtcacgcat 660

gaagggagca ccgtggagaa gacagtggcc cctacagaat gttcg 705

<210> 201
<211> 235
<212> PRT
<213> Homo sapiens

<400> 201

Met Asp Met Arg Val Pro Ala Gln Leu Leu Gly Leu Leu Leu Leu Trp
1               5                   10                  15

Leu Arg Gly Ala Arg Ser Tyr Glu Leu Thr Gln Pro Pro Ser Val Ser
            20                  25                  30

Val Ala Pro Gly Lys Thr Thr Arg Ile Thr Cys Gly Gly Asn Asn Ile
        35                  40                  45

Gly Ser Lys Ser Ala His Trp Tyr Gln Gln Lys Pro Gly Gln Ala Pro
    50                  55                  60

Val Leu Val Ile Tyr Tyr Asp Ser Asp Arg Pro Ser Gly Ile Pro Glu
65                  70                  75                  80

Arg Phe Ser Gly Ser Asn Ser Gly Asn Thr Ala Thr Leu Thr Ile Ser
                85                  90                  95

Arg Val Glu Ala Gly Asp Glu Ala Asp Tyr Tyr Cys Gln Val Trp Asp
            100                 105                 110

Ser Ser Ser Asp His Trp Val Phe Gly Gly Gly Thr Lys Leu Thr Val
        115                 120                 125

Leu Gly Gln Pro Lys Ala Ala Pro Ser Val Thr Leu Phe Pro Pro Ser
    130                 135                 140

Ser Glu Glu Leu Gln Ala Asn Lys Ala Thr Leu Val Cys Leu Ile Ser
145                 150                 155                 160

Asp Phe Tyr Pro Gly Ala Val Thr Val Ala Trp Lys Ala Asp Ser Ser
                165                 170                 175

Pro Val Lys Ala Gly Val Glu Thr Thr Thr Pro Ser Lys Gln Ser Asn
            180                 185                 190

Asn Lys Tyr Ala Ala Ser Ser Tyr Leu Ser Leu Thr Pro Glu Gln Trp
        195                 200                 205

Lys Ser His Lys Ser Tyr Ser Cys Gln Val Thr His Glu Gly Ser Thr
    210                 215                 220

Val Glu Lys Thr Val Ala Pro Thr Glu Cys Ser
225 230 235

<210> 202
<211> 33
<212> DNA
<213> Homo sapiens

<400> 202
gggggaaaca acattggaag taaaagtgcg cac 33

<210> 203
<211> 11
<212> PRT
<213> Homo sapiens

<400> 203

Gly Gly Asn Asn Ile Gly Ser Lys Ser Ala His
1 5 10

<210> 204
<211> 21
<212> DNA
<213> Homo sapiens

<400> 204
tatgatagcg accggccctc a 21

<210> 205
<211> 7
<212> PRT
<213> Homo sapiens

<400> 205

Tyr Asp Ser Asp Arg Pro Ser
1 5

<210> 206
<211> 33
<212> DNA
<213> Homo sapiens

<400> 206
caggtgtggg atagtagtag tgatcattgg gtg 33

<210> 207
<211> 11
<212> PRT
<213> Homo sapiens

<400> 207

Gln Val Trp Asp Ser Ser Ser Asp His Trp Val
1 5 10

<210> 208
<211> 1404
<212> DNA
<213> Artificial

<220>
<223> An artificially synthesized nucleotide sequence

<400> 208
atgaaacacc tgtggttctt cctcctacta gtggcagctc ccagatgggt cctgtcccag 60
gtgcagctgc aggagtcggg cccaggactg gtgaagcctt cacagaccct gtccctcacc 120
tgcactgtct ctggtggctc catcagcagt ggtggttact actggagctg gatccgccag 180
cacccaggga agggcctgga gtggattggg tacatctatt acagtgggag cacctactac 240
aacccgtccc tcaagagtcg agtcaccata tcagtagaca cgtctaagaa ccagttctcc 300
ctgaagctga gctctgtgac tgccgcggac acggccgtgt attactgtgc gaggacaccg 360
tgggagctac tagcttttga tatctggggc caagggacaa tggtcaccgt ctcggcctcc 420
accaagggcc catcggtctt ccccctggca ccctcctcca agagcacctc tgggggcaca 480
gcggccctgg gctgcctggt caaggactac ttccccgaac cggtgacggt gtcgtggaac 540
tcaggcgccc tgaccagcgg cgtgcacacc ttcccggctg tcctacagtc ctcaggactc 600
tactccctca gcagcgtggt gaccgtgccc tccagcagct tgggcaccca gacctacatc 660
tgcaacgtga atcacaagcc cagcaacacc aaggtggaca agaaagcaga gcccaaatct 720
tgtgacaaaa ctcacacatg cccaccgtgc ccagcacctg aactcctggg gggaccgtca 780
gtcttcctct tccccccaaa acccaaggac accctcatga tctcccggac ccctgaggtc 840
acatgcgtgg tggtggacgt gagccacgaa gaccctgagg tcaagttcaa ctggtacgtg 900
gacggcgtgg aggtgcataa tgccaagaca aagccgcgtg aggagcagta caactgcacg 960
taccgtgtgg tcagcgtcct caccgtcctg caccaggact ggctgaatgg caaggagtac 1020
aagtgcaagg tctccaacaa agccctccca gcccccatcg agaaaaccat ctccaaagcc 1080
aaagggcagc cccgagaacc acaggtgtac accctgcccc catcccggga tgagctgacc 1140
aagaaccagg tcagcctgac ctgcctggtc aaaggcttct atcccagcga catcgccgtg 1200
gagtgggaga gcaatgggca gccggagaac aactacaaga ccacgcctcc cgtgctggac 1260
tccgacggct ccttcttcct ctacagcaag ctcaccgtgg acaagagcag gtggcagcag 1320
gggaacgtct tctcatgctc cgtgatgcat gaggctctgc acaaccacta cacgcagaag 1380
agcctctccc tgtctccggg taaa 1404

<210> 209
<211> 468
<212> PRT
<213> Artificial

<220>
<223> An artificially synthesized peptide sequence

<400> 209

```
Met Lys His Leu Trp Phe Phe Leu Leu Leu Val Ala Ala Pro Arg Trp
1               5                   10                  15

Val Leu Ser Gln Val Gln Leu Gln Glu Ser Gly Pro Gly Leu Val Lys
            20                  25                  30

Pro Ser Gln Thr Leu Ser Leu Thr Cys Thr Val Ser Gly Gly Ser Ile
        35                  40                  45

Ser Ser Gly Gly Tyr Tyr Trp Ser Trp Ile Arg Gln His Pro Gly Lys
    50                  55                  60

Gly Leu Glu Trp Ile Gly Tyr Ile Tyr Tyr Ser Gly Ser Thr Tyr Tyr
65                  70                  75                  80

Asn Pro Ser Leu Lys Ser Arg Val Thr Ile Ser Val Asp Thr Ser Lys
                85                  90                  95

Asn Gln Phe Ser Leu Lys Leu Ser Ser Val Thr Ala Ala Asp Thr Ala
            100                 105                 110

Val Tyr Tyr Cys Ala Arg Thr Pro Trp Glu Leu Leu Ala Phe Asp Ile
        115                 120                 125

Trp Gly Gln Gly Thr Met Val Thr Val Ser Ala Ser Thr Lys Gly Pro
    130                 135                 140

Ser Val Phe Pro Leu Ala Pro Ser Ser Lys Ser Thr Ser Gly Gly Thr
145                 150                 155                 160

Ala Ala Leu Gly Cys Leu Val Lys Asp Tyr Phe Pro Glu Pro Val Thr
                165                 170                 175

Val Ser Trp Asn Ser Gly Ala Leu Thr Ser Gly Val His Thr Phe Pro
            180                 185                 190

Ala Val Leu Gln Ser Ser Gly Leu Tyr Ser Leu Ser Ser Val Val Thr
        195                 200                 205

Val Pro Ser Ser Ser Leu Gly Thr Gln Thr Tyr Ile Cys Asn Val Asn
    210                 215                 220

His Lys Pro Ser Asn Thr Lys Val Asp Lys Lys Ala Glu Pro Lys Ser
225                 230                 235                 240

Cys Asp Lys Thr His Thr Cys Pro Pro Cys Pro Ala Pro Glu Leu Leu
                245                 250                 255
```

```
Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser
        275                 280                 285

His Glu Asp Pro Glu Val Lys Phe Asn Trp Tyr Val Asp Gly Val Glu
    290                 295                 300

Val His Asn Ala Lys Thr Lys Pro Arg Glu Glu Gln Tyr Asn Cys Thr
305                 310                 315                 320

Tyr Arg Val Val Ser Val Leu Thr Val Leu His Gln Asp Trp Leu Asn
                325                 330                 335

Gly Lys Glu Tyr Lys Cys Lys Val Ser Asn Lys Ala Leu Pro Ala Pro
            340                 345                 350

Ile Glu Lys Thr Ile Ser Lys Ala Lys Gly Gln Pro Arg Glu Pro Gln
        355                 360                 365

Val Tyr Thr Leu Pro Pro Ser Arg Asp Glu Leu Thr Lys Asn Gln Val
    370                 375                 380

Ser Leu Thr Cys Leu Val Lys Gly Phe Tyr Pro Ser Asp Ile Ala Val
385                 390                 395                 400

Glu Trp Glu Ser Asn Gly Gln Pro Glu Asn Asn Tyr Lys Thr Thr Pro
                405                 410                 415

Pro Val Leu Asp Ser Asp Gly Ser Phe Phe Leu Tyr Ser Lys Leu Thr
            420                 425                 430

Val Asp Lys Ser Arg Trp Gln Gln Gly Asn Val Phe Ser Cys Ser Val
        435                 440                 445

Met His Glu Ala Leu His Asn His Tyr Thr Gln Lys Ser Leu Ser Leu
    450                 455                 460

Ser Pro Gly Lys
465
```

<210> 210
<211> 39
<212> DNA
<213> Artificial

<220>
<223> An artificially synthesized primer sequence

<400> 210
gtgcataatg ccaagacatg cccgcgtgag gagcagtac 39

<210> 211
<211> 39
<212> DNA
<213> Artificial

<220>
<223> An artificially synthesized primer sequence

<400> 211
gtactgctcc tcacgcgggc atgtcttggc attatgcac 39

<210> 212
<211> 39
<212> DNA
<213> Artificial

<220>
<223> An artificially synthesized primer sequence

<400> 212
cataatgcca agacaaagtg ccgtgaggag cagtacaac 39

<210> 213
<211> 39
<212> DNA
<213> Artificial

<220>
<223> An artificially synthesized primer sequence

<400> 213
gttgtactgc tcctcacggc actttgtctt ggcattatg 39

<210> 214
<211> 33
<212> DNA
<213> Artificial

<220>
<223> An artificially synthesized primer sequence

<400> 214
gccaagacaa agccgtgtga ggagcagtac aac 33

<210> 215
<211> 33
<212> DNA
<213> Artificial

<220>
<223> An artificially synthesized primer sequence

<400> 215
gttgtactgc tcctcacacg gctttgtctt ggc 33

<210> 216
<211> 39
<212> DNA
<213> Artificial

<220>
<223> An artificially synthesized primer sequence

<400> 216
tacaacagca cgtaccgttg cgtcagcgtc ctcaccgtc 39

<210> 217
<211> 39
<212> DNA
<213> Artificial

<220>
<223> An artificially synthesized primer sequence

<400> 217
gacggtgagg acgctgacgc aacggtacgt gctgttgta 39

<210> 218
<211> 39
<212> DNA
<213> Artificial

<220>
<223> An artificially synthesized primer sequence

<400> 218
aacagcacgt accgtgtgtg cagcgtcctc accgtcctg 39

<210> 219
<211> 39
<212> DNA
<213> Artificial

<220>
<223> An artificially synthesized primer sequence

<400> 219
caggacggtg aggacgctgc acacacggta cgtgctgtt 39

<210> 220
<211> 37
<212> DNA
<213> Artificial

<220>
<223> An artificially synthesized primer sequence

<400> 220
accgctcgag atggacatga gggtccccgc tcagctc 37

<210> 221
<211> 46
<212> DNA
<213> Artificial

<220>
<223> An artificially synthesized primer sequence

<400> 221
atagtttagc ggccgcttac gaacattctg taggggccac tgtctt 46

<210> 222
<211> 34
<212> DNA
<213> Artificial

<220>
<223> An artificially synthesized primer sequence

<400> 222
accgctcgag atgaaacacc tgtggttctt cctc 34

<210> 223
<211> 24
<212> DNA
<213> Artificial

<220>
<223> An artificially synthesized primer sequence

<400> 223
cgagacggtg accattgtcc cttg 24

<210> 224
<211> 39
<212> DNA
<213> Artificial

<220>
<223> An artificially synthesized primer sequence

<400> 224
gacggcgtgg aggtgcattg tgccaagaca aagccgcgt 39

<210> 225
<211> 39
<212> DNA
<213> Artificial

<220>
<223> An artificially synthesized primer sequence

<400> 225
acgcggcttt gtcttggcac aatgcacctc cacgccgtc 39

<210> 226
<211> 39
<212> DNA
<213> Artificial

<220>
<223> An artificially synthesized primer sequence

<400> 226
ggcgtggagg tgcataattg caagacaaag ccgcgtaag 39

<210> 227
<211> 39
<212> DNA
<213> Artificial

<220>
<223> An artificially synthesized primer sequence

<400> 227
cttacgcggc tttgtcttgc aattatgcac ctccacgcc 39

<210> 228
<211> 39
<212> DNA
<213> Artificial

<220>
<223> An artificially synthesized primer sequence

<400> 228
gtggaggtgc ataatgcctg tacaaagccg cgtgaggag 39

<210> 229
<211> 39
<212> DNA
<213> Artificial

<220>
<223> An artificially synthesized primer sequence

<400> 229
ctcctcacgc ggctttgtac aggcattatg cacctccac 39

<210> 230
<211> 39
<212> DNA
<213> Artificial

<220>
<223> An artificially synthesized primer sequence

<400> 230
gaggtgcata atgccaagtg caagccgcgt gaggagcag 39

<210> 231
<211> 39
<212> DNA
<213> Artificial

<220>
<223> An artificially synthesized primer sequence

<400> 231
ctgctcctca cgcggcttgc acttggcatt atgcacctc 39

<210> 232
<211> 39
<212> DNA

<213> Artificial

<220>
<223> An artificially synthesized primer sequence

<400> 232
acgtaccgtg tggtcagctg cctcaccgtc ctgcaccag 39

<210> 233
<211> 39
<212> DNA
<213> Artificial

<220>
<223> An artificially synthesized primer sequence

<400> 233
ctggtgcagg acggtgaggc agctgaccac acggtacgt 39

<210> 234
<211> 39
<212> DNA
<213> Artificial

<220>
<223> An artificially synthesized primer sequence

<400> 234
taccgtgtgg tcagcgtctg caccgtcctg caccaggac 39

<210> 235
<211> 39
<212> DNA
<213> Artificial

<220>
<223> An artificially synthesized primer sequence

<400> 235
gtcctggtgc aggacggtgc agacgctgac cacacggta 39

<210> 236
<211> 39
<212> DNA
<213> Artificial

<220>
<223> An artificially synthesized primer sequence

<400> 236
cgtgtggtca gcgtcctctg cgtcctgcac caggactgg 39

<210> 237
<211> 39
<212> DNA
<213> Artificial

<220>
<223> An artificially synthesized primer sequence

<400> 237
ccagtcctgg tgcaggacgc agaggacgct gaccacacg 39

<210> 238
<211> 39
<212> DNA
<213> Artificial

<220>
<223> An artificially synthesized primer sequence

<400> 238
gtggtcagcg tcctcacctg cctgcaccag gactggctg 39

<210> 239
<211> 39
<212> DNA
<213> Artificial

<220>
<223> An artificially synthesized primer sequence

<400> 239
cagccagtcc tggtgcaggc aggtgaggac gctgaccac 39

<210> 240
<211> 1413
<212> DNA
<213> Artificial

<220>
<223> An artificially synthesized nucleotide sequence

<400> 240
atgggatgga gctgggtctt tctcttcctc ctgtcagtaa ctacaggtgt ccactcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

```
cctgaggtca catgcgtggt ggtggacgtg agccacgaag accctgaggt caagttcaac    900
tggtacgtgg acggcgtgga ggtgcataat gccaagacaa agccgcgtga ggagcagtac    960
aacagcacgt accgtgtggt cagcgtcctc accgtctgtc accaggactg gctgaatggc   1020
aaggagtaca agtgcaaggt ctccaacaaa gccctcccag cccccatcga gaaaaccatc   1080
tccaaagcca aagggcagcc ccgagaacca caggtgtaca ccctgccccc atcccgggat   1140
gagctgacca agaaccaggt cagcctgacc tgcctggtca aaggcttcta tcccagcgac   1200
atcgccgtgg agtgggagag caatgggcag ccggagaaca actacaagac cacgcctccc   1260
gtgctggact ccgacggctc cttcttcctc tacagcaagc tcaccgtgga caagagcagg   1320
tggcagcagg ggaacgtctt ctcatgctcc gtgatgcatg aggctctgca caaccactac   1380
acgcagaaga gcctctccct gtctccgggt aaa                                1413
```

<210> 241
<211> 471
<212> PRT
<213> Artificial

<220>
<223> An artificially synthesized peptide sequence

<400> 241

```
Met Gly Trp Ser Trp Val Phe Leu Phe Leu Leu Ser Val Thr Thr Gly
1               5                   10                  15

Val His Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140
```

```
Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Cys His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400
```

```
Ile Ala Val Glu Trp Glu Ser Asn Gly Gln Pro Glu Asn Asn Tyr Lys
            405                 410                 415

Thr Thr Pro Pro Val Leu Asp Ser Asp Gly Ser Phe Phe Leu Tyr Ser
        420                 425                 430

Lys Leu Thr Val Asp Lys Ser Arg Trp Gln Gln Gly Asn Val Phe Ser
    435                 440                 445

Cys Ser Val Met His Glu Ala Leu His Asn His Tyr Thr Gln Lys Ser
    450                 455                 460

Leu Ser Leu Ser Pro Gly Lys
465                 470
```

<210> 242
<211> 990
<212> DNA
<213> Artificial

<220>
<223> An artificially synthesized nucleotide sequence

<400> 242

```
gcctccacca agggcccatc ggtcttcccc ctggcaccct cctccaagag cacctctggg    60
ggcacagcgg ccctgggctg cctggtcaag gactacttcc ccgaaccggt gacggtgtcg   120
tggaactcag gcgccctgac cagcggcgtg cacaccttcc cggctgtcct acagtcctca   180
ggactctact ccctcagcag cgtggtgacc gtgccctcca gcagcttggg cacccagacc   240
tacatctgca acgtgaatca caagcccagc aacaccaagg tggacaagaa agcagagccc   300
aaatcttgtg acaaaactca cacatgccca ccgtgcccag cacctgaact cctgggggga   360
ccgtcagtct tcctcttccc cccaaaaccc aaggacaccc tcatgatctc ccggacccct   420
gaggtcacat gcgtggtggt ggacgtgagc cacgaagacc ctgaggtcaa gttcaactgg   480
tacgtggacg gcgtggaggt gcataatgcc aagacaaagc cgcgtgagga gcagtacaac   540
agcacgtacc gtgtggtcag cgtcctcacc gtctgtcacc aggactggct gaatggcaag   600
gagtacaagt gcaaggtctc caacaaagcc ctcccagccc ccatcgagaa aaccatctcc   660
aaagccaaag ggcagccccg agaaccacag gtgtacaccc tgcccccatc ccgggatgag   720
ctgaccaaga accaggtcag cctgacctgc ctggtcaaag gcttctatcc cagcgacatc   780
gccgtggagt gggagagcaa tgggcagccg gagaacaact acaagaccac gcctcccgtg   840
ctggactccg acggctcctt cttcctctac agcaagctca ccgtggacaa gagcaggtgg   900
cagcagggga acgtcttctc atgctccgtg atgcatgagg ctctgcacaa ccactacacg   960
cagaagagcc tctccctgtc tccgggtaaa                                    990
```

<210> 243
<211> 330
<212> PRT
<213> Artificial

<220>
<223> An artificially synthesized peptide sequence

<400> 243

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Cys
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220
```

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 244
<211> 651
<212> DNA
<213> Artificial

<220>
<223> An artificially synthesized nucleotide sequence

<400> 244
gcacctgaac tcctgggggg accgtcagtc ttcctcttcc ccccaaaacc caaggacacc 60

ctcatgatct cccggacccc tgaggtcaca tgcgtggtgg tggacgtgag ccacgaagac 120

cctgaggtca agttcaactg gtacgtggac ggcgtggagg tgcataatgc caagacaaag 180

ccgcgtgagg agcagtacaa cagcacgtac cgtgtggtca gcgtcctcac cgtctgtcac 240

caggactggc tgaatggcaa ggagtacaag tgcaaggtct ccaacaaagc cctcccagcc 300

cccatcgaga aaaccatctc caaagccaaa gggcagcccc gagaaccaca ggtgtacacc 360

ctgcccccat cccgggatga gctgaccaag aaccaggtca gcctgacctg cctggtcaaa 420

ggcttctatc ccagcgacat cgccgtggag tgggagagca atgggcagcc ggagaacaac 480

tacaagacca cgcctcccgt gctggactcc gacggctcct tcttcctcta cagcaagctc 540

accgtggaca agagcaggtg gcagcagggg aacgtcttct catgctccgt gatgcatgag 600

gctctgcaca accactacac gcagaagagc ctctccctgt ctccgggtaa a 651

<210> 245
<211> 217
<212> PRT
<213> Artificial

<220>
<223> An artificially synthesized peptide sequence

<400> 245
Ala Pro Glu Leu Leu Gly Gly Pro Ser Val Phe Leu Phe Pro Pro Lys
1 5 10 15

Pro Lys Asp Thr Leu Met Ile Ser Arg Thr Pro Glu Val Thr Cys Val
20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Val Val Ser Val Leu Thr Val Cys His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Asp Glu Leu
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 246
<211> 315
<212> DNA
<213> Artificial

<220>
<223> An artificially synthesized nucleotide sequence

<400> 246
cagcccaagg ctgccccctc ggtcactctg ttcccgccct cctctgagga gcttcaagcc 60

aacaaggcca cactggtgtg tctcataagt gacttctacc cgggagccgt gacagtggcc 120

tggaaggcag atagcagccc cgtcaaggcg ggagtggaga ccaccacacc ctccaaacaa 180

agcaacaaca agtacgcggc cagcagctac ctgagcctga cgcctgagca gtggaagtcc 240

cacaaaagct acagctgcca ggtcacgcat gaagggagca ccgtggagaa gacagtggcc 300

cctacagaat gttcg 315

<210> 247
<211> 105
<212> PRT
<213> Artificial

<220>
<223> An artificially synthesized peptide sequence

<400> 247
Gln Pro Lys Ala Ala Pro Ser Val Thr Leu Phe Pro Pro Ser Ser Glu
1 5 10 15

Glu Leu Gln Ala Asn Lys Ala Thr Leu Val Cys Leu Ile Ser Asp Phe
20 25 30

Tyr Pro Gly Ala Val Thr Val Ala Trp Lys Ala Asp Ser Ser Pro Val
35 40 45

Lys Ala Gly Val Glu Thr Thr Thr Pro Ser Lys Gln Ser Asn Asn Lys
50 55 60

Tyr Ala Ala Ser Ser Tyr Leu Ser Leu Thr Pro Glu Gln Trp Lys Ser
65 70 75 80

His Lys Ser Tyr Ser Cys Gln Val Thr His Glu Gly Ser Thr Val Glu
85 90 95

Lys Thr Val Ala Pro Thr Glu Cys Ser
100 105